# EUROPEAN PATENT APPLICATION

(11) **EP 1 104 811 A1**
(43) Date of publication of application: **06.06.2001**
(21) Application number: 99870252.6
(22) Date of filing: 03.12.1999
(51) Int. Cl.: C12N 15/36, C07K 14/02, C12Q 1/70, C12Q 1/68, A61K 39/29, G01N 33/569, A61P 31/20

(54) **HBV sequences**

(71) Applicant: INNOGENETICS N.V., 9052 Gent (BE)
(72) Inventor: Stuyver, Lieven, 9552 Herzele (BE)

(57) **Abstract**

The complete nucleic acid sequence of a new human hepatitis B virus genotype, provisionally named genotype G, is reported. This genotype was found with a high prevalence in patients chronically infected with HBV and residing in Europe and the USA. The present invention further relates to polypeptides encoded by said nucleic acid sequence and to antibodies recognizing said polypeptides. The present invention also relates to the use of said nucleic acid, polypeptides and antibodies in HBV diagnosis, prophylaxis and therapy.

### Annex to the application documents - subsequently filed sequences listing

SEQUENCE LISTING

<110> INNOGENETICS N.V.

<120> New HBV Sequences

<130> EP99.110.HBVG

<140> 99870252.6
<141> 1999-12-03

<160> 168

<170> PatentIn Ver. 2.1

<210> 1
<211> 3248
<212> DNA
<213> Hepatitis B virus

<220>
<221> misc_feature
<222> (1)..(3248)
<223> Circular DNA

<400> 1
ctctacagca ttccaccaag ctctacaaaa tcccaaagtc aggggcctgt attttcctgc 60
tggtggctcc agttcaggga tagtgaaccc tgttccgact attgcctctc acatctcgtc 120
aatcttctcc aggattgggg accctgcacc gaacatggag aacatcacat caggattcct 180
aggacccctg ctcgtgttac aggcggggtt tttcttgttg acaagaatcc tcacaatacc 240
gcagagtcta gactcgtggt ggacttctct caattttcta gggggagtgc ccgtgtgtcc 300
tggcctaaat tcgcagtccc caacctccaa tcactcacca atctcctgtc ctccaacttg 360
tcctggctat cgctggatgt gtctgcggcg ttttatcata ttcctcttca tcctgctgct 420
atgcctcatc ttcttgttgg ttcttctgga ctatcaaggt atgttgcccg tttgtcctct 480
gattccagga tcctcgacca ccagtacggg accctgcaaa acctgcacga ctcctgctca 540
aggcaactct atgtatccct catgttgctg tacaaaacct tcggacggaa attgcacctg 600
tattcccatc ccatcatctt gggctttcgc aaaataccta tgggagtggg cctcagtccg 660
tttctcttgg ctcagtttac tagtgccatt tgttcagtgg ttcgtagggc tttcccccac 720
tgtctggctt tcagctatat ggatgatgtg gtattggggg ccaaatctgt acaacatctt 780
gagtcccttt ataccgctgt taccaatttt cttttgtctt tgggtataca tctaaaccct 840
aacaaaacaa aaagatgggg ttattcctta aattttatgg gatatgtaat tggaagttgg 900
ggtactttgc cacaagaaca catcacacag aaaattaagc aatgttttcg gaaactccct 960
gttaacaggc caattgattg gaaagtctgt caacgaataa ctggtctgtt gggtttcgct 1020
gctcctttca cccaatgtgg ttaccctgcc ttaatgcctt tatatgcatg tatacaagct 1080
aagcaggctt ttactttctc gccaacttat aaggcctttc tctgtaaaca atacatgaac 1140
ctttaccccg ttgctaggca acggcccggt ctgtgccaag tgtttgctga cgcaaccccc 1200
actggttggg gcttggccat cggccatcag cgcatgcgtg gaacctttgt ggctcctctg 1260
ccgatccata ctgcggaact cctagctgct tgttttgctc gcagccggtc tggagcaaaa 1320

ctcattggga ctgacaattc tgtcgtcctt tctcggaaat atacatcctt tccatggctg 1380
ctaggctgtg ctgccaactg gatccttcgc gggacgtcct ttgtttacgt cccgtcagcg 1440
ctgaatccag cggacgaccc ctcccggggc cgtttggggc tctgtcgccc ccttctccgt 1500
ctgccgttcc tgccgaccac ggggcgcacc tctctttacg cggtctcccc gtctgttcct 1560
tctcatctgc cggaccgtgt gcacttcgct tcacctctgc acgttacatg gaaaccgcca 1620
tgaacacctc tcatcatctg ccaaggcagt tatataagtg gactcttgga ctgtttgtta 1680
tgtcaacaac cggggtggag aaatacttca aggactgtgt ttttgctgag tgggaagaat 1740
taggcaatga gtccaggtta atgacctttg tattaggagg ctgtaggcat aaattggtct 1800
gcgcaccagc accatgtaac tttttcacct ctgcctaatc atctcttgtt catgtcctac 1860
tgttcaagcc tccaagctgt gccttgggtg gctttagggc atggatagaa caactttgcc 1920
atatggcctt tttggcttag acattgaccc ttataaagaa tttggagcta ctgtggagtt 1980
gctctcgttt ttgccttctg actttttccc gtctgttcgt gatcttctcg acaccgcttc 2040
agctttgtac cgggaatcct tagagtcctc tgatcattgt tcgcctcacc atacagcact 2100
caggcaagca atcctgtgct ggggtgagtt gatgactcta gctacctggg tgggtaataa 2160
tttggaagat ccagcatcca gagatttggt ggtcaattat gttaatacta atatgggttt 2220
aaaaatcagg caactattgt ggtttcacat ttcctgtctt acttttggga gagaaaccgt 2280
tcttgagtat ttggtgtctt ttggagtgtg gattcgcact cctcctgctt atagaccacc 2340
aaatgcccct atcctatcaa cacttccgga gactactgtt gttagacgaa gaggcaggtc 2400
ccctcgaaga agaactccct cgcctcgcag acgaagatct caatcgccgc gtcgcagaag 2460
atctgcatct ccagcttccc aatgttagta ttccttggac tcacaaggtg ggaaacttta 2520
cggggctgta ttcttctact atacctgtct ttaatcctga ttggcaaact ccttcttttc 2580
caaatatcca tttgcatcaa gacattataa ctaaatgtga acaatttgtg ggccctctca 2640
cagtaaatga gaaacgaaga ttaaaactag ttatgcctgc cagatttttc ccaaactcta 2700
ctaaatattt accattagac aaaggtatca aaccgtatta tccagaaaat gtagttaatc 2760
attacttcca gaccagacat tatttacata ccctttggaa ggcgggtatt ctatataaga 2820
gagaaacatc ccgtagcgct tcattttgtg ggtcaccata tacttgggaa caagatctac 2880
agcatggggc tttcttggac ggtccctctc gagtggggaa agaacctttc cgccagcaat 2940
cctctaggat tccttcccga tcaccagttg gacccagcat tcagagcaaa taccaacaat 3000
ccagattggg acttcaatcc caaaaaggac ccttggccag aggccaacaa ggtaggagtt 3060
ggagcctatg gacccgggtt cacccctcca cacggaggcc ttttggggtg gagccctcag 3120
tctcagggca cactaacaac tttgccagca gatccgcctc ctgcctccac caatcgtcag 3180
tcagggaggc agcctactcc catctctcca ccactaagag acagtcatcc tcaggccatg 3240
cagtggaa 3248

<210> 2
<211> 153
<212> PRT
<213> Hepatitis B virus

<400> 2
Met Ala Ala Arg Leu Cys Cys Gln Leu Asp Pro Ser Arg Asp Val Leu
1               5                   10                  15

Cys Leu Arg Pro Val Ser Ala Glu Ser Ser Gly Arg Pro Leu Pro Gly
            20                  25                  30

Pro Phe Gly Ala Leu Ser Pro Pro Ser Pro Ser Ala Val Pro Ala Asp

35                  40                  45

His Gly Ala His Leu Ser Leu Arg Gly Leu Pro Val Cys Ser Phe Ser
    50                  55                  60

Ser Ala Gly Pro Cys Ala Leu Arg Phe Thr Ser Ala Arg Tyr Met Glu
65                  70                  75                  80

Thr Ala Met Asn Thr Ser His His Leu Pro Arg Gln Leu Tyr Lys Trp
                85                  90                  95

Thr Leu Gly Leu Phe Val Met Ser Thr Thr Gly Val Glu Lys Tyr Phe
            100                 105                 110

Lys Asp Cys Val Phe Ala Glu Trp Glu Glu Leu Gly Asn Glu Ser Arg
        115                 120                 125

Leu Met Thr Phe Val Leu Gly Gly Cys Arg His Lys Leu Val Cys Ala
    130                 135                 140

Pro Ala Cys Asn Phe Phe Thr Ser Ala
145                 150

<210> 3
<211> 25
<212> PRT
<213> Hepatitis B virus

<400> 3
Leu Phe His Leu Cys Leu Ile Ile Ser Cys Ser Cys Pro Thr Val Gln
1               5                   10                  15

Ala Ser Lys Leu Cys Leu Gly Trp Leu
            20                  25

<210> 4
<211> 196
<212> PRT
<213> Hepatitis B virus

<400> 4
Gly Met Asp Arg Thr Thr Leu Pro Tyr Gly Leu Phe Gly Leu Asp Ile
1               5                   10                  15

Asp Pro Tyr Lys Glu Phe Gly Ala Thr Val Glu Leu Leu Ser Phe Leu
            20                  25                  30

Pro Ser Asp Phe Phe Pro Ser Val Arg Asp Leu Leu Asp Thr Ala Ser
        35                  40                  45

Ala Leu Tyr Arg Glu Ser Leu Glu Ser Ser Asp His Cys Ser Pro His
    50                  55                  60

His Thr Ala Leu Arg Gln Ala Ile Leu Cys Trp Gly Glu Leu Met Thr
65                  70                  75                  80

Leu Ala Thr Trp Val Gly Asn Asn Leu Glu Asp Pro Ala Ser Arg Asp
                85                  90                  95

Leu Val Val Asn Tyr Val Asn Thr Asn Met Gly Leu Lys Ile Arg Gln
            100                 105                 110

Leu Leu Trp Phe His Ile Ser Cys Leu Thr Phe Gly Arg Glu Thr Val
        115                 120                 125

Leu Glu Tyr Leu Val Ser Phe Gly Val Trp Ile Arg Thr Pro Pro Ala
    130                 135                 140

Tyr Arg Pro Pro Asn Ala Pro Ile Leu Ser Thr Leu Pro Glu Thr Thr
145                 150                 155                 160

Val Val Arg Arg Arg Gly Arg Ser Pro Arg Arg Arg Thr Pro Ser Pro
                165                 170                 175

Arg Arg Arg Arg Ser Gln Ser Pro Arg Arg Arg Arg Ser Ala Ser Pro
            180                 185                 190

Ala Ser Gln Cys
        195

<210> 5
<211> 399
<212> PRT
<213> Hepatitis B virus

<400> 5
Met Gly Leu Ser Trp Thr Val Pro Leu Glu Trp Gly Lys Asn Leu Ser
1               5                   10                  15

Ala Ser Asn Pro Leu Gly Phe Leu Pro Asp His Gln Leu Asp Pro Ala

            20                  25                  30

Phe Arg Ala Asn Thr Asn Asn Pro Asp Trp Asp Phe Asn Pro Lys Lys
        35                  40                  45

Asp Pro Trp Pro Glu Ala Asn Lys Val Gly Val Gly Ala Tyr Gly Pro
    50                  55                  60

Gly Phe Thr Pro Pro His Gly Gly Leu Leu Gly Trp Ser Pro Gln Ser
65                  70                  75                  80

Gln Gly Thr Leu Thr Thr Leu Pro Ala Asp Pro Pro Pro Ala Ser Thr
                85                  90                  95

Asn Arg Gln Ser Gly Arg Gln Pro Thr Pro Ile Ser Pro Pro Leu Arg
            100                 105                 110

Asp Ser His Pro Gln Ala Met Gln Trp Asn Ser Thr Ala Phe His Gln
        115                 120                 125

Ala Leu Gln Asn Pro Lys Val Arg Gly Leu Tyr Phe Pro Ala Gly Gly
    130                 135                 140

Ser Ser Ser Gly Ile Val Asn Pro Val Pro Thr Ile Ala Ser His Ile
145                 150                 155                 160

Ser Ser Ile Phe Ser Arg Ile Gly Asp Pro Ala Pro Asn Met Glu Asn
                165                 170                 175

Ile Thr Ser Gly Phe Leu Gly Pro Leu Leu Val Leu Gln Ala Gly Phe
            180                 185                 190

Phe Leu Leu Thr Arg Ile Leu Thr Ile Pro Gln Ser Leu Asp Ser Trp
        195                 200                 205

Trp Thr Ser Leu Asn Phe Leu Gly Gly Val Pro Val Cys Pro Gly Leu
    210                 215                 220

Asn Ser Gln Ser Pro Thr Ser Asn His Ser Pro Ile Ser Cys Pro Pro
225                 230                 235                 240

Thr Cys Pro Gly Tyr Arg Trp Met Cys Leu Arg Arg Phe Ile Ile Phe
                245                 250                 255

Leu Phe Ile Leu Leu Leu Cys Leu Ile Phe Leu Leu Val Leu Leu Asp
            260                 265                 270

Tyr Gln Gly Met Leu Pro Val Cys Pro Leu Ile Pro Gly Ser Ser Thr

275                 280                 285

Thr Ser Thr Gly Pro Cys Lys Thr Cys Thr Thr Pro Ala Gln Gly Asn
    290                 295                 300

Ser Met Tyr Pro Ser Cys Cys Cys Thr Lys Pro Ser Asp Gly Asn Cys
305                 310                 315                 320

Thr Cys Ile Pro Ile Pro Ser Ser Trp Ala Phe Ala Lys Tyr Leu Trp
                325                 330                 335

Glu Trp Ala Ser Val Arg Phe Ser Trp Leu Ser Leu Leu Val Pro Phe
            340                 345                 350

Val Gln Trp Phe Val Gly Leu Ser Pro Thr Val Trp Leu Ser Ala Ile
        355                 360                 365

Trp Met Met Trp Tyr Trp Gly Pro Asn Leu Tyr Asn Ile Leu Ser Pro
    370                 375                 380

Phe Ile Pro Leu Leu Pro Ile Phe Phe Cys Leu Trp Val Tyr Ile
385                 390                 395

<210> 6
<211> 842
<212> PRT
<213> Hepatitis B virus

<400> 6
Met Pro Leu Ser Tyr Gln His Phe Arg Arg Leu Leu Leu Leu Asp Glu
  1               5                  10                  15

Glu Ala Gly Pro Leu Glu Glu Glu Leu Pro Arg Leu Ala Asp Glu Asp
             20                  25                  30

Leu Asn Arg Arg Val Ala Glu Asp Leu His Leu Gln Leu Pro Asn Val
         35                  40                  45

Ser Ile Pro Trp Thr His Lys Val Gly Asn Phe Thr Gly Leu Tyr Ser
     50                  55                  60

Ser Thr Ile Pro Val Phe Asn Pro Asp Trp Gln Thr Pro Ser Phe Pro
 65                  70                  75                  80

Asn Ile His Leu His Gln Asp Ile Ile Thr Lys Cys Glu Gln Phe Val
                 85                  90                  95

Gly Pro Leu Thr Val Asn Glu Lys Arg Arg Leu Lys Leu Val Met Pro
            100                 105                 110

Ala Arg Phe Phe Pro Asn Ser Thr Lys Tyr Leu Pro Leu Asp Lys Gly
        115                 120                 125

Ile Lys Pro Tyr Tyr Pro Glu Asn Val Val Asn His Tyr Phe Gln Thr
    130                 135                 140

Arg His Tyr Leu His Thr Leu Trp Lys Ala Gly Ile Leu Tyr Lys Arg
145                 150                 155                 160

Glu Thr Ser Arg Ser Ala Ser Phe Cys Gly Ser Pro Tyr Thr Trp Glu
                165                 170                 175

Gln Asp Leu Gln His Gly Ala Phe Leu Asp Gly Pro Ser Arg Val Gly
            180                 185                 190

Lys Glu Pro Phe Arg Gln Gln Ser Ser Arg Ile Pro Ser Arg Ser Pro
        195                 200                 205

Val Gly Pro Ser Ile Gln Ser Lys Tyr Gln Gln Ser Arg Leu Gly Leu
    210                 215                 220

Gln Ser Gln Lys Gly Pro Leu Ala Arg Gly Gln Gln Gly Arg Ser Trp
225                 230                 235                 240

Ser Leu Trp Thr Arg Val His Pro Ser Thr Arg Arg Pro Phe Gly Val
                245                 250                 255

Glu Pro Ser Val Ser Gly His Thr Asn Asn Phe Ala Ser Arg Ser Ala
            260                 265                 270

Ser Cys Leu His Gln Ser Ser Val Arg Glu Ala Ala Tyr Ser His Leu
        275                 280                 285

Ser Thr Thr Lys Arg Gln Ser Ser Ser Gly His Ala Val Glu Leu Tyr
    290                 295                 300

Ser Ile Pro Pro Ser Ser Thr Lys Ser Gln Ser Gln Gly Pro Val Phe
305                 310                 315                 320

Ser Cys Trp Trp Leu Gln Phe Arg Asp Ser Glu Pro Cys Ser Asp Tyr
                325                 330                 335

Cys Leu Ser His Leu Val Asn Leu Leu Gln Asp Trp Gly Pro Cys Thr
            340                 345                 350

Glu His Gly Glu His His Ile Arg Ile Pro Arg Thr Pro Ala Arg Val
        355                 360                 365

Thr Gly Gly Val Phe Leu Val Asp Lys Asn Pro His Asn Thr Ala Glu
    370                 375                 380

Ser Arg Leu Val Val Asp Phe Ser Gln Phe Ser Arg Gly Ser Ala Arg
385                 390                 395                 400

Val Ser Trp Pro Lys Phe Ala Val Pro Asn Leu Gln Ser Leu Thr Asn
                405                 410                 415

Leu Leu Ser Ser Asn Leu Ser Trp Leu Ser Leu Asp Val Ser Ala Ala
            420                 425                 430

Phe Tyr His Ile Pro Leu His Pro Ala Ala Met Pro His Leu Leu Val
        435                 440                 445

Gly Ser Ser Gly Leu Ser Arg Tyr Val Ala Arg Leu Ser Ser Asp Ser
    450                 455                 460

Arg Ile Leu Asp His Gln Tyr Gly Thr Leu Gln Asn Leu His Asp Ser
465                 470                 475                 480

Cys Ser Arg Gln Leu Tyr Val Ser Leu Met Leu Leu Tyr Lys Thr Phe
                485                 490                 495

Gly Arg Lys Leu His Leu Tyr Ser His Pro Ile Ile Leu Gly Phe Arg
            500                 505                 510

Lys Ile Pro Met Gly Val Gly Leu Ser Pro Phe Leu Leu Ala Gln Phe
        515                 520                 525

Thr Ser Ala Ile Cys Ser Val Val Arg Arg Ala Phe Pro His Cys Leu
    530                 535                 540

Ala Phe Ser Tyr Met Asp Asp Val Val Leu Gly Ala Lys Ser Val Gln
545                 550                 555                 560

His Leu Glu Ser Leu Tyr Thr Ala Val Thr Asn Phe Leu Leu Ser Leu
                565                 570                 575

Gly Ile His Leu Asn Pro Asn Lys Thr Lys Arg Trp Gly Tyr Ser Leu
            580                 585                 590

Asn Phe Met Gly Tyr Val Ile Gly Ser Trp Gly Thr Leu Pro Gln Glu
        595                 600                 605

His Ile Thr Gln Lys Ile Lys Gln Cys Phe Arg Lys Leu Pro Val Asn
    610                 615                 620

Arg Pro Ile Asp Trp Lys Val Cys Gln Arg Ile Thr Gly Leu Leu Gly
625                 630                 635                 640

Phe Ala Ala Pro Phe Thr Gln Cys Gly Tyr Pro Ala Leu Met Pro Leu
                645                 650                 655

Tyr Ala Cys Ile Gln Ala Lys Gln Ala Phe Thr Phe Ser Pro Thr Tyr
            660                 665                 670

Lys Ala Phe Leu Cys Lys Gln Tyr Met Asn Leu Tyr Pro Val Ala Arg
        675                 680                 685

Gln Arg Pro Gly Leu Cys Gln Val Phe Ala Asp Ala Thr Pro Thr Gly
    690                 695                 700

Trp Gly Leu Ala Ile Gly His Gln Arg Met Arg Gly Thr Phe Val Ala
705                 710                 715                 720

Pro Leu Pro Ile His Thr Ala Glu Leu Leu Ala Ala Cys Phe Ala Arg
                725                 730                 735

Ser Arg Ser Gly Ala Lys Leu Ile Gly Thr Asp Asn Ser Val Val Leu
            740                 745                 750

Ser Arg Lys Tyr Thr Ser Phe Pro Trp Leu Leu Gly Cys Ala Ala Asn
        755                 760                 765

Trp Ile Leu Arg Gly Thr Ser Phe Val Tyr Val Pro Ser Ala Leu Asn
    770                 775                 780

Pro Ala Asp Asp Pro Ser Arg Gly Arg Leu Gly Leu Cys Arg Pro Leu
785                 790                 795                 800

Leu Arg Leu Pro Phe Leu Pro Thr Thr Gly Arg Thr Ser Leu Tyr Ala
                805                 810                 815

Val Ser Pro Ser Val Pro Ser His Leu Pro Asp Arg Val His Phe Ala
            820                 825                 830

Ser Pro Leu His Val Thr Trp Lys Pro Pro
        835                 840

<210> 7
<211> 675
<212> DNA
<213> Hepatitis B virus

<400> 7
atgcaacttt ttcacctctg cctaatcatc tcttgttcat gtcctactgt tcaagcctcc 60
aagctgtgcc ttgggtggct ttagggcatg gatagaacaa ctttgccata tggccttttt 120
ggcttagaca ttgaccctta taaagaattt ggagctactg tggagttgct ctcgtttttg 180
ccttctgact ttttcccgtc tgttcgtgat cttctcgaca ccgcttcagc tttgtaccgg 240
gaatccttag agtcctctga tcattgttcg cctcaccata cagcactcag gcaagcaatt 300
ctgtgctggg gtgagttgat gactctagct acctgggtgg gtaataattt ggaagatcca 360
gcatccagag atttggtggt caattatgtt aatactaata tgggtttaaa aatcaggcaa 420
ctattgtggt ttcacatttc ctgtcttact tttgggagag aaaccgttct tgagtatttg 480
gtgtcttttg gagtgtggat tcgcactcct cctgcttata gaccaccaaa tgcccctatc 540
ctatcaacac ttccggagac tactgttgtt agacgaagag gcaggtcccc tcgaagaaga 600
actccctcgc ctcgcagacg aagatctcaa tcgccgcgtc gcagaagatc tgcatctcca 660
gcttcccaat gttag 675

<210> 8
<211> 1203
<212> DNA
<213> Hepatitis B virus

<400> 8
atggggcttt cttggacggt ccctctcgag tgnnngggaa agaacctttc caccagcaat 60
cctctaggat tccttcccga tcaccagttg gacccagcat tcagagcaaa taccaacaat 120
ccagattggg acttcaatcc caaaaaggac ccttggccag nagcgaacaa ggtaggagtt 180
ggagcctatg gacccgggtt cacccctcca cacggaggcc ttttggggtg gagccctcag 240
tctcagggca cactaacaac tttgccagca gatccgcctc ctgcctccac caatcgtcag 300
tcagggaggc agcctactcc catctctcca ccactaagag acagtcatcc tcaggccatg 360
cagtggaact ctacagcatt ccaccaagct ctacaaaatc ccaaagtcag gggcctgtat 420
tttcctgctg gtggctccag ttcagggata gtgaaccctg ttccgactat tgcctctcac 480
atctcgtcaa tcttctccag gattggggac cctgcaccga acatggagaa catcacatca 540
ggattcctag gacccctgct cgtgttacag gcggggtttt tcttgttgac aagaatcctc 600
acaataccgc agagtctaga ctcgtggtgg acttctctca attttctagg gggagtgccc 660
gtgtgtcctg gcctaaattc gcagtcccca acctccaatc actcaccaat ctcctgtcct 720
ccaacttgtc ctggctatcg ctggatgtgt ctgcggcgtt ttatcatatt cctcttcatc 780
ctgctgctat gcctcatctt cttgttggtt cttctggact atcaaggtat gttgcccgtt 840
tgtcctctga ttccaggatc ctcgaccacc agtacgggac cctgcaaaac ctgcacgact 900
cctgctcaag gcaactctat gtatccctca tgttgctgta caaaaccttc ggacggaaat 960
tgcacctgta ttcccatccc atcgacttgg gctttcgcaa aatacctatg ggagtgggcc 1020
tcagtccgtt tctcttggct cagtttacta gtgccatttg ttcagtggtt cgtagggctt 1080
tcccccactg tctggctttc agctatatgg atgatgtggt actgggggcc aagtctgtac 1140
aacatcttga gtccctttat accgctgtta ccaattttct tttgtcttag ggtatacatt 1200
taa 1203

<210> 9
<211> 214
<212> PRT
<213> Hepatitis B virus

<400> 9
Met Gln Leu Phe His Leu Cys Leu Ile Ile Ser Cys Thr Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Val Glu Leu Met Thr
                85                  90                  95

Leu Ala Thr Trp Val Gly Asn Asn Leu Glu Asp Pro Ala Ser Arg Asp
            100                 105                 110

Leu Val Val Asn Tyr Val Asn Thr Asn Met Gly Leu Lys Ile Arg Gln
        115                 120                 125

Leu Leu Trp Phe His Ile Ser Cys Leu Thr Phe Gly Arg Glu Thr Val
    130                 135                 140

Leu Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Asp Arg Gly Arg Ser Pro Arg Arg Arg Thr Pro
            180                 185                 190

Ser Pro Arg Arg Arg Arg Ser Gln Ser Pro Arg Arg Arg Arg Ser Gln
        195                 200                 205

Ser Arg Glu Ser Gln Cys
    210

<210> 10
<211> 212
<212> PRT
<213> Hepatitis B virus

<400> 10
Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Ser Val Glu Leu Leu Ser Phe Leu
        35                  40                  45

Pro Ser Asp Phe Phe Pro Ser Ile Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Glu Leu Met Asn
                85                  90                  95

Leu Ala Thr Trp Val Gly Ser Asn Leu Glu Asp Pro Ala Ser Arg Glu
            100                 105                 110

Leu Val Val Ser Tyr Val Asn Val Asn Met Gly Leu Lys Ile Arg Gln
        115                 120                 125

Leu Leu Trp Phe His Ile Ser Cys Leu Thr Phe Gly Arg Glu Thr Val
    130                 135                 140

Leu Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210

<210> 11
<211> 212
<212> PRT
<213> Hepatitis B virus

<400> 11
Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Ser Val Glu Leu Leu Ser Phe Leu
        35                  40                  45

Pro Ser Asp Phe Phe Pro Ser Ile Arg Asp Leu Leu Asp Thr Ala Ser
    50                  55                  60

Ala Leu Tyr Arg Glu Ala Leu Glu Ser Pro Glu His Cys Ser Pro His
65                  70                  75                  80

His Thr Ala Leu Arg Gln Ala Ile Leu Cys Trp Gly Glu Leu Met Asn
                85                  90                  95

Leu Ala Thr Trp Val Gly Ser Asn Leu Glu Asp Pro Ala Ser Arg Glu
            100                 105                 110

Leu Val Val Ser Tyr Val Asn Val Asn Met Gly Leu Lys Ile Arg Gln
        115                 120                 125

Leu Leu Trp Phe His Ile Ser Cys Leu Thr Phe Gly Arg Glu Thr Val
    130                 135                 140

Leu Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg

195                 200                 205

Glu Ser Gln Cys
    210



<210> 12
<211> 212
<212> PRT
<213> Hepatitis B virus

<400> 12
Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Asp Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Val Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Arg
        195                 200                 205

Glu Ser Gln Cys
    210

<210> 13
<211> 212
<212> PRT
<213> Hepatitis B virus

<400> 13
Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Thr Val Glu Leu Leu Ser Phe Leu
        35                  40                  45

Pro Ser Asp Phe Phe Pro Ser Val Arg Asp Leu Leu Asp Thr Ala Ser
    50                  55                  60

Ala Leu Tyr Arg Asp Ala Leu Glu Ser Pro Glu His Cys Ser Pro His
65                  70                  75                  80

His Thr Ala Leu Arg Gln Ala Ile Leu Cys Trp Gly Glu Leu Met Thr
                85                  90                  95

Leu Ala Thr Trp Val Gly Val Asn Leu Glu Asp Pro Ala Ser Arg Asp
            100                 105                 110

Leu Val Val Ser Tyr Val Asn Thr Asn Met Gly Leu Lys Phe Arg Gln
        115                 120                 125

Leu Leu Trp Phe His Ile Ser Cys Leu Thr Phe Gly Arg Glu Thr Val
    130                 135                 140

Ile Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Asn Thr Thr
                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Pro
        195                 200                 205

Ala Ser Gln Cys
    210

<210> 14
<211> 212
<212> PRT
<213> Hepatitis B virus

<400> 14
Met Gln Leu Phe His Leu Cys Leu Ile Ile Ser Cys Ser Cys Pro Thr
1               5                   10                  15

Val Gln Ala Ser Lys Leu Cys Leu Gly Trp Leu Trp Gly Met Asp Ile
            20                  25                  30

Asp Pro Tyr Lys Glu Phe Gly Ala Ser Val Glu Leu Phe Ser Phe Leu
        35                  40                  45

Ala Ser Asp Phe Phe Pro Ser Val Arg Asp Leu Leu Asp Thr Ala Ser
    50                  55                  60

Ala Leu Tyr Arg Asp Ala Leu Glu Ser Pro Glu His Cys Thr Pro Asn
65                  70                  75                  80

His Thr Ala Leu Arg Gln Ala Ile Leu Cys Trp Gly Glu Leu Met Thr
                85                  90                  95

Leu Ala Ser Trp Val Gly Asn Asn Leu Glu Asp Pro Ala Ala Arg Asp
            100                 105                 110

Leu Val Val Asn Tyr Val Asn Thr Asn Met Gly Leu Lys Ile Arg Gln
        115                 120                 125

Leu Leu Trp Phe His Ile Ser Cys Leu Thr Phe Gly Arg Glu Thr Val
    130                 135                 140

Leu Glu Tyr Leu Val Ser Phe Gly Val Trp Ile Arg Thr Pro Pro Ala
145                 150                 155                 160

Tyr Arg Pro Pro Asn Ala Pro Ile Leu Ser Thr Leu Pro Glu Thr Thr

                165                 170                 175

Val Val Arg Arg Arg Gly Arg Ser Pro Arg Arg Arg Thr Pro Ser Pro
            180                 185                 190

Arg Arg Arg Arg Ser Gln Ser Pro Arg Arg Arg Arg Ser Gln Ser Pro
        195                 200                 205

Ala Ser Gln Cys
    210

<210> 15
<211> 400
<212> PRT
<213> Hepatitis B virus

<400> 15
Met Gly Gly Trp Ser Ser Lys Pro Arg Lys Gly Met Gly Thr Asn Leu
1               5                   10                  15

Ser Val Pro Asn Pro Leu Gly Phe Phe Pro Asp His Gln Leu Asp Pro
            20                  25                  30

Ala Phe Gly Ala Asn Ser Asn Asn Pro Asp Trp Asp Phe Asn Pro Ile
        35                  40                  45

Lys Asp His Trp Pro Ala Ala Asn Gln Val Gly Val Gly Ala Phe Gly
    50                  55                  60

Pro Arg Leu Thr Pro Pro His Gly Gly Ile Leu Gly Trp Ser Pro Gln
65                  70                  75                  80

Ala Gln Gly Ile Leu Thr Thr Val Ser Thr Ile Pro Pro Pro Ala Ser
                85                  90                  95

Thr Asn Arg Gln Ser Gly Arg Gln Pro Thr Pro Ile Ser Pro Pro Leu
            100                 105                 110

Arg Asp Ser His Pro Gln Ala Met Gln Trp Asn Ser Thr Ala Phe His
        115                 120                 125

Gln Ala Leu Gln Asp Pro Lys Val Arg Gly Leu Tyr Leu Pro Ala Gly
    130                 135                 140

Gly Ser Ser Ser Gly Thr Val Asn Pro Ala Pro Asn Ile Ala Ser His
145                 150                 155                 160

Ile Ser Ser Ile Ser Ala Arg Thr Gly Asp Pro Val Thr Asn Met Glu
                165                 170                 175

Asn Ile Thr Ser Gly Phe Leu Gly Pro Leu Leu Val Leu Gln Ala Gly
            180                 185                 190

Phe Phe Leu Leu Thr Arg Ile Leu Thr Ile Pro Gln Ser Leu Asp Ser
        195                 200                 205

Trp Trp Thr Ser Leu Asn Phe Leu Gly Gly Ser Pro Val Cys Leu Gly
    210                 215                 220

Gln Asn Ser Gln Ser Pro Thr Ser Asn His Ser Pro Thr Ser Cys Pro
225                 230                 235                 240

Pro Ile Cys Pro Gly Tyr Arg Trp Met Cys Leu Arg Arg Phe Ile Ile
                245                 250                 255

Phe Leu Phe Ile Leu Leu Leu Cys Leu Ile Phe Leu Leu Val Leu Leu
            260                 265                 270

Asp Tyr Gln Gly Met Leu Pro Val Cys Pro Leu Ile Pro Gly Ser Thr
        275                 280                 285

Thr Thr Ser Thr Gly Pro Cys Lys Thr Cys Thr Thr Pro Ala Gln Gly
    290                 295                 300

Asn Ser Met Phe Pro Ser Cys Cys Cys Thr Lys Pro Thr Asp Gly Asn
305                 310                 315                 320

Cys Thr Cys Ile Pro Ile Pro Ser Ser Trp Ala Phe Ala Lys Tyr Leu
                325                 330                 335

Trp Glu Trp Ala Ser Val Arg Phe Ser Trp Leu Ser Leu Leu Val Pro
            340                 345                 350

Phe Val Gln Trp Phe Val Gly Leu Ser Pro Thr Val Trp Leu Ser Ala
        355                 360                 365

Ile Trp Met Met Trp Tyr Trp Gly Pro Arg Leu Tyr Ser Ile Val Ser
    370                 375                 380

Pro Phe Ile Pro Leu Leu Pro Ile Phe Phe Cys Leu Trp Val Tyr Ile
385                 390                 395                 400

<210> 16
<211> 400
<212> PRT
<213> Hepatitis B virus

<400> 16
Met Gly Gly Trp Ser Ser Lys Pro Arg Gln Gly Met Gly Thr Asn Leu
1               5                   10                  15

Ser Val Pro Asn Pro Leu Gly Phe Phe Pro Asp His Gln Leu Asp Pro
            20                  25                  30

Ala Phe Lys Ala Asn Ser Asp Asn Pro Asp Trp Asp Leu Asn Pro His
        35                  40                  45

Lys Asp Asn Trp Pro Asp Ser Asn Lys Val Gly Val Gly Ala Phe Gly
    50                  55                  60

Pro Gly Phe Thr Pro Pro His Gly Gly Leu Leu Gly Trp Ser Pro Gln
65                  70                  75                  80

Ala Gln Gly Ile Leu Thr Thr Val Pro Thr Ala Pro Pro Pro Ala Ser
                85                  90                  95

Thr Asn Arg Gln Leu Gly Arg Lys Pro Thr Pro Leu Ser Pro Pro Leu
            100                 105                 110

Arg Asp Thr His Pro Gln Ala Met Gln Trp Asn Ser Thr Thr Phe His
        115                 120                 125

Gln Thr Leu Gln Asp Pro Arg Val Arg Ala Leu Tyr Phe Pro Ala Gly
    130                 135                 140

Gly Ser Ser Ser Gly Thr Val Asn Pro Val Gln Asn Thr Ala Ser Ser
145                 150                 155                 160

Ile Ser Ser Ile Leu Ser Thr Thr Gly Asp Pro Val Pro Asn Met Glu
                165                 170                 175

Asn Ile Ala Ser Gly Leu Leu Gly Pro Leu Leu Val Leu Gln Ala Gly
            180                 185                 190

Phe Phe Ser Leu Thr Lys Ile Leu Thr Ile Pro Leu Ser Leu Asp Ser
        195                 200                 205

Trp Trp Thr Ser Leu Asn Phe Leu Gly Glu Thr Pro Val Cys Leu Gly
    210                 215                 220

Gln Asn Ser Gln Ser Gln Ile Ser Ser His Ser Pro Thr Cys Cys Pro
225                 230                 235                 240

Pro Ile Cys Pro Gly Tyr Arg Trp Met Cys Leu Arg Arg Phe Ile Ile
                245                 250                 255

Phe Leu Cys Ile Leu Leu Leu Cys Leu Ile Phe Leu Leu Val Leu Leu
            260                 265                 270

Asp Tyr Gln Gly Met Leu Pro Val Cys Pro Leu Ile Pro Gly Ser Ser
        275                 280                 285

Thr Thr Ser Thr Gly Pro Cys Lys Thr Cys Thr Thr Pro Ala Gln Gly
    290                 295                 300

Thr Ser Met Phe Pro Ser Cys Cys Cys Thr Lys Pro Thr Asp Gly Asn
305                 310                 315                 320

Cys Thr Cys Ile Pro Ile Pro Ser Ser Trp Ala Phe Ala Lys Tyr Leu
                325                 330                 335

Trp Glu Trp Ala Ser Val Arg Phe Ser Trp Leu Ser Leu Leu Val Pro
            340                 345                 350

Phe Val Gln Trp Phe Val Gly Leu Ser Pro Thr Val Trp Leu Ser Val
        355                 360                 365

Ile Trp Met Met Trp Phe Trp Gly Pro Ser Leu Tyr Asn Ile Leu Ser
    370                 375                 380

Pro Phe Met Pro Leu Leu Pro Ile Phe Phe Cys Leu Trp Val Tyr Ile
385                 390                 395                 400

<210> 17
<211> 400
<212> PRT
<213> Hepatitis B virus

<400> 17
Met Gly Gly Trp Ser Ser Lys Pro Arg Gln Gly Met Gly Thr Asn Leu

1               5                   10                  15

Ser Val Pro Asn Pro Leu Gly Phe Phe Pro Asp His Gln Leu Asp Pro
            20                  25                  30

Ala Phe Gly Ala Asn Ser His Asn Pro Asp Trp Asp Phe Asn Pro Asn
        35                  40                  45

Lys Asp His Trp Pro Glu Ala Asn Gln Val Gly Ala Gly Ala Phe Gly
    50                  55                  60

Pro Gly Phe Thr Pro Pro His Gly Gly Leu Leu Gly Trp Ser Pro Gln
65                  70                  75                  80

Ala Gln Gly Val Leu Thr Thr Val Pro Val Ala Pro Pro Pro Ala Ser
                85                  90                  95

Thr Asn Arg Gln Ser Gly Arg Gln Pro Thr Pro Ile Ser Pro Pro Leu
            100                 105                 110

Arg Asp Ser His Pro Gln Ala Met Gln Trp Asn Ser Thr Thr Phe His
        115                 120                 125

Gln Ala Leu Leu Asp Pro Arg Val Arg Gly Leu Tyr Phe Pro Ala Gly
    130                 135                 140

Gly Ser Ser Ser Gly Thr Val Asn Pro Val Pro Thr Thr Ala Ser Pro
145                 150                 155                 160

Ile Ser Ser Ile Ser Ser Arg Thr Gly Asp Pro Ala Pro Asn Met Glu
                165                 170                 175

Asn Thr Thr Ser Gly Phe Leu Gly Pro Leu Leu Val Leu Gln Ala Gly
            180                 185                 190

Phe Phe Leu Leu Thr Arg Ile Leu Thr Ile Pro Gln Ser Leu Asp Ser
        195                 200                 205

Trp Trp Thr Ser Leu Asn Phe Leu Gly Gly Ala Pro Thr Cys Pro Gly
    210                 215                 220

Gln Asn Ser Gln Ser Pro Thr Ser Asn His Ser Pro Thr Ser Cys Pro
225                 230                 235                 240

Pro Ile Cys Pro Gly Tyr Arg Trp Met Cys Leu Arg Arg Phe Ile Ile
                245                 250                 255

Phe Leu Phe Ile Leu Leu Leu Cys Leu Ile Phe Leu Leu Val Leu Leu

            260                 265                 270

Asp Tyr Gln Gly Met Leu Pro Val Cys Pro Leu Leu Pro Gly Thr Ser
        275                 280                 285

Thr Thr Ser Thr Gly Pro Cys Lys Thr Cys Thr Ile Pro Ala Gln Gly
    290                 295                 300

Thr Ser Met Phe Pro Ser Cys Cys Cys Thr Lys Pro Ser Asp Gly Asn
305                 310                 315                 320

Cys Thr Cys Ile Pro Ile Pro Ser Ser Trp Ala Phe Ala Arg Phe Leu
                325                 330                 335

Trp Glu Gly Ala Ser Val Arg Phe Ser Trp Leu Ser Leu Leu Val Pro
            340                 345                 350

Phe Val Gln Trp Phe Val Gly Leu Ser Pro Thr Val Trp Leu Ser Val
        355                 360                 365

Ile Trp Met Met Trp Tyr Trp Gly Pro Ser Leu Tyr Asn Ile Leu Ser
    370                 375                 380

Pro Phe Leu Pro Leu Leu Pro Ile Phe Phe Cys Leu Trp Val Tyr Ile
385                 390                 395                 400

<210> 18
<211> 389
<212> PRT
<213> Hepatitis B virus

<400> 18
Met Gly Gln Asn Leu Ser Thr Ser Asn Pro Leu Gly Phe Phe Pro Asp
1               5                   10                  15

His Gln Leu Asp Pro Ala Phe Arg Ala Asn Thr Ala Asn Pro Asp Trp
            20                  25                  30

Asp Phe Asn Pro Asn Lys Asp Thr Trp Pro Asp Ala Asn Lys Val Gly
        35                  40                  45

Ala Gly Ala Phe Gly Leu Gly Phe Thr Pro Pro His Gly Gly Leu Leu
    50                  55                  60

Gly Trp Ser Pro Gln Ala Gln Gly Ile Ile Gln Thr Leu Pro Ala Asn
65                  70                  75                  80

Pro Pro Pro Ala Ser Thr Asn Arg Gln Ser Gly Arg Gln Pro Thr Pro
                85                  90                  95

Leu Ser Pro Pro Leu Arg Asn Thr His Pro Gln Ala Met Gln Trp Asn
            100                 105                 110

Ser Thr Thr Phe His Gln Thr Leu Gln Asp Pro Arg Val Arg Gly Leu
        115                 120                 125

Tyr Phe Pro Ala Gly Gly Ser Ser Ser Gly Thr Val Asn Pro Val Pro
    130                 135                 140

Thr Thr Ala Ser Pro Ile Ser Ser Ile Phe Ser Arg Ile Gly Asp Pro
145                 150                 155                 160

Ala Leu Asn Met Glu Asn Ile Thr Ser Gly Phe Leu Gly Pro Leu Leu
                165                 170                 175

Val Leu Gln Ala Gly Phe Phe Leu Leu Thr Arg Ile Leu Thr Ile Pro
            180                 185                 190

Gln Ser Leu Asp Ser Trp Trp Thr Ser Leu Asn Phe Leu Gly Gly Thr
        195                 200                 205

Thr Val Cys Leu Gly Gln Asn Ser Gln Ser Pro Thr Ser Asn His Ser
    210                 215                 220

Pro Thr Ser Cys Pro Pro Thr Cys Pro Gly Tyr Arg Trp Met Cys Leu
225                 230                 235                 240

Arg Arg Phe Ile Ile Phe Leu Phe Ile Leu Leu Leu Cys Leu Ile Phe
                245                 250                 255

Leu Leu Val Leu Leu Asp Tyr Gln Gly Met Leu Pro Val Cys Pro Leu
            260                 265                 270

Ile Pro Gly Ser Ser Thr Thr Ser Ala Gly Pro Cys Arg Thr Cys Thr
        275                 280                 285

Thr Thr Ala Gln Gly Thr Ser Met Tyr Pro Ser Cys Cys Cys Thr Lys
    290                 295                 300

Pro Ser Asp Gly Asn Cys Thr Cys Ile Pro Ile Pro Ser Ser Trp Ala
305                 310                 315                 320

Phe Gly Lys Phe Leu Trp Glu Trp Ala Ser Ala Arg Phe Ser Trp Leu
                325                 330                 335

Ser Leu Leu Val Pro Phe Val Gln Trp Phe Val Gly Leu Ser Pro Thr
            340                 345                 350

Val Trp Leu Ser Val Ile Trp Met Met Trp Tyr Trp Gly Pro Ser Leu
        355                 360                 365

Tyr Ser Ile Leu Ser Pro Phe Leu Pro Leu Leu Pro Ile Phe Phe Cys
    370                 375                 380

Leu Trp Val Tyr Ile
385

<210> 19
<211> 399
<212> PRT
<213> Hepatitis B virus

<400> 19
Met Gly Leu Ser Trp Thr Val Pro Leu Glu Trp Gly Lys Asn Ile Ser
  1               5                  10                  15

Thr Thr Asn Pro Leu Gly Phe Phe Pro Asp His Gln Leu Asp Pro Ala
             20                  25                  30

Phe Arg Ala Asn Thr Arg Asn Pro Asp Trp Asp His Asn Pro Asn Lys
         35                  40                  45

Asp His Trp Thr Glu Ala Asn Lys Val Gly Val Gly Ala Phe Gly Pro
     50                  55                  60

Gly Phe Thr Pro Pro His Gly Gly Leu Leu Gly Trp Ser Pro Gln Ala
 65                  70                  75                  80

Gln Gly Met Leu Lys Thr Leu Pro Ala Asp Pro Pro Pro Ala Ser Thr
                 85                  90                  95

Asn Arg Gln Ser Gly Arg Gln Pro Thr Pro Ile Thr Pro Pro Leu Arg
            100                 105                 110

Asp Thr His Pro Gln Ala Met Gln Trp Asn Ser Thr Thr Phe His Gln
        115                 120                 125

Ala Leu Gln Asp Pro Arg Val Arg Gly Leu Tyr Phe Pro Ala Gly Gly
    130                 135                 140

Ser Ser Ser Gly Thr Val Asn Pro Val Pro Thr Thr Ala Ser Leu Ile
145                 150                 155                 160

Ser Ser Ile Phe Ser Arg Ile Gly Asp Pro Ala Pro Asn Met Glu Ser
                165                 170                 175

Ile Thr Ser Gly Phe Leu Gly Pro Leu Leu Val Leu Gln Ala Gly Phe
            180                 185                 190

Phe Leu Leu Thr Lys Ile Leu Thr Ile Pro Gln Ser Leu Asp Ser Trp
        195                 200                 205

Trp Thr Ser Leu Asn Phe Leu Gly Gly Ala Pro Val Cys Leu Gly Gln
    210                 215                 220

Asn Ser Gln Ser Pro Thr Ser Asn His Ser Pro Thr Ser Cys Pro Pro
225                 230                 235                 240

Ile Cys Pro Gly Tyr Arg Trp Met Cys Leu Arg Arg Phe Ile Ile Phe
                245                 250                 255

Leu Phe Ile Leu Leu Leu Cys Leu Ile Phe Leu Leu Val Leu Leu Asp
            260                 265                 270

Tyr Gln Gly Met Leu Pro Val Cys Pro Leu Ile Pro Gly Ser Ser Thr
        275                 280                 285

Thr Ser Thr Gly Pro Cys Arg Thr Cys Met Thr Leu Ala Gln Gly Thr
    290                 295                 300

Ser Met Phe Pro Ser Cys Cys Cys Ser Lys Pro Ser Asp Gly Asn Cys
305                 310                 315                 320

Thr Cys Ile Pro Ile Pro Ser Ser Trp Ala Phe Gly Lys Phe Leu Trp
                325                 330                 335

Glu Trp Ala Ser Ala Arg Phe Ser Trp Leu Ser Leu Leu Val Pro Phe
            340                 345                 350

Val Gln Trp Phe Ala Gly Leu Ser Pro Thr Val Trp Leu Ser Val Ile
        355                 360                 365

Trp Met Met Trp Tyr Trp Gly Pro Ser Leu Tyr Asp Ile Leu Ser Pro
    370                 375                 380

Phe Ile Pro Leu Leu Pro Ile Phe Phe Cys Leu Trp Val Tyr Ile
385                 390                 395

<210> 20
<211> 400
<212> PRT
<213> Hepatitis B virus

<400> 20
Met Gly Ala Pro Leu Ser Thr Thr Arg Arg Gly Met Gly Gln Asn Leu
1               5                   10                  15

Ser Val Pro Asn Pro Leu Gly Phe Leu Pro Asp His Gln Leu Asp Pro
            20                  25                  30

Leu Phe Arg Ala Asn Ser Ser Ser Pro Asp Trp Asp Phe Asn Thr Asn
        35                  40                  45

Lys Asp Ser Trp Pro Met Ala Asn Lys Val Gly Val Gly Gly Tyr Gly
    50                  55                  60

Pro Gly Phe Thr Pro Pro His Gly Gly Leu Leu Gly Trp Ser Pro Gln
65                  70                  75                  80

Ala Gln Gly Val Leu Thr Thr Leu Pro Ala Asp Pro Pro Pro Ala Ser
                85                  90                  95

Thr Asn Arg Leu Ser Gly Arg Lys Pro Thr Gln Val Ser Pro Pro Leu
            100                 105                 110

Arg Asp Thr His Pro Gln Ala Met Gln Trp Asn Ser Thr His Phe His
        115                 120                 125

Gln Ala Leu Leu Asp Pro Arg Val Arg Ala Leu Tyr Phe Pro Ala Gly
    130                 135                 140

Gly Ser Ser Ser Gly Thr Gln Asn Pro Ala Pro Thr Ile Ala Ser Leu
145                 150                 155                 160

Thr Ser Ser Ile Ser Ser Lys Thr Gly Gly Pro Ala Met Asn Met Glu
                165                 170                 175

Asn Ile Thr Ser Gly Leu Leu Gly Pro Leu Arg Val Leu Gln Ala Val
            180                 185                 190

Cys Phe Leu Leu Thr Lys Ile Leu Thr Ile Pro Gln Ser Leu Asp Ser

195                 200                 205

Trp Trp Thr Ser Leu Asn Phe Leu Gly Gly Leu Pro Arg Cys Pro Gly
    210                 215                 220

Gln Asn Ser Gln Ser Pro Thr Ser Asn His Leu Pro Thr Ser Cys Pro
225                 230                 235                 240

Pro Thr Cys Pro Gly Tyr Arg Trp Met Cys Leu Arg Arg Phe Ile Ile
                245                 250                 255

Phe Leu Phe Ile Leu Leu Leu Cys Leu Ile Phe Leu Leu Val Leu Leu
            260                 265                 270

Asp Tyr Gln Gly Met Leu Pro Val Cys Pro Leu Leu Pro Gly Ser Thr
        275                 280                 285

Thr Thr Ser Thr Gly Pro Cys Lys Thr Cys Thr Ala Leu Ala Gln Gly
    290                 295                 300

Thr Ser Met Phe Pro Ser Cys Cys Cys Ser Lys Pro Ser Asp Gly Asn
305                 310                 315                 320

Cys Thr Cys Ile Pro Ile Pro Ser Ser Trp Ala Leu Gly Lys Tyr Leu
                325                 330                 335

Trp Glu Trp Ala Ser Ala Arg Phe Ser Trp Leu Ser Leu Leu Val Gln
            340                 345                 350

Phe Val Gln Trp Cys Val Gly Leu Ser Pro Thr Val Trp Leu Leu Val
        355                 360                 365

Ile Trp Met Ile Trp Tyr Trp Gly Pro Asn Leu Cys Ser Ile Leu Ser
    370                 375                 380

Pro Phe Ile Pro Leu Leu Pro Ile Phe Cys Tyr Leu Trp Val Ser Ile
385                 390                 395                 400

<210> 21
<211> 19
<212> DNA
<213> Hepatitis B virus

<400> 21
gggtcaccat attcttggg                                                    19

<210> 22
<211> 21
<212> DNA
<213> Hepatitis B virus

<400> 22
gaacaagagc tacagcatgg g                                                 21

<210> 23
<211> 21
<212> DNA
<213> Hepatitis B virus

<400> 23
ccactgcatg gcctgaggat g                                                 21

<210> 24
<211> 21
<212> DNA
<213> Hepatitis B virus

<400> 24
ctccacagwa gctccaaatt c                                                 21

<210> 25
<211> 17
<212> DNA
<213> Hepatitis B virus

<400> 25
tggggtggag ccctcag                                                      17

<210> 26
<211> 17
<212> DNA
<213> Hepatitis B virus

<400> 26
ctgagggctc cacccca                                                      17

<210> 27
<211> 23
<212> DNA
<213> Hepatitis B virus

<400> 27
caaggtatgt tgcccgtttg tcc                                              23

<210> 28
<211> 20
<212> DNA
<213> Hepatitis B virus

<400> 28
acataagagg actcttggac                                                  20

<210> 29
<211> 22
<212> DNA
<213> Hepatitis B virus

<400> 29
tacttcaaag actgtgtgtt ta                                               22

<210> 30
<211> 21
<212> DNA
<213> Hepatitis B virus

<400> 30
ggtawaaagg gactcamgat g                                                21

<210> 31
<211> 20
<212> DNA
<213> Hepatitis B virus

<400> 31
tttttcacct ctgcctaatc                                                  20

<210> 32
<211> 20

<212> DNA
<213> Hepatitis B virus

<400> 32
aaaaagttgc atggtgctgg                                                    20

<210> 33
<211> 25
<212> DNA
<213> Hepatitis B virus

<400> 33
cctctgccga tccatactgc ggaac                                              25

<210> 34
<211> 25
<212> DNA
<213> Hepatitis B virus

<400> 34
ctgcgaggcg agggagttct tcttc                                              25

<210> 35
<211> 25
<212> DNA
<213> Hepatitis B virus

<400> 35
ccggcagatg agaaggcaca gacgg                                              25

<210> 36
<211> 20
<212> DNA
<213> Hepatitis B virus

<400> 36
tgctgctatg cctcatcttc                                                    20

<210> 37
<211> 20
<212> DNA
<213> Hepatitis B virus

<400> 37
caragacaaa agaaaattgg                                                    20

<210> 38
<211> 20
<212> DNA
<213> Hepatitis B virus

<400> 38
cccaccttat gagtccaagg                                                    20

<210> 39
<211> 25
<212> DNA
<213> Hepatitis B virus

<400> 39
gttccgcagt atggatcggc agagg                                              25

<210> 40
<211> 21
<212> DNA
<213> Hepatitis B virus

<400> 40
tatggatgat gtggtattgg g                                                  21

<210> 41
<211> 20
<212> DNA
<213> Hepatitis B virus

<400> 41
ggagtgtgga ttcgcactcc                                                    20

<210> 42
<211> 21
<212> DNA
<213> Hepatitis B virus

<400> 42
cccatgctgt agctcttgtt c                                                  21

<210> 43
<211> 645
<212> DNA
<213> Hepatitis B virus

<400> 43
atgcaacttt ttcacctctg cctaatcatc tcttgtacat gtcccactgt tcaagcctcc 60
aagctgtgcc ttgggtggct ttggggcatg gacattgacc cttataaaga atttggagct 120
actgtggagt tactctcgtt tttgccttct gacttctttc cttccgtcag agatctccta 180
gacaccgcct cagctctgta tcgagaagcc ttagagtctc ctgagcattg ctcacctcac 240
catactgcac tcaggcaagc cattctctgc tgggtggaat tgatgactct agctacctgg 300
gtgggtaata atttggaaga tccagcatcc agggatctag tagtcaatta tgttaatact 360
aacatgggtt taaagatcag gcaactattg tggtttcata tatcttgcct tacttttgga 420
agagagactg tgcttgaata tttggtctct ttcggagtgt ggattcgcac tcctccagcc 480
tatagaccac caaatgcccc tatcttatca acacttccgg aaactactgt tgttagacga 540
cgggaccgag gcaggtcccc tagaagaaga actccctcgc ctcgcagacg cagatctcaa 600
tcgccgcgtc gcagaagatc tcaatctcgg gaatctcaat gttag 645

<210> 44
<211> 675
<212> DNA
<213> Hepatitis B virus

<400> 44
atgtaacttt ttcacctctg cctaatcatc tcttgttcat gtcctactgt tcaagcctcc 60
aagctgtgcc ttgggtggct ttagggcatg gatagaacaa ctttgccata tggccttttt 120
ggcttagaca ttgaccctta taaagaattt ggagctactg tggagttgct ctcgtttttg 180
ccttctgact ttttcccgtc tgttcgtgat cttctcgaca ccgcttcagc tttgtaccgg 240
gaatccttag agtcctctga tcattgttcg cctcaccata cagcactcag gcaagcaatc 300
ctgtgctggg gtgagttgat gactctagct acctgggtgg gtaataattt ggaagatcca 360
gcatccagag atttggtggt caattatgtt aatactaata tgggtttaaa aatcaggcaa 420
ctattgtggt ttcacatttc ctgtcttact tttgggagag aaaccgttct tgagtatttg 480
gtgtcttttg gagtgtggat tcgcactcct cctgcttata gaccaccaaa tgcccctatc 540
ctatcaacac ttccggagac tactgttgtt agacgaagag gcaggtcccc tcgaagaaga 600
actccctcgc ctcgcagacg aagatctcaa tcgccgcgtc gcagaagatc tgcatctcca 660
gcttcccaat gttag 675

<210> 45
<211> 649
<212> DNA
<213> Hepatitis B virus

<400> 45
atgtaacttt ttcacctctg cctaatcatc tcttgttcat gtcctactgt tcaagcctcc 60
aagctgtgcc ttgggtggct ttagggcatg gatagaacaa ctttgccata tggccttttt 120

ggcttagaca ttgaccctta taaagaattt ggagctactg tggagttgct ctcgtttttg 180
ccttctgact ttttcccgtc tgttcgtgat cttctcgaca ccgcttcagc tttgtaccgg 240
gaatccttag agtcctctga tcattgttcg cctcaccata cagcactcag gcaagcaatc 300
ctgtgctggg gtgagttgat gactctagct acctgggtgg gtaataattt ggaagatcca 360
gcatccagag atttggtggt caattatgtt aatactaata tgggtttaaa aatcaggcaa 420
ctattgtggt ttcacatttc ctgtcttact tttgggagag aaaccgttct tgagtatttg 480
gtgtcttttg gagtgtggat tcgcactcct cctgcttata gaccaccaaa tgcccctatc 540
ctatcaacac ttccggagac tactgttgtt agacgaagag gcaggtcccc tcgaagaaga 600
actccctcgc ctcgcagacg aagatctcaa tcgccgcgtc gcagaagat 649

<210> 46
<211> 649
<212> DNA
<213> Hepatitis B virus

<400> 46
atgtaacttt ttcacctctg cctaatcatc tcttgttcat gtcctactgt tcaagcctcc 60
aagctgtgcc ttgggtggct ttagggcatg gatagaacaa ctttgccata tggccttttt 120
ggcttagaca ttgaccctta taaagaattt ggagctactg tggagttgct ctcgtttttg 180
ccttctgact ttttcccgtc tgttcgtgat cttctcgaca ccgcttcagc tttgtaccgg 240
gaatccttag agtcctctga tcattgttcg cctcaccata cagcactcag gcaagcaatc 300
ctgtgctggg gtgagttgat gactctagct acctgggtgg gtaataattt ggaagatcca 360
gcatccagag atttggtggt caattatgtt aatactaata tgggtttaaa aatcaggcaa 420
ctattgtggt ttcacatttc ctgtcttact tttgggagag aaaccgttct tgagtatttg 480
gtgtcttttg gagtgtggat tcgcactcct cctgcttata gaccaccaaa tgcccctatc 540
ctatcaacac ttccggagac tactgttgtt agacgaagag gcaggtcccc tcgaagaaga 600
actccctcgc ctcgcagacg aagatctcaa tcgccgcgtc gcagaagat 649

<210> 47
<211> 649
<212> DNA
<213> Hepatitis B virus

<400> 47
atgtaacttt ttcacctctg cctaatcatc tcttgttcat gtcctactgt tcaagcctcc 60
aagctgtgcc ttgggtggct ttagggcatg gatagaacaa ctttgccata tggccttttt 120
ggcttagaca ttgaccctta taaagaattt ggagctactg tggagttgct ctcgtttttg 180
ccttctgact ttttcccgtc tgttcgtgat cttctcgaca ccgcttcagc tttgtaccgg 240
gaatccttag agtcctctga tcattgttcg cctcaccata cagcactcag gcaagcaatc 300
ctgtgctggg gtgagttgat gactctagct acctgggtgg gtaataattt ggaagatcca 360
gcatccagag atttggtggt caattatgtt aatactaata tgggtttaaa aatcaggcaa 420
ctattgtggt ttcacatttc ctgtcttact tttgggagag aaaccgttct tgagtatttg 480
gtgtcttttg gagtgtggat tcgcactcct cctgcttata gaccaccaaa tgcccctatc 540
ctatcaacac ttccggagac tactgttgtt agacgaagag gcaggtcccc tcgaagaaga 600
actccctcgc ctcgcagacg aagatctcaa tcgccgcgtc gcagaagat 649

<210> 48
<211> 649
<212> DNA
<213> Hepatitis B virus

<400> 48
atgyaacttt ttcacctctg cctaatcatc tcttgttcat gtcctactgt tcaagcctcc 60
aagctgtgcc ttgggtggct ttagggcatg gatagaacaa ctttgccata tggcctttct 120
ggcttagaca ttgaccctta taaagaattt ggagctactg tggagttgct ctcgtttttg 180
ccttctgact tttttccgtc tgttcgtgat cttctcgaca ccgcttcagc tttgtaccgg 240
gaatccttag agtcctttga tcattgttcg cctcaccata cagcactcag gcaagcaatc 300
ctgtgctggg gtragttgat ractctagct acctgggtgg gtaataattt ggaagatcca 360
gcatccagag atttggtggt crattatgtt aataytaata tgggtttaaa aatcaggcar 420
cgattgtggw ttcacatctc ctgtcttact wtwgggasag aamccgttct tgagtattyg 480
gtgtcwktkg gagtgtgsat tcgcactcct cmtgcttata gaccaccaaa tgccccwatc 540
cyatcaacac ttccggagrc trctgttgtt agrcgaagag gcaggtcccc tcgaagaaga 600
actccctcgc ctcgcagacg aagatctcaa tcgccgcgtc gcagaagat 649

<210> 49
<211> 658
<212> DNA
<213> Hepatitis B virus

<400> 49
atgtaacttt ttcacctctg cctaatcatc tcttgttcat gtcctactgt tcaagcctcc 60
aagctgtgcc ttgggtggct ttagggcatg gatagaacaa ctttgccata tggccttttt 120
ggcttagaca ttgaccctta taaagaattt ggagctactg tggagttgct ctcgtttttg 180
ccttctgact ttttcccgtc tgttcgtgat cttctcgaca ccgcttcagc tttgtaccgg 240
gaatccttag agtcctctga tcattgttcg cctcaccata cagcactcag gcaagcaatc 300
ctgtgctggg gtgagttgat gactctagcc acctgggtgg gtaataattt ggaagatcca 360
gcatccagag atttggtggt caattatgtt aatactaata tgggtttaaa aatcaggcaa 420
ctattgtggt ttcacatttc ctgtcttact tttgggagag aaaccgttct tgagtatttg 480
gtgtcttttg gagtgtggat tcgcactcct cctgcttata gaccaccaaa tgcccctatc 540
ctatcaacac ttccggagac tactgttgtt agacgaagag gcaggtcccc tcgaagaaga 600
actccctcgc ctcgcagacg aagatctcaa tcgccgcgtc gcagaagata tgcatctc 658

<210> 50
<211> 649
<212> DNA
<213> Hepatitis B virus

<400> 50
atgtaacttt ttcacctctg cctaatcatc tcttgttcat gtcctactgt tcaagcctcc 60
aagctgtgcc ttgggtggct ttagggcatg gatagaacaa ctttgccata tggccttttt 120
ggcttagaca ttgaccctta taaagaattt ggagctactg tggagttgct ctcgtttttg 180

ccttctgact ttttcccgtc tgttcgtgat cttctcgaca ccgcttcagc tttgtaccgg 240
gaatccttag agtcctctga tcattgttcg cctcaccata cagcactcag gcaagcaatc 300
ctgtgctggg gtgagttgat gactctagct acctgggtgg gtaataattt ggaagatcca 360
gcatccagag atttggtggt caattatgtt aatactaata tgggtttaaa aatcaggcaa 420
ctattgtggt ttcacatttc ctgtcttact tttgggagag aaaccgttct tgagtatttg 480
gtgtcttttg gagtgtggat tcgcactcct cctgcttata gaccaccaaa tgcccctatc 540
ctatcaacac ttccggagac tactgttgtt agacgaagag gcaggtcccc tcgaagaaga 600
actccctcgc ctcgcagacg aagatctcaa tcgccgcgtc gcagaagat 649

<210> 51
<211> 649
<212> DNA
<213> Hepatitis B virus

<400> 51
atgtaacttt ttcacctctg cctaatcatc tcttgttcat gtcctactgt tcaagcctcc 60
aagctgtgcc ttgggtggct ttagggcatg gatagaacaa ctttgccata tggccttttt 120
ggcttagaca ttgaccctta taaagaattt ggagctactg tggagttgct ctcgtttttg 180
ccttctgact ttttcccgtc tgttcgtgat cttctcgaca ccgcttcagc tttgtaccgg 240
gaatccttag agtcctctga tcattgttcg cctcaccata cagcactcag gcaagcaatc 300
ctgtgctggg gtgagttgat gactctagct acctgggtgg gtaataattt ggaagatcca 360
gcatccagag atttggtggt caattatgtt aatactaata tgggtttaaa aatcaggcaa 420
ctattgtggt ttcacatttc ctgtcttact tttgggagag aaaccgttct tgagtatttg 480
gtgtcttttg gagtgtggat tcgcactcct cctgcttata gaccaccaaa tgcccctatc 540
ctatcaacac ttccggagac tactgttgtt agacgaagag gcaggtcccc tcgaagaaga 600
actccctcgc ctcgcagacg aagatctcaa tcgccgcgtc gcagaagat 649

<210> 52
<211> 1132
<212> DNA
<213> Hepatitis B virus

<400> 52
atgggaggtt ggtcatcaaa acctcgcaaa ggcatgggga cgaatctttc tgttcccaac 60
cctctgggat tctttcccga tcatcagttg gaccctgcat tcggagccaa ctcaaacaat 120
ccagattggg acttcaaccc catcaaggac cactggccag cagccaacca ggtaggagtg 180
ggagcattcg ggccaaggct cacccctcca cacggcggta ttttggggtg gagccctcag 240
gctcagggca tattgaccac agtgtcaaca attcctcctc ctgcctccac caatcggcag 300
tcaggaaggc agcctactcc catctctcca cctctaagag acagtcatcc tcaggccatg 360
cagtggaatt ccactgcctt ccaccaagct ctgcaggatc ccaaagtcag gggtctgtat 420
cttcctgctg gtggctccag ttcaggaaca gtaaaccctg ctccgaatat tgcctctcac 480
atctcgtcaa tctccgcgag gactggggac cctgtgacga acatggagaa catcacatca 540
ggattcctag gacccctgct cgtgttacag gcggggtttt tcttgttgac aagaatcctc 600
acaataccgc agagtctaga ctcgtggtgg acttctctca attttctagg ggggtcaccc 660
gtgtgtcttg gccaaaattc gcagtcccca acctccaatc actcaccaac ctcctgtcct 720
ccaatttgtc ctggttatcg ctggatgtgt ctgcggcgtt ttatcatatt cctcttcatc 780

ctgctgctat gcctcatctt cttgttggtt cttctggatt atcaaggtat gttgcccgtt 840
tgtcctctaa ttccaggatc aacaacaacc agtacgggac catgcaaaac ctgcacgact 900
cctgctcaag gcaactctat gtttccctca tgttgctgta caaaacctac ggatggaaat 960
tgcacctgta ttcccatccc atcgtcctgg gctttcgcaa aatacctatg ggagtgggcc 1020
tcagtccgtt tctcttggct cagtttacta gtgccatttg ttcagtggtt cgtagggctt 1080
tcccccactg tttggctttc agctatatgg atgatgtggt attgggggcc aa 1132

<210> 53
<211> 1129
<212> DNA
<213> Hepatitis B virus

<400> 53
atggggcttt cttggacggt ccctctcgag tggggaaaga acctttccgc cagcaatcct 60
ctaggattcc ttcccgatca ccagttggac ccagcattca gagcaaatac caacaatcca 120
gattgggact tcaatcccaa aaaggaccct tggccagagg ccaacaaggt aggagttgga 180
gcctatggac ccgggttcac ccctccacac ggaggccttt tggggtggag ccctcagtct 240
cagggcacac taacaacttt gccagcagat ccgcctcctg cctccaccaa tcgtcagtca 300
gggaggcagc ctactcccat ctctccacca ctaagagaca gtcatcctca ggccatgcag 360
tggaactcta cagcattcca ccaagctcta caaaatccca aagtcagggg cctgtatttt 420
cctgctggtg gctccagttc agggatagtg aaccctgttc cgactattgc ctctcacatc 480
tcgtcaatct tctccaggat tggggaccct gcaccgaaca tggagaacat cacatcagga 540
ttcctaggac ccctgctcgt gttacaggcg gggtttttct tgttgacaag aatcctcaca 600
ataccgcaga gtctagactc gtggtggact tctctcaatt ttctaggggg agtgcccgtg 660
tgtcctggcc taaattcgca gtccccaacc tccaatcact caccaatctc ctgtcctcca 720
acttgtcctg gctatcgctg gatgtgtctg cggcgtttta tcatattcct cttcatcctg 780
ctgctatgcc tcatcttctt gttggttctt ctggactatc aaggtatgtt gcccgtttgt 840
cctctgattc caggatcctc gaccaccagt acgggaccct gcaaaacctg cacgactcct 900
gctcaaggca actctatgta tccctcatgt tgctgtacaa aaccttcgga cggaaattgc 960
acctgtattc ccatcccatc atcttgggct ttcgcaaaat acctatggga gtgggcctca 1020
gtccgtttct cttggctcag tttactagtg ccatttgttc agtggttcgt agggctttcc 1080
cccactgtct ggctttcagc tatatggatg atgtggtatt gggggccaa 1129

<210> 54
<211> 1122
<212> DNA
<213> Hepatitis B virus

<400> 54
tttcttggac ggtccctctc gagtggggaa agaacctttc caccagcaat cctctaggat 60
tccttcccga tcaccagttg gacccagcat tcagagcaaa taccaacaat ccagattggg 120
acttcaatcc caaaaaggac ccttggccag aggccaacaa ggtaggagtt ggagcctatg 180
gacccgggtt cacccctcca cacggaggcc ttttggggtg gagccctcag tctcagggca 240
cactaacaac tttgccagca gatccgcctc ctgcctccac caatcgtcag tcagggaggc 300
agcctactcc catctctcca ccactaakag acagtcatcc tcaggccatg cagyggaact 360
ctacagcatt ccaccaagct ctacaaaatc ccaaagtcag gggcctgtat tttcctgctg 420

gtggctccag ttcagggata gtgaaccctg ttccgactat tgcctctcac atctcgtcaa 480
tcttctccag gattggggac cctgcaccga acatggagaa catcacatca ggattcctag 540
gacccctgct cgtgttacag gcggggtttt tcttgttgac aagaatcctc acaataccgc 600
agagtctaga ctcgtggtgg acttctctca attttctagg gggagtgccc gtgtgtcctg 660
gcctaaattc gcagtcccca acctccaatc actcaccaat ctcctgtcct ccaacttgtc 720
ctggctatcg ctggatgtgt ctgcggcgtt ttatcatatt cctcttcatc ctgctgctat 780
gcctcatctt cttgttggtt cttctggact atcaaggtat gttgcccgtt tgtcctctga 840
ttccaggatc ctcgaccacc agtacgggac cctgcaaaac ctgcacgact cctgctcaag 900
gcaactctat gtatccctca tgttgctgta caaaaccttc ggacggaaat tgcacctgta 960
ttcccatccc atcatcttgg gctttcgcaa aatacctatg ggagtgggcc tcagtccgtt 1020
tctcttggct cagtttacta gtgccatttg ttcagtggtt cgtagggctt tcccccactg 1080
tctggctttc agctatatgg atgatgtggt attgggggcc aa 1122

<210> 55
<211> 1076
<212> DNA
<213> Hepatitis B virus

<400> 55
gtccctctcg agtggggaaa gaacctttcc accagcaatc ctctaggatt ccttcccgat 60
caccagttgg acccagcatt cagagcaaat accaacaatc cagattggga cttcaatccc 120
aaaaaggacc cttggccaga ggccaacaag gtaggagttg gagcctatgg acccgggttc 180
acccctccac acggaggcct tttggggtgg agccctcagt ctcagggcac actaacaact 240
ttgccagcag atccgcctcc tgcctccacc aatcgtcagt cagggaggca gcctactccc 300
atctctccac cactaagaga cagtcatcct caggccatgc agtggaactc tacagcattc 360
caccaagctc tacaaaatcc caaagtcagg ggcctgtatt ttcctgctgg tggctccagt 420
tcagggatag tgaaccctgt tccgactatt gcctctcaca tctcgtcaat cttctccagg 480
attggggacc ctgcaccgaa catggagaac atcacatcag gattcctagg acccctgctc 540
gtgttacagg cggggttttt cttgttgaca agaatcctca caataccgca gagtctagac 600
tcgtggtgga cttctctcaa ttttctaggg gaagtgcccg tgtgtcctgg cttaaattcg 660
cagtccccaa cctccaatca ctcaccaatc tcctgtcctc caacttgtcc tggctatcgc 720
tggatgtgtc tgcggcgttt tatcatattc ctcttcatcc tgctgctatg cctcatcttc 780
ttgttggttc ttctggacta tcaaggtatg ttgcccgttt gtcctctgat tccaggatcc 840
tcgaccacca gtacgggacc ctgcaaaacc tgcacgactc ctgctcaagg caactctatg 900
tatccctcat gttgctgtac aaaaccttcg gacggaaatt gcacctgtat tcccatccca 960
tcatcttggg ctttcgcaaa atacctatgg gagtgggcct cagtccgttt ctcttggctc 1020
agtttactag tgccatttgt tcagtggttc gtagggcttt cccccactgt ctggct 1076

<210> 56
<211> 1077
<212> DNA
<213> Hepatitis B virus

<400> 56
cgtccctctc gagtggggaa agaacctttc caccagcaat cctctaggat tccttcccga 60
tcaccagttg gacccagcat tcagagcaaa taccaacaat ccagattggg acttcaatcc 120

caaaaaggac ccttggccag aggccaacaa ggtaggagtt ggagcctatg gacccgggtt 180
cacccctcca cacggaggcc ttttggggtg gagccctcag tctcagggca cactaacaac 240
tttgccagca gatccgcctc ctgcctccac caatcgtcag tcagggaggc agcctactcc 300
catctctcca ccactaagag acagtcatcc tcaggccatg cagtggaact ctacagcatt 360
ccaccaagct ctacaaaatc ccaaagtcag gggcctgtat tttcctgctg gtggctccag 420
ttcaaggata gtgaaccctg ttccgactat tgcctctcac atctcgtcaa tcttctccag 480
gattggggac cctgcaccga acatggagaa catcatatca ggattcctag gacccctgct 540
cgtgttacag gcggggtttt tcttgttgac aagaatcctc acaataccgc agagtctaga 600
ctcgtggtgg acttctctca attttctagg gggagtgccc gtgtgtcctg gcctaaattc 660
gcagtcccca acctccaatc actcaccaat ctcctgtcct ccaacttgtc ctggctatcg 720
ctggatgtgt ctgcggcgtt ttatcatatt cctcttcatc ctgctgctat gcctcatctt 780
cttgttggtt cttctggact atcaaggtat gttgcccgtt tgtcctctga ttccaggatc 840
ctcgaccacc agtacgggac cctgcaaaac ctgcacgact cctgctcaag gcaactctat 900
gtatccctca tgttgctgta caaaaccttc ggacggaaat tgcacctgta ttcccatccc 960
atcatcttgg gctttcgcaa aatacctatg ggagtgggcc tcagtccgtt tctcttggct 1020
cagtttacta gtgccatttg ttcagtggtt cgtagggctt tcccccactg tctggct 1077

<210> 57
<211> 1104
<212> DNA
<213> Hepatitis B virus

<400> 57
ccctctcgag tggggaagga acctttccac cagcaatcct ctaggattcc ttcccgatca 60
ccagtaggtc ccagcattca gagcaaatac caacaatcca gattgggact tcaatcccaa 120
aaaggaccct tggccagagg ccaacaaggt aggagttgga gcctatggac ccgggttcac 180
ccctccacac ggaggccttt tggggtggag ccctcagtct cagggcacac taacaacttt 240
gccagcagat ccgcctcctg cctccaccaa tcgtcagtca gggaggcagc ctactcccat 300
ctctccacca ctaagagaca gtcatcctca ggccatgcag tggaactcta cagcattcca 360
ccaagctcta caaaatccca aagtcagggg cctgtatttt cctgctggtg gctccagttc 420
agggatagtg aaccctgttc cgactattgc ctctcacatc tcgtcaatct tctccaggat 480
tggggaccct gcaccgaaca tggagaacat cacatcagga ttcctaggac ccctgctcgt 540
gttacaggcg gggtttttct tgttgacaag aatcctcaca ataccgcaga gtctagactc 600
gtggtggact tctctcaatt ttctaggggg agtgcccgtg tgtcctggcc taaattcgca 660
gtccccaacc tccaatcact caccaatctc ctgtcctcca acttgtcctg gctatcgctg 720
gatgtgtttg cggcgtttta tcatattcct cttcatcctg ctgctatgcc tcatcttctt 780
gttggttctt ctggactatc aaggtatgtt gcccgtttgt cctctgattc caggatcctc 840
gaccaccagt acgggaccct gcaaaacctg cacgactcct gctcaaggca actctatgta 900
tccctcatgt tgctgtacaa aaccttcgga cggaaattgc acctgtattc ccatcccatc 960
atcttgggct ttcgcaaaat acctatggga gtgggcctca gtccgtttct cttggctcag 1020
tttactagtg ccatttgttc agtggttcgt agggctttcc cccactgtct ggctttcagc 1080
tatatggatg atgtggtatt gggg 1104

<210> 58
<211> 1087
<212> DNA

<213> Hepatitis B virus

<400> 58
ccctctcgag tggggaaaga acctttccac cagcaatcct ctaggattcc ttcccgatca 60
ccagttggac ccagcattca gagcaaatac caacaatcca gattgggact tcaatcccaa 120
aaaggaccct tggccagagg ccaacaaggt aggagttgga gcctatggac ccgggttcac 180
ccctccacac ggaggccttt tggggtggag ccctcagtct cagggcacac taacaacttt 240
gccagcagat ccgcctcctg cctccaccaa tcgtcagtca gggaggcagc ctactcccat 300
ctctccacca ctaagagaca gtcatcctca ggccatgcag tggaactcta cagcattcca 360
ccaagctcta caaaatccca aagtcagggg cctgtatttt cctgctggtg gctccagttc 420
agggatagtg aaccctgttc cgactattgc ctctcacatc tcgtcaatct tctccaggat 480
tggggaccct gcaccgaaca tggagaacat cacatcagga ttcctaggac ccctgctcgt 540
gttacaggcg gggtttttct tgttgacaag aatcctcaca ataccgcaga gtctagactc 600
gtggtggact tctctcaatt ttctaggggg agtgcccgtg tgtcctggcc taaattcgca 660
gtccccaacc tccaatcact caccaatctc ctgtcctcca acttgtcctg gctatcgctg 720
gatgtgtctg cggcgtttta tcatattcct cttcatcctg ctgctatgcc tcatcttctt 780
gttggttctt ctggactatc aaggtatgtt gcccgtttgt cctctgattc caggatcctc 840
gaccaccagt acgggaccct gcaaaacctg cacgactcct gctcaaggca actctatgta 900
tccctcatgt tgctgtacaa aaccttcgga cggaaattgc acctgtattc ccatcccatc 960
atcttgggct ttcgcaaaat acctatggga gtgggcctca gtccgtttct cttggctcag 1020
tttactagtg ccatttgttc agtggttcgt agggctttcc cccactgtmt ggctttcagc 1080
tatatgg 1087

<210> 59
<211> 1084
<212> DNA
<213> Hepatitis B virus

<400> 59
gagtggggaa agaacctttc caccagcaat cctctaggat tccttcccga tcaccagttg 60
gacccagcat tcagagcaaa taccaacaat ccagattggg acttcaatcc caaaaaggac 120
ccttggccag aggccaacaa ggtaggagtt ggagcctatg gacccgggtt cacccctcca 180
cacggaggcc ttttggggtg gagccctcag tctcagggca cactaacaac tttgccagca 240
gatccgcctc ctgcctccac caatcgtcag tcagggaggc agcctactcc catctctcca 300
ccactaagag acagtcatcc tcaggccatg cagtggaact ctacagcatt ccaccaagct 360
ctacaaaatc ccaaagtcag gggcctgtat tttcctgctg gtggctccag ttcagggata 420
gtgaaccctg ttccgactat tgcctctcac atctcgtcaa tcttctccag gattggggac 480
cctgcaccga acatggagaa catcacatca ggattcctag gacccctgct cgtgttacag 540
gcggggtttt tcttgttgac aagaatcctc acaataccgc agagtctaga ctcgtggtgg 600
acttctctca attttctagg gggagtgccc gtgtgtcctg gcctaaattc gcagtcccca 660
acctccaatc actcaccaat ctcctgtcct ccaacttgtc ctggctatcg ctggatgtgt 720
ctgcggcgtt ttatcatatt cctcttcatc ctgctgctat gcctcatctt cttgttggtt 780
cttctggact atcaaggtat gttgcccgtt tgtcctctga ttccaggatc ctcgaccacc 840
agtacgggac cctgcaaaac ctgcacgact cctgctcaag gcaactctat gtatccctca 900
tgttgctgta caaaaccttc ggacggaaat tgcacctgta ttcccatccc atcatcttgg 960
gctttcgcaa aatacctatg ggagtgggcc tcagtccgtt tctcttggct cagtttacta 1020
gtgccatttg ttcagtggtt cgtagggctt tcccccactg tctggctttc agctatatgg 1080

atga                                                              1084

<210> 60
<211> 1051
<212> DNA
<213> Hepatitis B virus

<400> 60
ttggcggccc tctgagtggg gaaagaacct ttccaccagc aatcctctag gattccttcc 60
cgatcaccag ttggacccag cattcagagc aaataccaac aatccagatt gggacttcaa 120
tcccaaaaag gacccttggc cagaggccaa caaggtagga gttggagcct atggacccgg 180
gttcacccct ccacacggag gccttttggg gtggagccct cagtctcagg gcacactaac 240
aactttgcca gcagatccgc ctcctgcctc caccaatcgt cagtcaggga ggcagcctac 300
tcccatctct ccaccactaa gagacagtca tcctcaggcc atgcagtgga actctacagc 360
attccaccaa gctctacaaa atcccaaagt cgcccctgct ggtggctcca gttcagggat 420
agtgaaccct gttccgacta ttgcctctca catctcgtca atcttctcca ggattgggga 480
ccctgcaccg aacatggaga acatcacatc aggattccta ggacccctgc tcgtgttaca 540
ggcggggttt ttcttgttga caagaatcct cacaataccg cagagtctag actcgtggtg 600
gacttctctc aattttctag ggggagtgcc cgtgtgtcct ggcctaaatt cgcagtcccc 660
aacctccaat cactcaccaa tctcctgtcc tccaacttgt cctggctatc gctggatgtg 720
tctgcggcgt tttatcatat tcctcttcat cctgctgcta tgcctcatct tcttgttggt 780
tcttctggac tatcaaggta tgttgcccgt ttgtcctctg attccaggat cctcgaccac 840
cagtacggga ccctgcaaaa cctgcacgac tcctgctcaa ggcaactcta tgtatccctc 900
atgttgctgt acaaaacctt cggacggaaa ttgcacctgt attcccatcc catcatcttg 960
ggctttcgca aaatacctat gggagtgggc ctcagtccgt ttctcttggc tcagtttact 1020
agtgccattt gttcagggtt cgagggcttt c                                1051

<210> 61
<211> 36
<212> DNA
<213> Hepatitis B virus

<400> 61
tagaacaact ttgccatatg gcctttttgg cttaga                           36

<210> 62
<211> 5
<212> DNA
<213> Hepatitis B virus

<400> 62
tagaa                                                             5

<210> 63

<211> 5
<212> DNA
<213> Hepatitis B virus

<400> 63
agaac                                                                    5

<210> 64
<211> 5
<212> DNA
<213> Hepatitis B virus

<400> 64
gaaca                                                                    5

<210> 65
<211> 5
<212> DNA
<213> Hepatitis B virus

<400> 65
aacaa                                                                    5

<210> 66
<211> 5
<212> DNA
<213> Hepatitis B virus

<400> 66
acaac                                                                    5

<210> 67
<211> 5
<212> DNA
<213> Hepatitis B virus

<400> 67
caact                                                                    5

<210> 68
<211> 5
<212> DNA
<213> Hepatitis B virus

<400> 68
aactt                                                                    5

<210> 69
<211> 5
<212> DNA
<213> Hepatitis B virus

<400> 69
acttt                                                                    5

<210> 70
<211> 5
<212> DNA
<213> Hepatitis B virus

<400> 70
ctttg                                                                    5

<210> 71
<211> 5
<212> DNA
<213> Hepatitis B virus

<400> 71
tttgc                                                                    5

<210> 72
<211> 5
<212> DNA
<213> Hepatitis B virus

<400> 72
ttgcc                                                                    5

<210> 73
<211> 5
<212> DNA
<213> Hepatitis B virus

<400> 73
tgcca                                                                    5

<210> 74
<211> 5
<212> DNA
<213> Hepatitis B virus

<400> 74
gccat                                                                    5

<210> 75
<211> 5
<212> DNA
<213> Hepatitis B virus

<400> 75
ccata                                                                    5

<210> 76
<211> 5
<212> DNA
<213> Hepatitis B virus

<400> 76
catat                                                                    5

<210> 77
<211> 5
<212> DNA
<213> Hepatitis B virus

<400> 77
atatg                                                                    5

<210> 78
<211> 5
<212> DNA
<213> Hepatitis B virus

<400> 78
tatgg                                                                    5

<210> 79

<211> 5
<212> DNA
<213> Hepatitis B virus

<400> 79
atggc                                                                    5


<210> 80
<211> 5
<212> DNA
<213> Hepatitis B virus

<400> 80
tggcc                                                                    5


<210> 81
<211> 5
<212> DNA
<213> Hepatitis B virus

<400> 81
ggcct                                                                    5


<210> 82
<211> 5
<212> DNA
<213> Hepatitis B virus

<400> 82
gcctt                                                                    5


<210> 83
<211> 5
<212> DNA
<213> Hepatitis B virus

<400> 83
ccttt                                                                    5


<210> 84
<211> 5
<212> DNA
<213> Hepatitis B virus

<400> 84
ctttt                                                                 5

<210> 85
<211> 5
<212> DNA
<213> Hepatitis B virus

<400> 85
ttttt                                                                 5

<210> 86
<211> 5
<212> DNA
<213> Hepatitis B virus

<400> 86
ttttg                                                                 5

<210> 87
<211> 5
<212> DNA
<213> Hepatitis B virus

<400> 87
tttgg                                                                 5

<210> 88
<211> 5
<212> DNA
<213> Hepatitis B virus

<400> 88
ttggc                                                                 5

<210> 89
<211> 5
<212> DNA
<213> Hepatitis B virus

<400> 89
tggct                                                                 5

<210> 90
<211> 5
<212> DNA
<213> Hepatitis B virus

<400> 90
ggctt                                                                    5


<210> 91
<211> 5
<212> DNA
<213> Hepatitis B virus

<400> 91
gctta                                                                    5


<210> 92
<211> 5
<212> DNA
<213> Hepatitis B virus

<400> 92
cttag                                                                    5


<210> 93
<211> 5
<212> DNA
<213> Hepatitis B virus

<400> 93
ttaga                                                                    5


<210> 94
<211> 6
<212> DNA
<213> Hepatitis B virus

<400> 94
tagaac                                                                   6


<210> 95

<211> 6
<212> DNA
<213> Hepatitis B virus

<400> 95
agaaca                                                                 6


<210> 96
<211> 6
<212> DNA
<213> Hepatitis B virus

<400> 96
gaacaa                                                                 6


<210> 97
<211> 6
<212> DNA
<213> Hepatitis B virus

<400> 97
aacaac                                                                 6


<210> 98
<211> 6
<212> DNA
<213> Hepatitis B virus

<400> 98
acaact                                                                 6


<210> 99
<211> 6
<212> DNA
<213> Hepatitis B virus

<400> 99
caactt                                                                 6


<210> 100
<211> 6
<212> DNA
<213> Hepatitis B virus

<400> 100
aacttt                                                                 6

<210> 101
<211> 6
<212> DNA
<213> Hepatitis B virus

<400> 101
actttg                                                                 6

<210> 102
<211> 6
<212> DNA
<213> Hepatitis B virus

<400> 102
ctttgc                                                                 6

<210> 103
<211> 6
<212> DNA
<213> Hepatitis B virus

<400> 103
tttgcc                                                                 6

<210> 104
<211> 6
<212> DNA
<213> Hepatitis B virus

<400> 104
ttgcca                                                                 6

<210> 105
<211> 6
<212> DNA
<213> Hepatitis B virus

<400> 105
tgccat                                                                 6

<210> 106
<211> 6
<212> DNA
<213> Hepatitis B virus

<400> 106
gccata                                                                 6

<210> 107
<211> 6
<212> DNA
<213> Hepatitis B virus

<400> 107
ccatat                                                                 6

<210> 108
<211> 6
<212> DNA
<213> Hepatitis B virus

<400> 108
catatg                                                                 6

<210> 109
<211> 6
<212> DNA
<213> Hepatitis B virus

<400> 109
atatgg                                                                 6

<210> 110
<211> 6
<212> DNA
<213> Hepatitis B virus

<400> 110
tatggc                                                                 6

<210> 111

<211> 6
<212> DNA
<213> Hepatitis B virus

<400> 111
atggcc                                                                 6


<210> 112
<211> 6
<212> DNA
<213> Hepatitis B virus

<400> 112
tggcct                                                                 6


<210> 113
<211> 6
<212> DNA
<213> Hepatitis B virus

<400> 113
ggcctt                                                                 6


<210> 114
<211> 6
<212> DNA
<213> Hepatitis B virus

<400> 114
gccttt                                                                 6


<210> 115
<211> 6
<212> DNA
<213> Hepatitis B virus

<400> 115
cctttt                                                                 6


<210> 116
<211> 6
<212> DNA
<213> Hepatitis B virus

<400> 116
cttttt                                                                 6

<210> 117
<211> 6
<212> DNA
<213> Hepatitis B virus

<400> 117
ttttg                                                                  6

<210> 118
<211> 6
<212> DNA
<213> Hepatitis B virus

<400> 118
ttttgg                                                                 6

<210> 119
<211> 6
<212> DNA
<213> Hepatitis B virus

<400> 119
tttggc                                                                 6

<210> 120
<211> 6
<212> DNA
<213> Hepatitis B virus

<400> 120
ttggct                                                                 6

<210> 121
<211> 6
<212> DNA
<213> Hepatitis B virus

<400> 121
tggctt                                                                 6

<210> 122
<211> 6
<212> DNA
<213> Hepatitis B virus

<400> 122
ggctta                                                                 6

<210> 123
<211> 6
<212> DNA
<213> Hepatitis B virus

<400> 123
gcttag                                                                 6

<210> 124
<211> 6
<212> DNA
<213> Hepatitis B virus

<400> 124
cttaga                                                                 6

<210> 125
<211> 7
<212> DNA
<213> Hepatitis B virus

<400> 125
tagaaca                                                                7

<210> 126
<211> 7
<212> DNA
<213> Hepatitis B virus

<400> 126
agaacaa                                                                7

<210> 127

<211> 7
<212> DNA
<213> Hepatitis B virus

<400> 127
gaacaac                                                                 7

<210> 128
<211> 7
<212> DNA
<213> Hepatitis B virus

<400> 128
aacaact                                                                 7

<210> 129
<211> 7
<212> DNA
<213> Hepatitis B virus

<400> 129
acaactt                                                                 7

<210> 130
<211> 7
<212> DNA
<213> Hepatitis B virus

<400> 130
caacttt                                                                 7

<210> 131
<211> 7
<212> DNA
<213> Hepatitis B virus

<400> 131
aactttg                                                                 7

<210> 132
<211> 7
<212> DNA
<213> Hepatitis B virus

<400> 132
actttgc                                                                 7

<210> 133
<211> 7
<212> DNA
<213> Hepatitis B virus

<400> 133
ctttgcc                                                                 7

<210> 134
<211> 7
<212> DNA
<213> Hepatitis B virus

<400> 134
tttgcca                                                                 7

<210> 135
<211> 7
<212> DNA
<213> Hepatitis B virus

<400> 135
ttgccat                                                                 7

<210> 136
<211> 7
<212> DNA
<213> Hepatitis B virus

<400> 136
tgccata                                                                 7

<210> 137
<211> 7
<212> DNA
<213> Hepatitis B virus

<400> 137
gccatat                                                                 7

<210> 138
<211> 7
<212> DNA
<213> Hepatitis B virus

<400> 138
ccatatg                                                                 7

<210> 139
<211> 7
<212> DNA
<213> Hepatitis B virus

<400> 139
catatgg                                                                 7

<210> 140
<211> 7
<212> DNA
<213> Hepatitis B virus

<400> 140
atatggc                                                                 7

<210> 141
<211> 7
<212> DNA
<213> Hepatitis B virus

<400> 141
tatggcc                                                                 7

<210> 142
<211> 7
<212> DNA
<213> Hepatitis B virus

<400> 142
atggcct                                                                 7

<210> 143

<211> 7
<212> DNA
<213> Hepatitis B virus

<400> 143
tggcctt                                                                 7


<210> 144
<211> 7
<212> DNA
<213> Hepatitis B virus

<400> 144
ggccttt                                                                 7


<210> 145
<211> 7
<212> DNA
<213> Hepatitis B virus

<400> 145
gcctttt                                                                 7


<210> 146
<211> 7
<212> DNA
<213> Hepatitis B virus

<400> 146
ccttttt                                                                 7


<210> 147
<211> 7
<212> DNA
<213> Hepatitis B virus

<400> 147
ctttttg                                                                 7


<210> 148
<211> 7
<212> DNA
<213> Hepatitis B virus

<400> 148
tttttgg                                                                 7

<210> 149
<211> 7
<212> DNA
<213> Hepatitis B virus

<400> 149
ttttggc                                                                 7

<210> 150
<211> 7
<212> DNA
<213> Hepatitis B virus

<400> 150
tttggct                                                                 7

<210> 151
<211> 7
<212> DNA
<213> Hepatitis B virus

<400> 151
ttggctt                                                                 7

<210> 152
<211> 7
<212> DNA
<213> Hepatitis B virus

<400> 152
tggctta                                                                 7

<210> 153
<211> 7
<212> DNA
<213> Hepatitis B virus

<400> 153
ggcttag                                                                 7

<210> 154
<211> 7
<212> DNA
<213> Hepatitis B virus

<400> 154
gcttaga                                                                  7


<210> 155
<211> 12
<212> PRT
<213> Hepatitis B virus

<220>
<221> misc_feature
<222> (1)..(3248)
<223> Circular DNA

<400> 155
Arg Thr Thr Leu Pro Tyr Gly Leu Phe Gly Leu Asp
1               5                   10


<210> 156
<211> 6
<212> PRT
<213> Hepatitis B virus

<400> 156
Arg Thr Thr Leu Pro Tyr
1               5


<210> 157
<211> 6
<212> PRT
<213> Hepatitis B virus

<400> 157
Thr Thr Leu Pro Tyr Gly
1               5

<210> 158
<211> 6
<212> PRT
<213> Hepatitis B virus

<400> 158
Thr Leu Pro Tyr Gly Leu
1               5

<210> 159
<211> 6
<212> PRT
<213> Hepatitis B virus

<400> 159
Leu Pro Tyr Gly Leu Phe
1               5

<210> 160
<211> 6
<212> PRT
<213> Hepatitis B virus

<400> 160
Pro Tyr Gly Leu Phe Gly
1               5

<210> 161
<211> 6
<212> PRT
<213> Hepatitis B virus

<400> 161
Tyr Gly Leu Phe Gly Leu
1               5

<210> 162
<211> 6
<212> PRT

<213> Hepatitis B virus

<400> 162
Gly Leu Phe Gly Leu Asp
1               5

<210> 163
<211> 7
<212> PRT
<213> Hepatitis B virus

<400> 163
Arg Thr Thr Leu Pro Tyr Gly
1               5

<210> 164
<211> 7
<212> PRT
<213> Hepatitis B virus

<400> 164
Thr Thr Leu Pro Tyr Gly Leu
1               5

<210> 165
<211> 7
<212> PRT
<213> Hepatitis B virus

<400> 165
Thr Leu Pro Tyr Gly Leu Phe
1               5

<210> 166
<211> 7
<212> PRT
<213> Hepatitis B virus

<400> 166
Leu Pro Tyr Gly Leu Phe Gly

1                   5

<210> 167
<211> 7
<212> PRT
<213> Hepatitis B virus

<400> 167
Pro Tyr Gly Leu Phe Gly Leu
1                   5

<210> 168
<211> 7
<212> PRT
<213> Hepatitis B virus

<400> 168
Tyr Gly Leu Phe Gly Leu Asp
1                   5

## Description

### FIELD OF THE INVENTION

The present invention relates to the field of Hepatitis B virus (HBV) diagnostics and therapeutics. The present invention provides sequences of a new HBV genotype G and a new mechanism for HBeAg modulation for use in diagnosis, prevention and treatment of HBV infections.

### BACKGROUND OF THE INVENTION

Human Hepatitis B virus (HBV), which is the prototype member of the family *Hepadnaviridae,* is a circular, partially double-stranded DNA virus of approximately 3200 nucleotides (Magnius and Norder, 1995). This highly compact genome contains the four major open reading frames (ORFs) encoding the envelope (preS1, preS2 and surface antigen HBsAg), core (preCore precursor protein, HBeAg and HBcAg), polymerase (HBpol) and X (HBX) proteins, respectively.

By using subtype-specific antibodies against HBsAg, nine different serological subtypes were defined, reflecting the genetic variability of HBV. Of the defined determinants, one is common to all subtypes (a determinant), but also two pairs of mutually exclusive subdeterminants (d or y, and w or r) were commonly found. By using this tool in epidemiological studies, nine serological subtypes have been identified: ayw1, ayw2, ayw3, ayw4, ayr, adw2, adw4, adrq+ and adrq- (Swenson et al., 1991).

Genotypically, HBV genomes have been classified into six groups, designated A-F, based on an intergroup divergence of 8% or more in the complete nucleotide sequence (Okamoto et al., 1988; Norder et al., 1992; Magnius and Norder, 1995). These six different genotypes show a characteristic geographical distribution: genotype A is pandemic, but most prevalent in north-west Europe, North America, and Central Africa; genotype B is mostly found in Indonesia, China and Vietnam; genotype C is found in East Asia, Korea, China, Japan, Polynesia and Vietnam; genotype D is also more or less pandemic, but is predominant in the Mediterranean area and the Middle East extending to India; genotype E is endemic in Africa; and genotype F is found in American natives and in Polynesia (Van Geyt et al., 1998; Magnus & Norder, 1995). Some studies have shown that, in certain populations where HBV is endemic, a higher variability of HBV might be expected (Bowyer et al., 1997; Carman et al., 1997). In the present prevalence study of the HBV genotypes in France and the USA, HBV strains were identified that did not allow unique type recognition.

### AIMS OF THE INVENTION

It is an aim of the present invention to provide new HBV polynucleic acid sequences that are unique to a theretofore-unidentified HBV genotype, genotype G, which is phylogenetically different from HBV genotypes A, B, C, D, E and F.

It is another aim of the invention to provide fragments of the HBV polynucleic acids as described above, that are unique to the theretofore-unidentified HBV genotype G and that contain at least one nucleotide or a combination of nucleotides different from known nucleic acid sequences of HBV genotypes A, B, C, D, E or F, or the complement of said fragments.

It is another aim of the present invention to provide fragments of the HBV polynucleic acids as described above that are universal to genotypes A, B, C, D, E, F and G.

It is another aim of the invention to provide a method for the detection in a biological sample of the presence of an HBV genotype G specific polynucleic acid.

It is another aim of the invention to provide a universal method for the detection of HBV genotypes A, B, C, D, E, F and G.

It is another aim of the invention to provide an oligonucleotide primer that is able to act as primer for specifically sequencing or specifically amplifying a nucleic acid or part of a nucleic acid characteristic to HBV genotype G.

It is another aim of the invention to provide a universal primer that is able to act as a primer for sequencing or amplifying nucleic acids from HBV genotypes A, B, C, D, E, F and G.

It is another aim of the invention to provide an oligonucleotide probe that is able to act as a hybridization probe for specific detection of a nucleic acid or part of a nucleic acid characteristic to HBV genotype G.

It is another aim of the invention to provide a universal probe that is able to act as a hybridization probe for the universal detection of nucleic acids from HBV genotypes A, B, C, D, E, F and G.

It is another aim of the invention to provide a diagnostic kit for the detection of a nucleic acid or part of a nucleic acid characteristic to HBV genotype G.

It is another aim of the invention to provide a diagnostic kit for the universal detection of HBV genotypes A, B, C, D, E, F and G.

It is another aim of the present invention to provide a HBV genotype G specific polypeptide encoded by a HBV genotype G specific nucleic acid of the invention. It is another aim of the present invention to provide a universal peptide characteristic to genotypes A, B, C, D, E, F, and G.

It is another aim of the present invention to provide a method for the production of a polypeptide of the invention.

It is another aim of the present invention to provide an expression vector that allows the expression of a peptide of the invention.

It is another aim of the present invention to provide a host cells transformed with an expression vector of the invention.

It is another aim of the present invention to provide a method for the detection of antibodies that are specific to the theretofore-unidentified HBV genotype or antibodies that recognize HBV genotypes A, B, C, D, E, F and G.

It is another aim of the present invention to provide a diagnostic kit for the detection of antibodies that are specific to the theretofore-unidentified HBV genotype or for the detection of antibodies that recognize HBV genotypes A, B, C, D, E, F and G.

It is another aim of the present invention to provide an antibody that binds a peptide of the invention.

It is another aim of the present invention to provide an antibody that specifically recognizes the HBV polymerase, X protein, preCore, HBcAg, HBeAg, preS1, preS2 or HBsAg protein of genotype G.

It is another aim of the present invention to provide an antibody that recognizes the HBV polymerase, preCore, HBcAg, HBeAg, preS1, preS2 or HBsAg protein of genotypes A, B, C, D, E, F and G.

It is another aim of the present invention to provide a method for the detection of a polypeptide of the invention.

It is another aim of the present invention to provide a diagnostic kit that allows the detection of a polypeptide of the invention.

It is another aim of the present invention to provide a monoclonal antibody for the prevention or treatment of HBV infections.

It is another aim of the present invention to provide a vaccine or a pharmaceutical composition for the prevention or treatment of HBV infections.

It is another aim of the present invention to provide a method to identify compounds or agents that can be used for the prevention or treatment of HBV infections.

It is another aim of the present invention to provide an HBV genotype G infected clone for use in a drug-screening assay.

### FIGURE LEGENDS

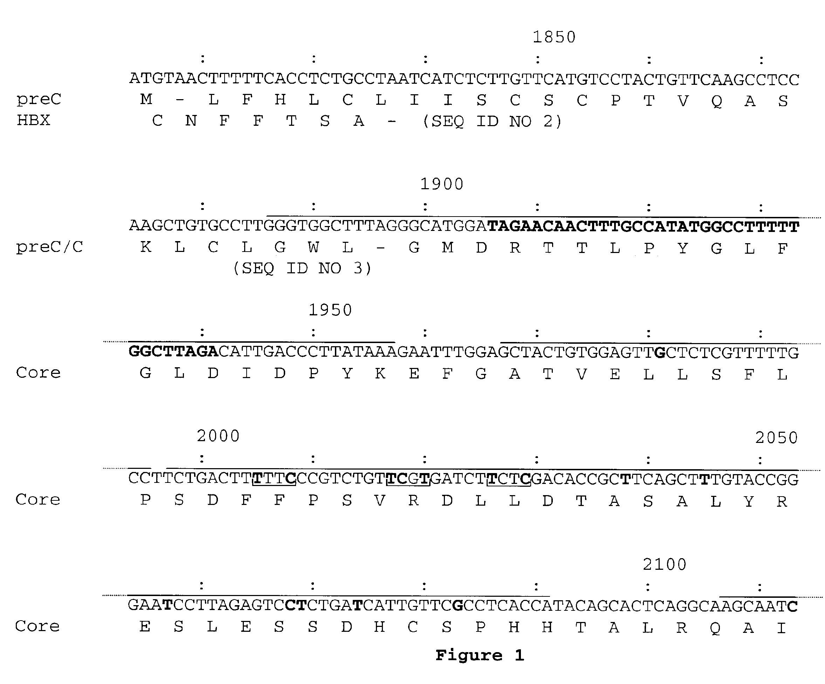

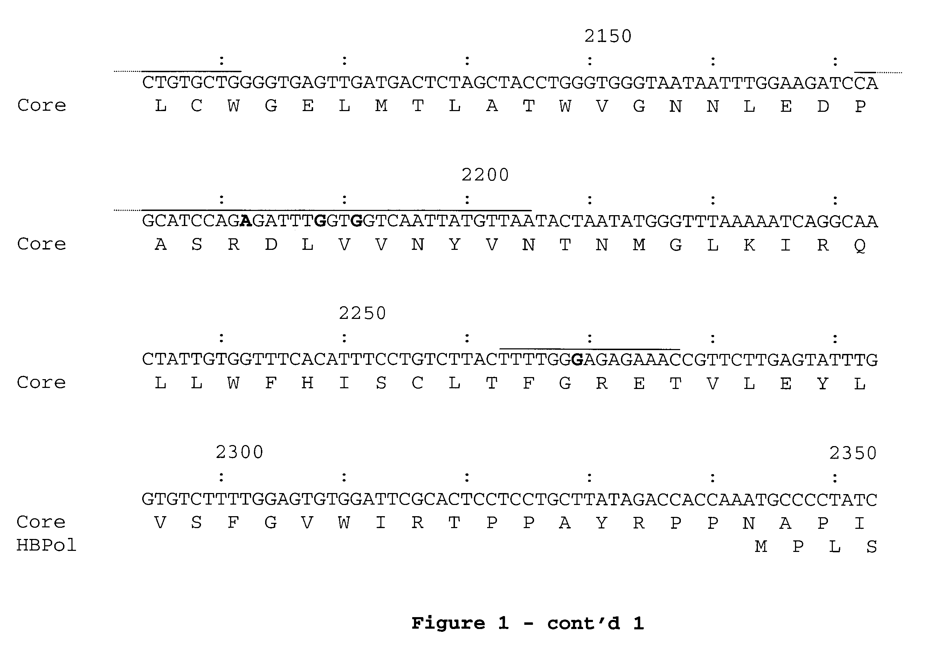

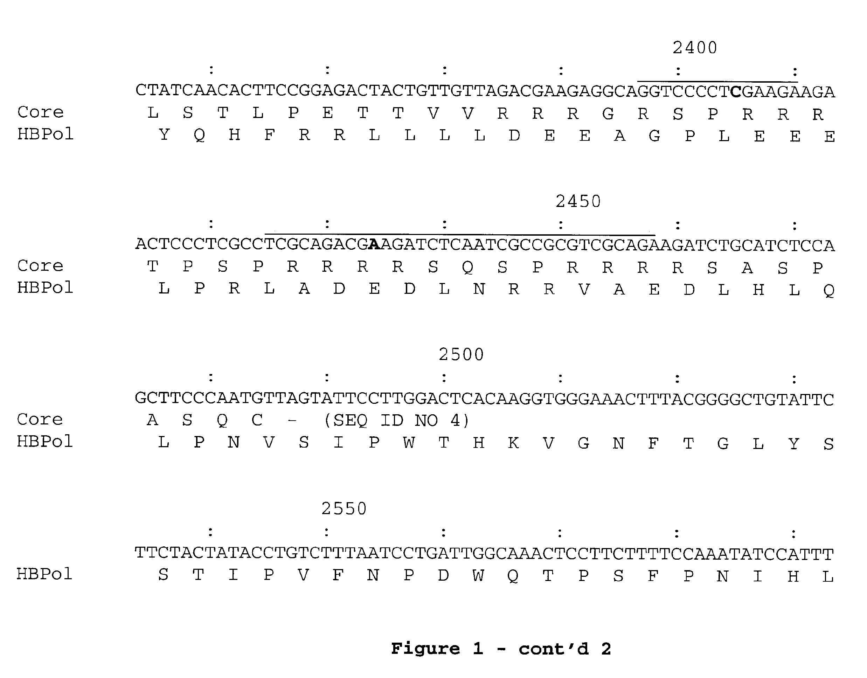

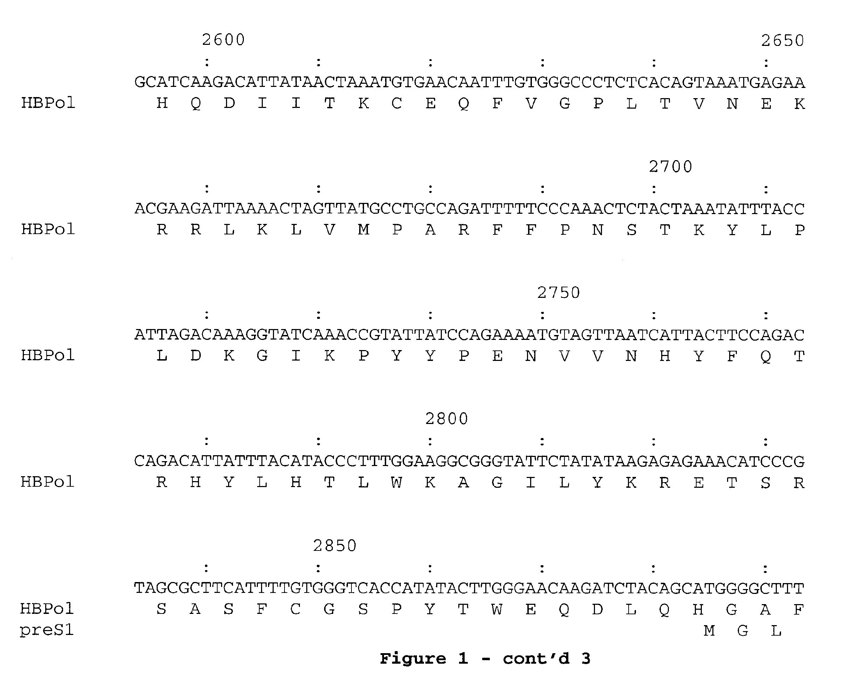

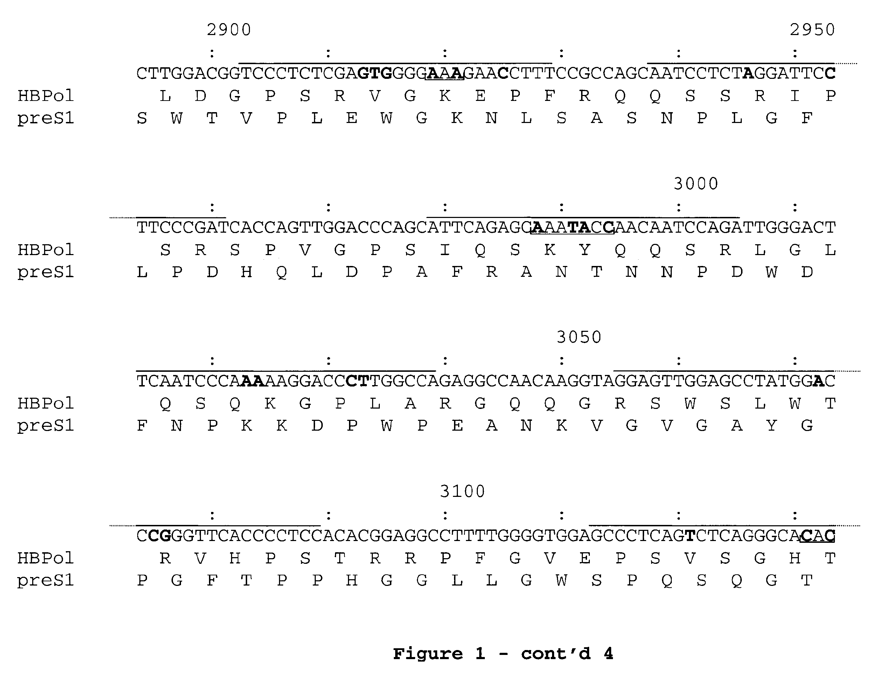

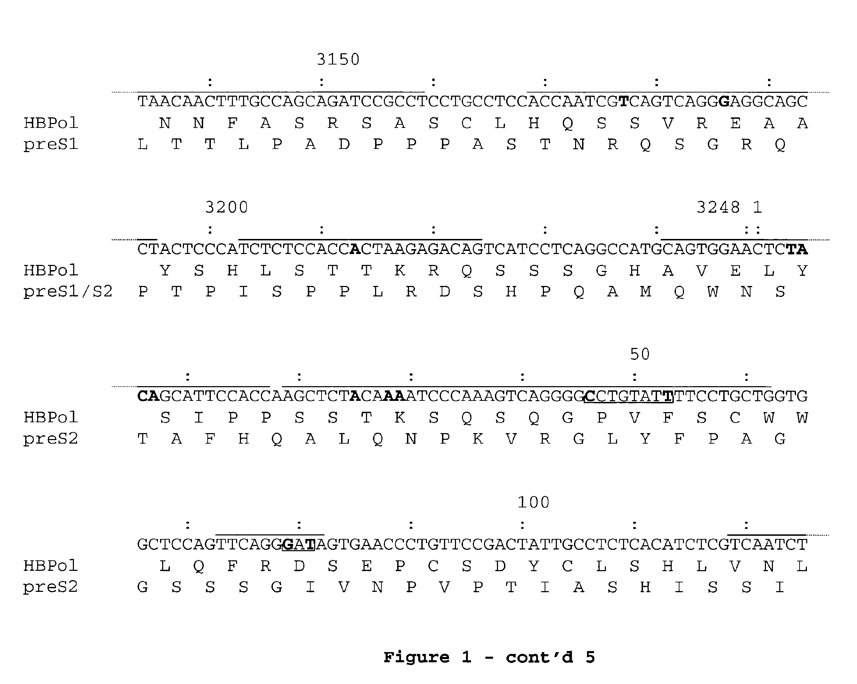

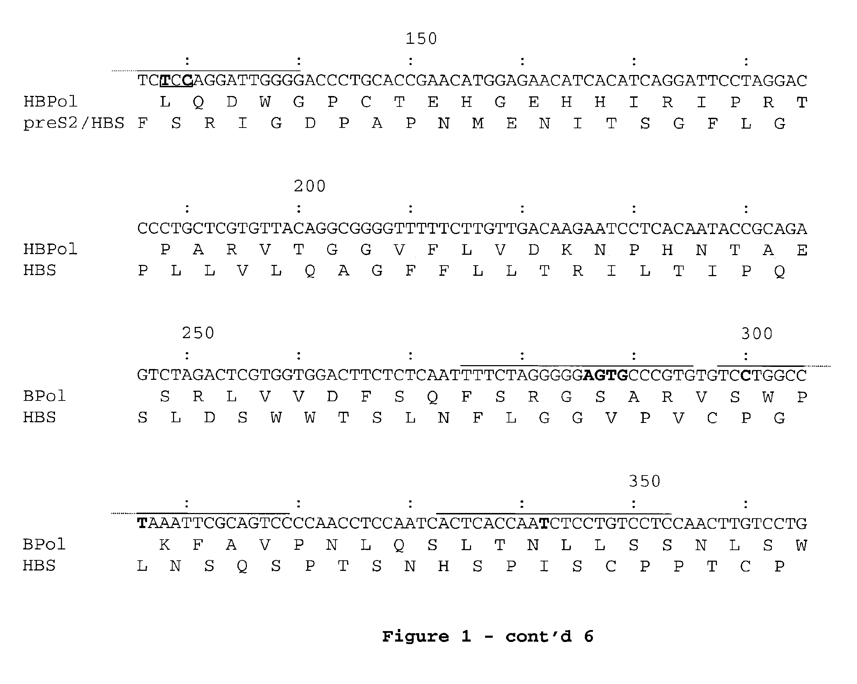

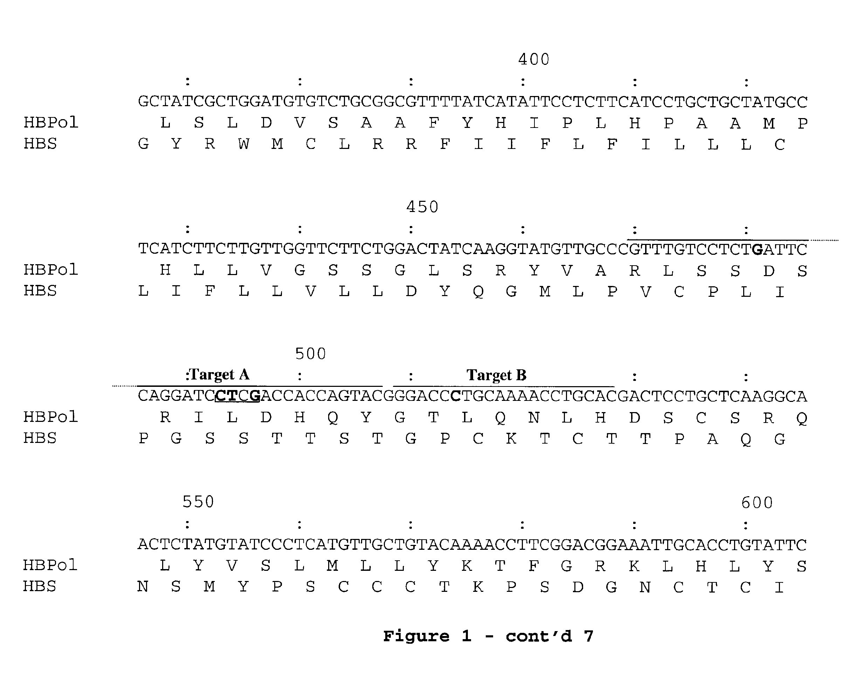

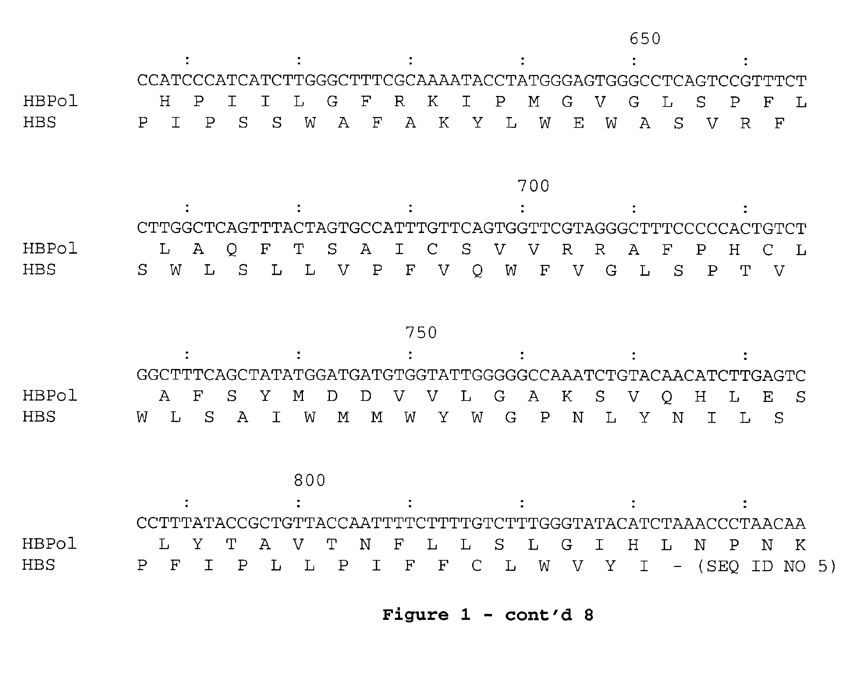

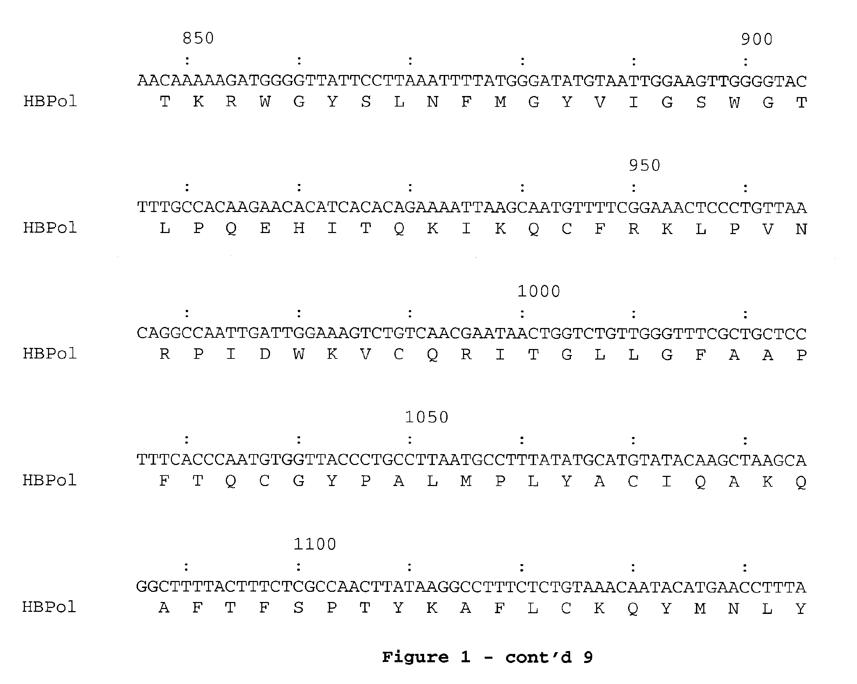

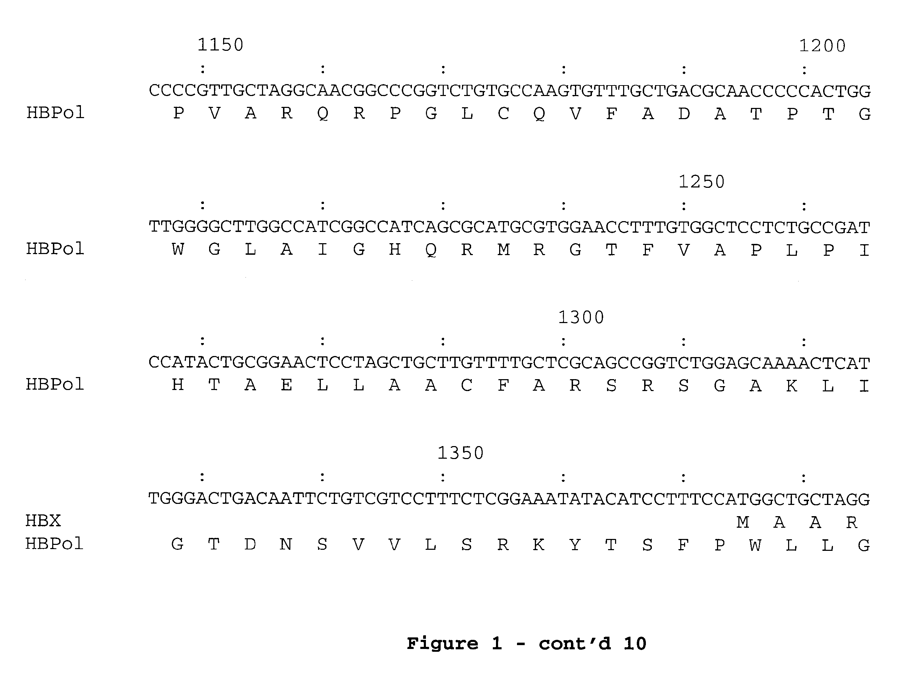

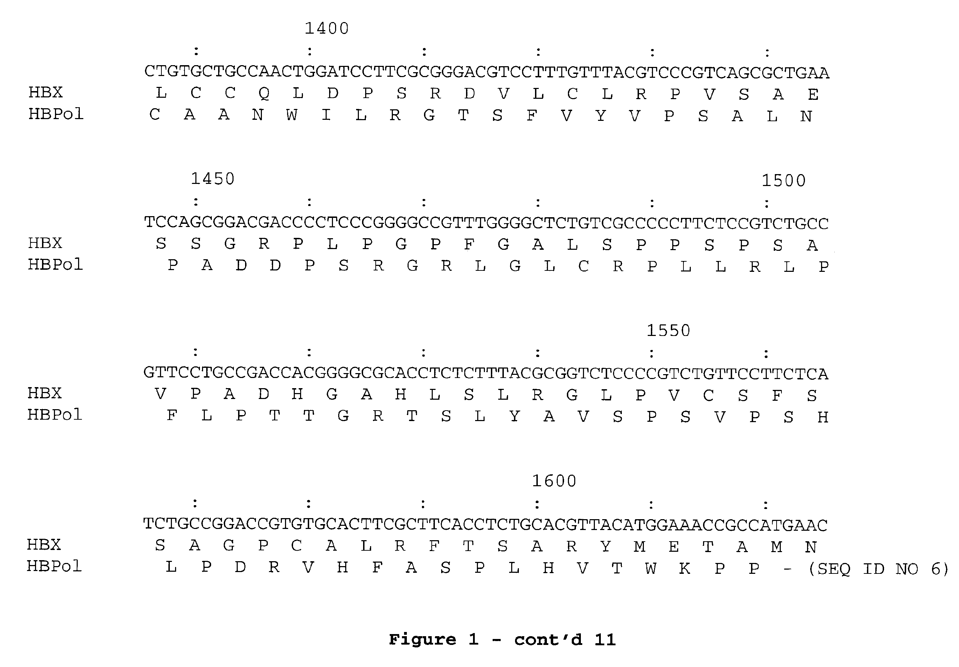

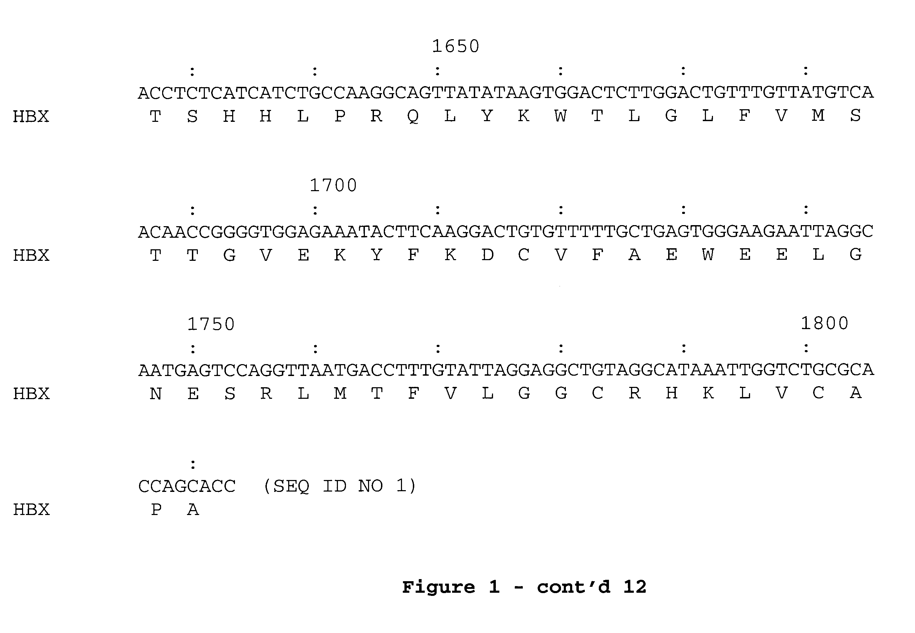

**Figure 1.** Complete nucleotide sequence of FR1 with amino acid translation of the four major ORFs. Target sequences for HBV genotype G specific probes and/or primers are indicated with an horizontal line. Nucleotides and nucleotide combinations that are unique to HBV genotype G are shown in bold and with a 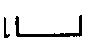 , respectively.

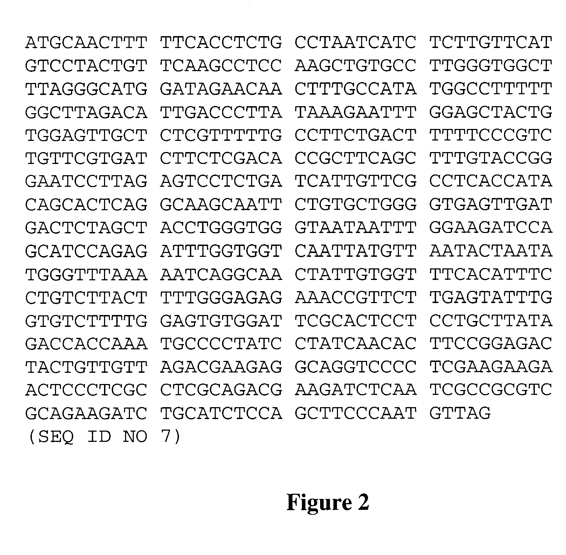

**Figure 2.** Complete coding sequence of the HBV preC/C gene as described by Tran et al. (1991).

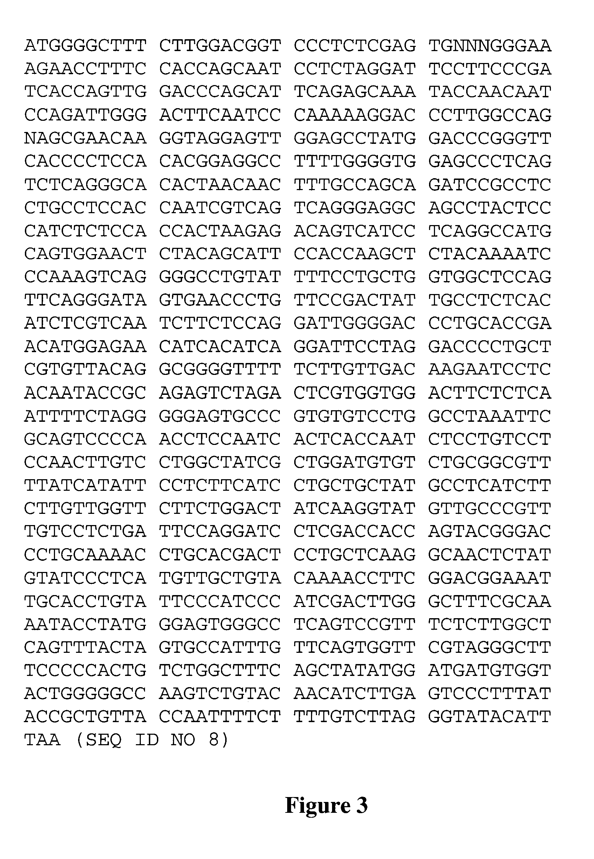

**Figure 3.** Complete coding sequence of the HBV preS/S gene as described by Tran et al. (1991).

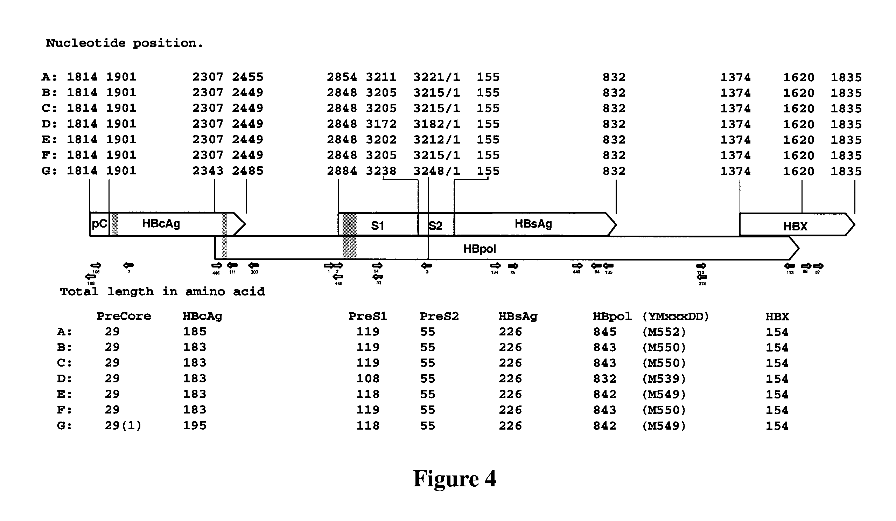

**Figure 4.** The genome organization and open reading frames of HBV genotype G virus compared to other genotypes. For each genotype, only one representative genome is included (genotype A: X70185; B: D00331; C: X01587; D: X72702; E: X75664; F: X75663; G: FR1). Positions in the viral genome where extensive variability is observed between the genotypes, are indicated as a grey zone. Nucleotide and amino acid numbering is indicated. (1): Most isolates of genotype G contain translational stop codons, influencing the length of the preCore region. xxx: stands for the amino acid numbering for the M-residue in the conserved YMDD motif of the different genotypes. The positions and orientation of the primers shown in table 1 are indicated with arrows.

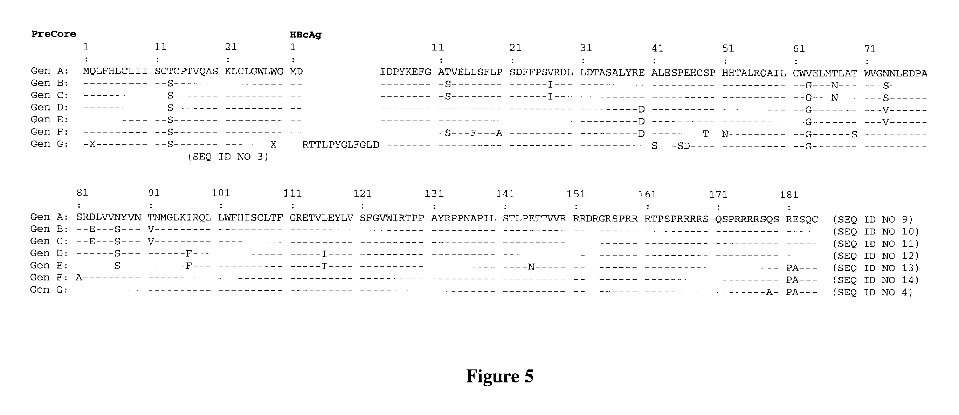

**Figure 5.** Amino acid sequence alignment of the preCore and Core regions of the different HBV genotypes. For each genotype, only one representative genome is included (genotype A: X70185; B: D00331; C: X01587; D: X72702; E: X75664; F: X75663; G: FR1). The amino acid sequence was derived from the nucleotide sequence. X: translational stop.

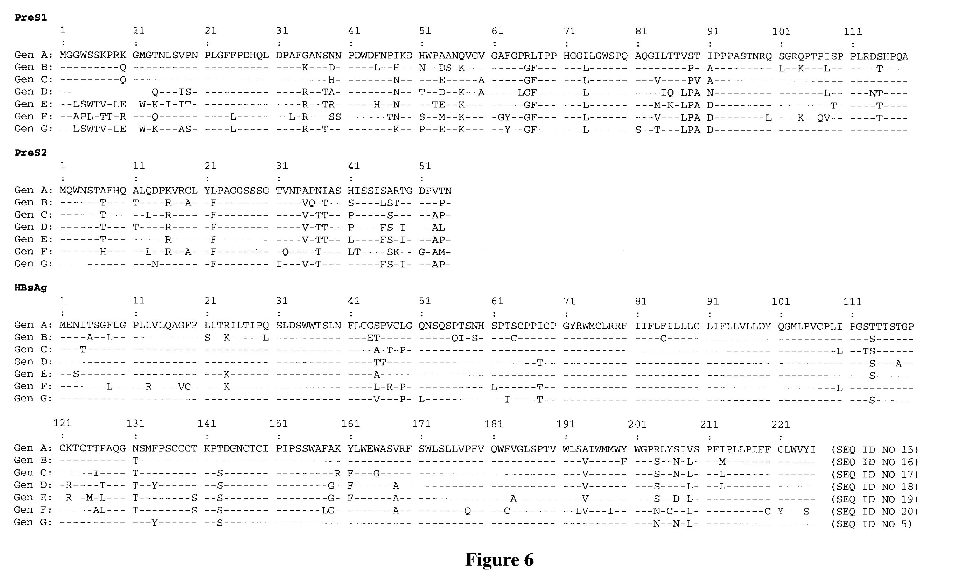

**Figure 6.** Amino acid sequence alignment of the preS1, preS2 and HBsAg open reading frame of the different HBV genotypes. For each genotype, only one representative genome is included (genotype A: X70185; B: D00331; C: X01587; D: X72702; E: X75664; F: X75663; G: FR1). Top: preS1; middle: preS2; bottom: HBsAg. The amino acid sequence was derived from the nucleotide sequence.

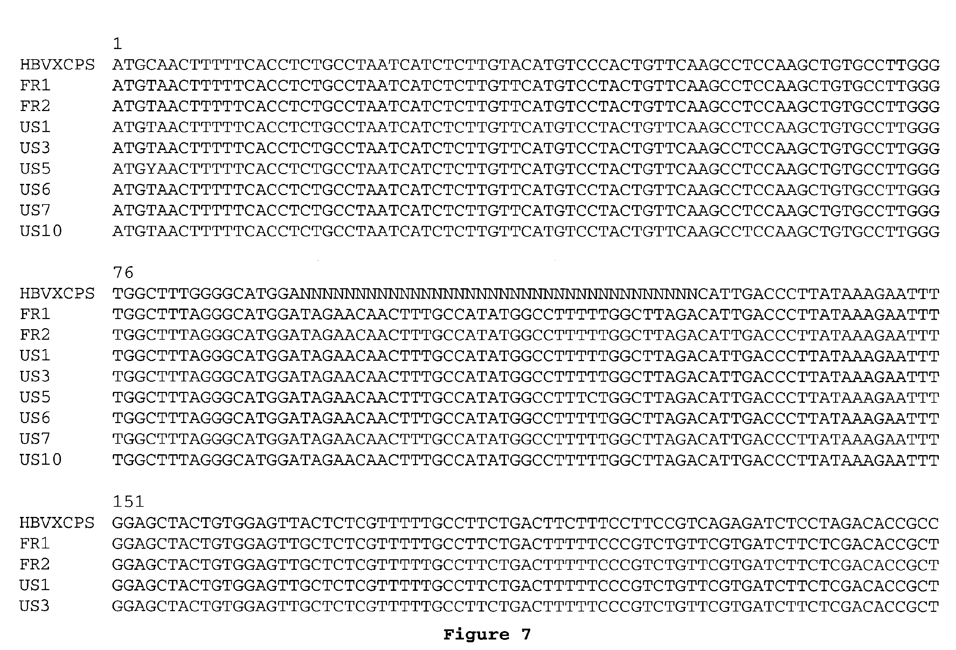

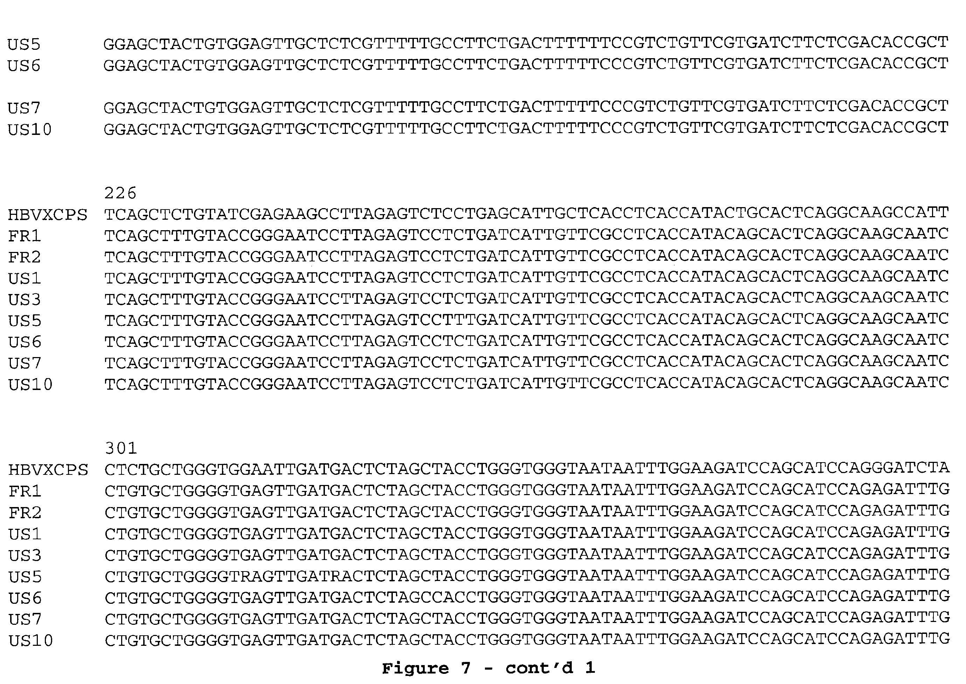

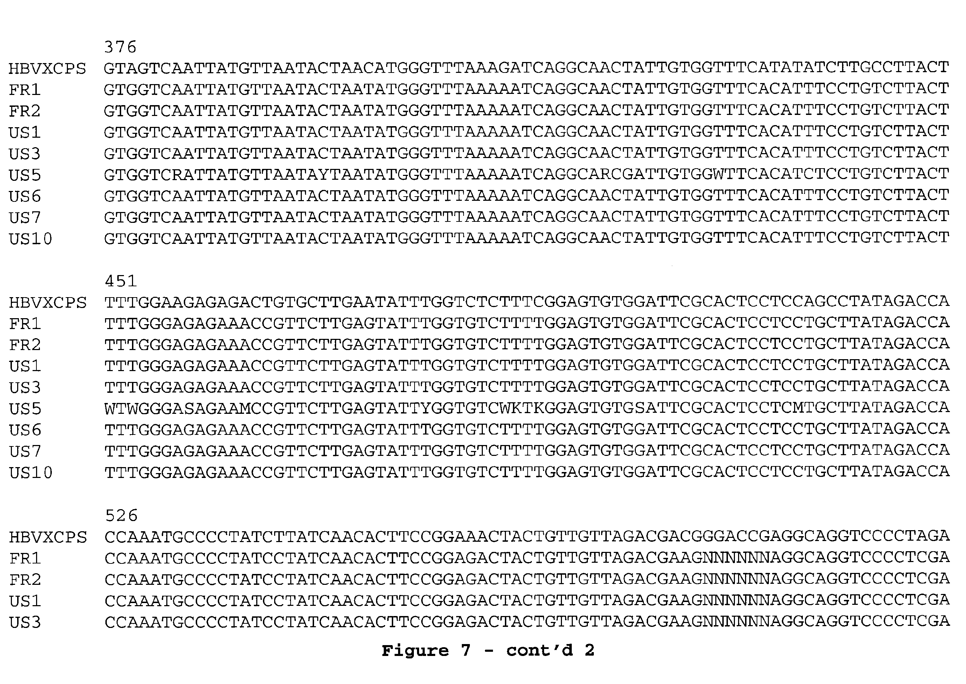

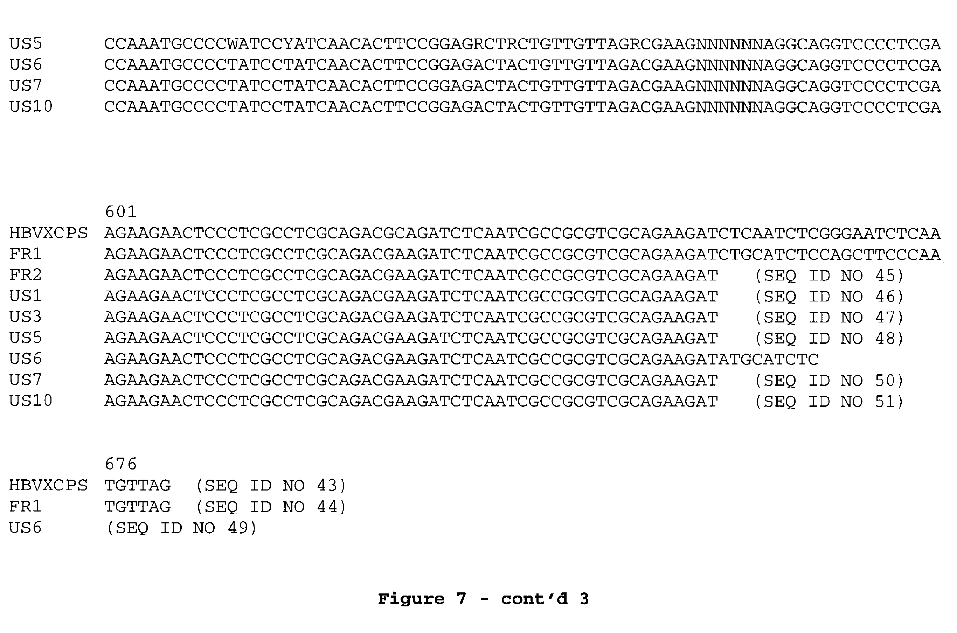

**Figure 7.** Alignment of preCore/Core sequences of a HBV genotype A strain (HBVXCPS) and of 7 HBV strains that belong to the newly identified genotype G (FR1, FR2, US1, US3, US5, US6, US7, US10). N indicates the absence of a nucleotide at this position.

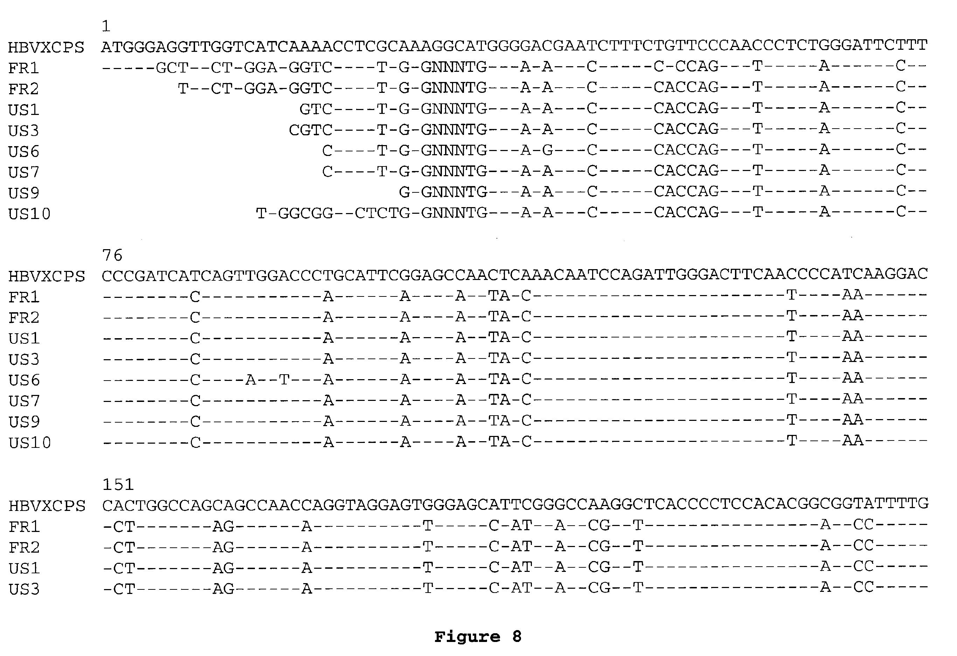

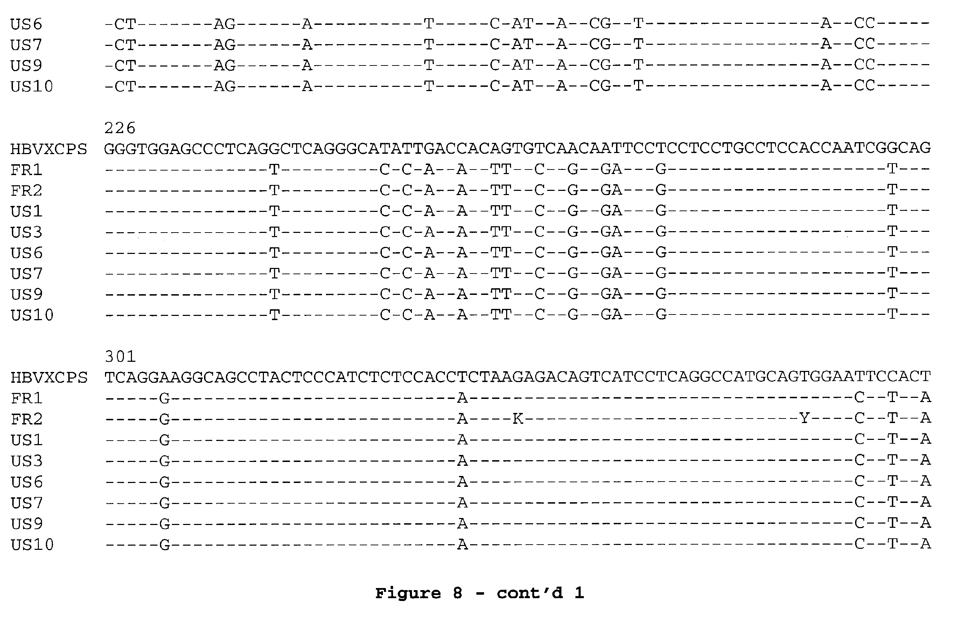

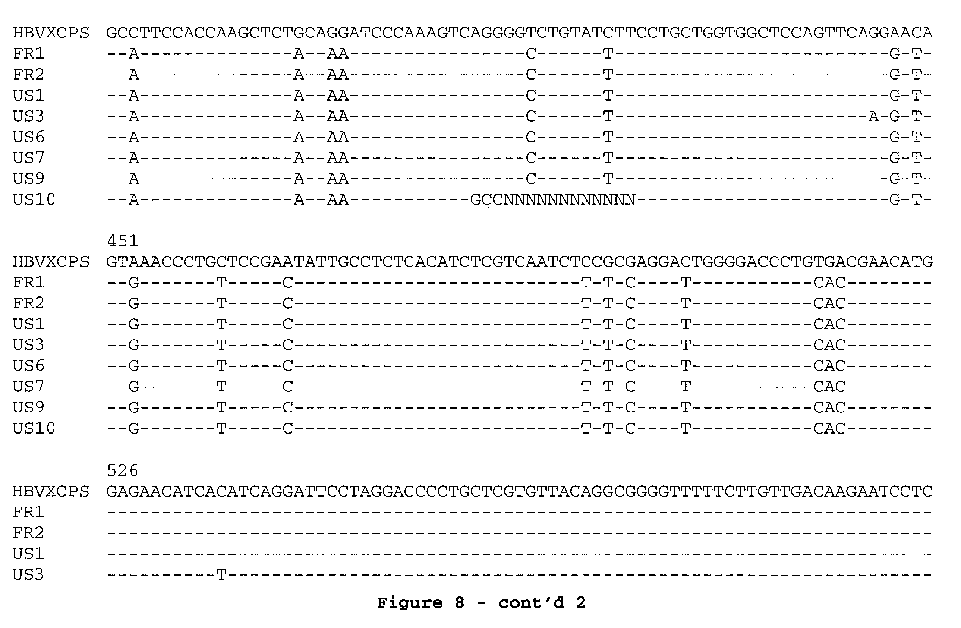

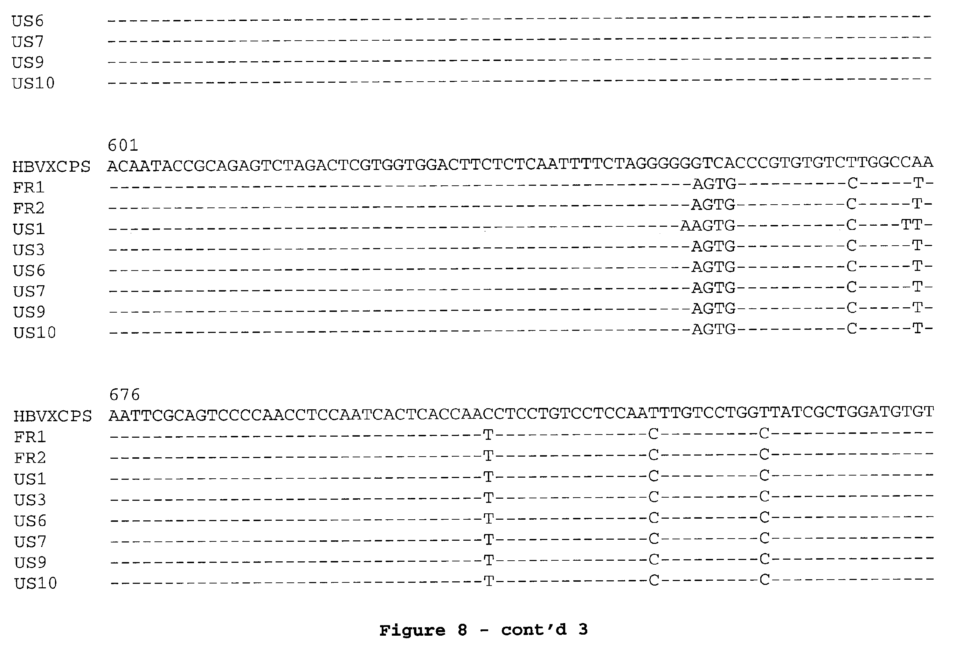

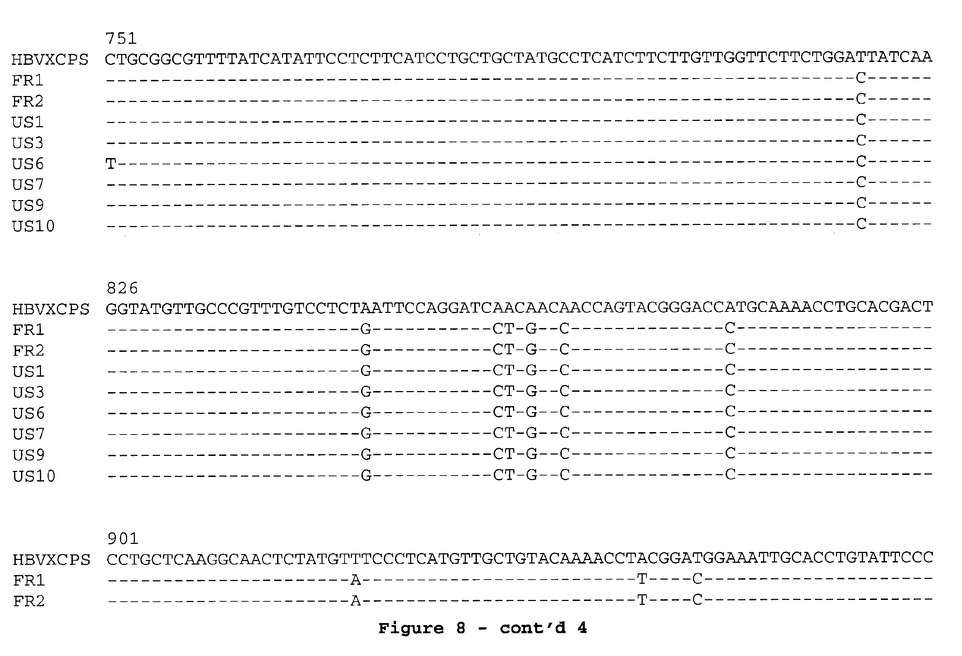

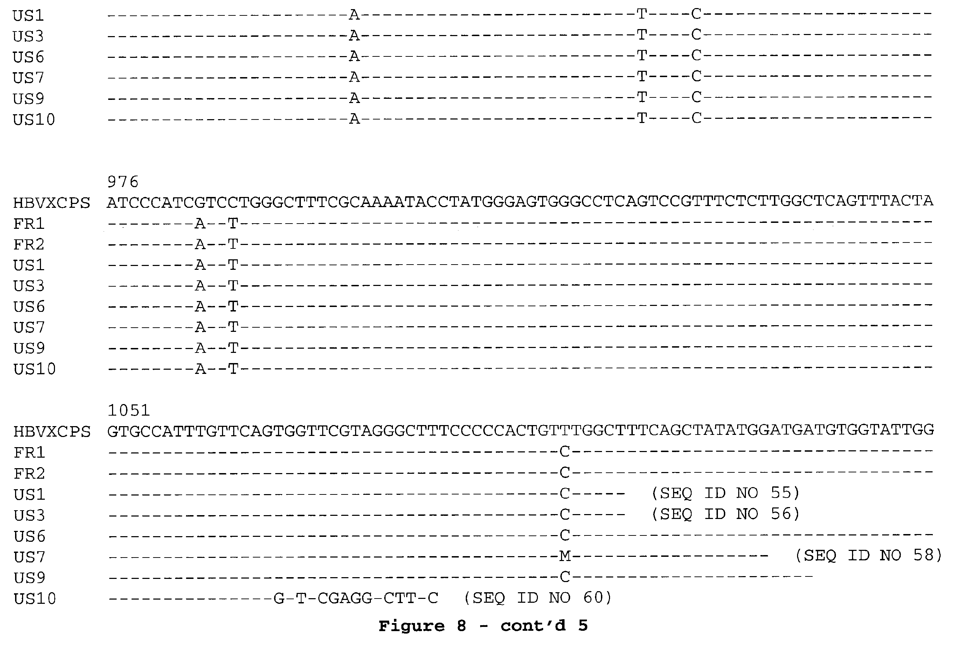

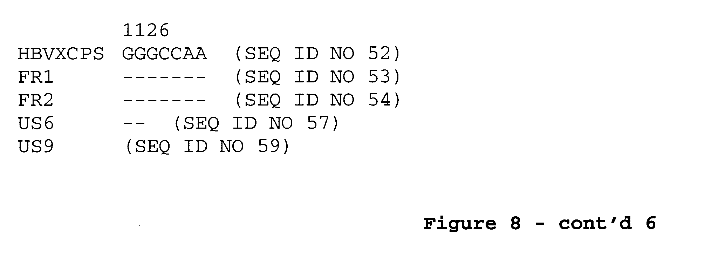

**Figure 8.** Alignment of preS1/preS2/HBsAg sequences of a HBV genotype A strain (HBVXCPS) and of 7 HBV strains that belong to the newly identified genotype G (FR1, FR2, US1, US3, US6, US7, US9, US10). N indicates the absence of a nucleotide at this position.

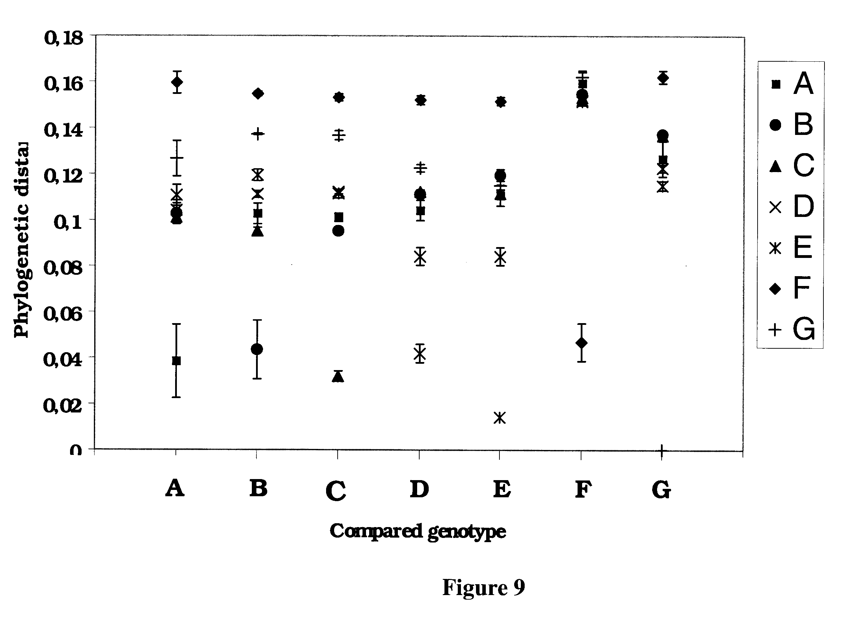

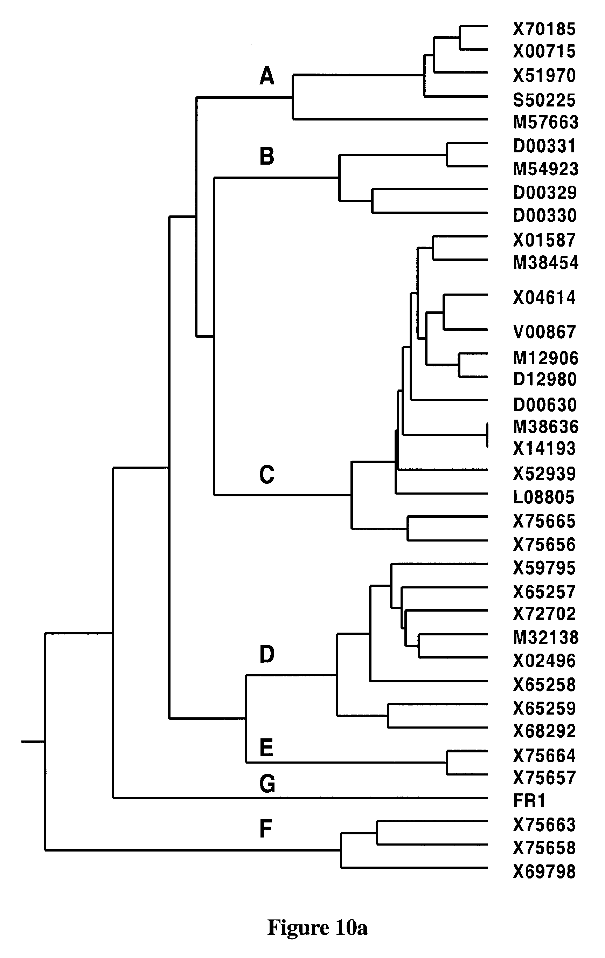

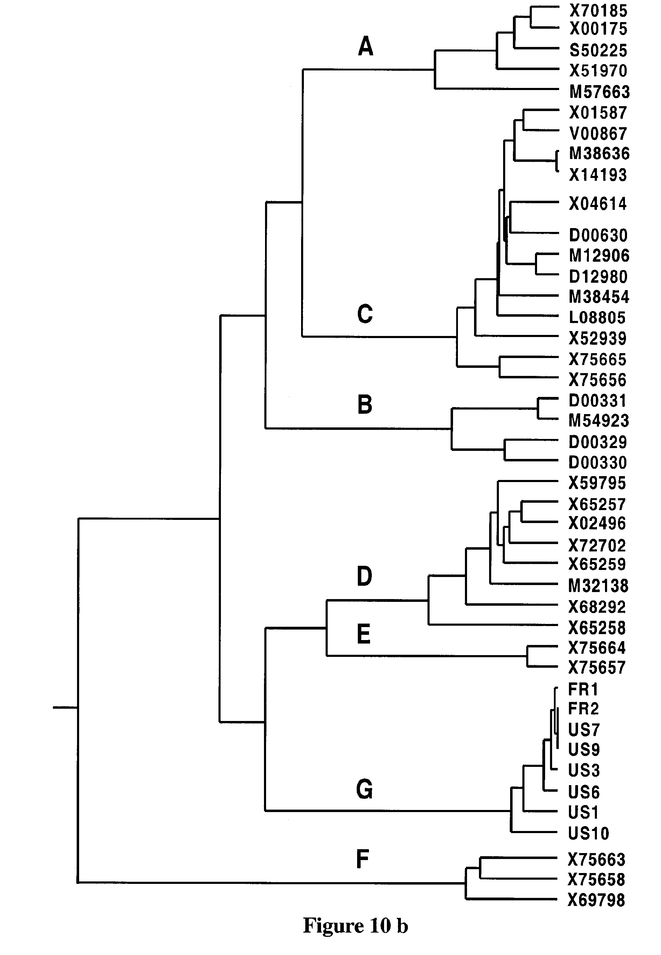

**Figure 9.** Phylogenetic distances, obtained by the program DNADIST, between the different HBV genotypes. In total 36 complete genomes (accession numbers are given in Fig. 10) were compared with the FR1 (genotype G) sequence. The mean for each group is indicated (A: ■; B: ●; C: ▲; D: ×; E: ∗; F:◆; G: 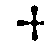 ), including the standard error of the mean; the latter gives a 95% confidence interval around these distances.

**Figure 10.** Phylogenetic trees of the HBV genotypes. Virus isolates are indicated by their GenBank accession number. (a) Complete genomes. (b) Open reading frame of the surface gene (including preS1/preS2/HBsAg).

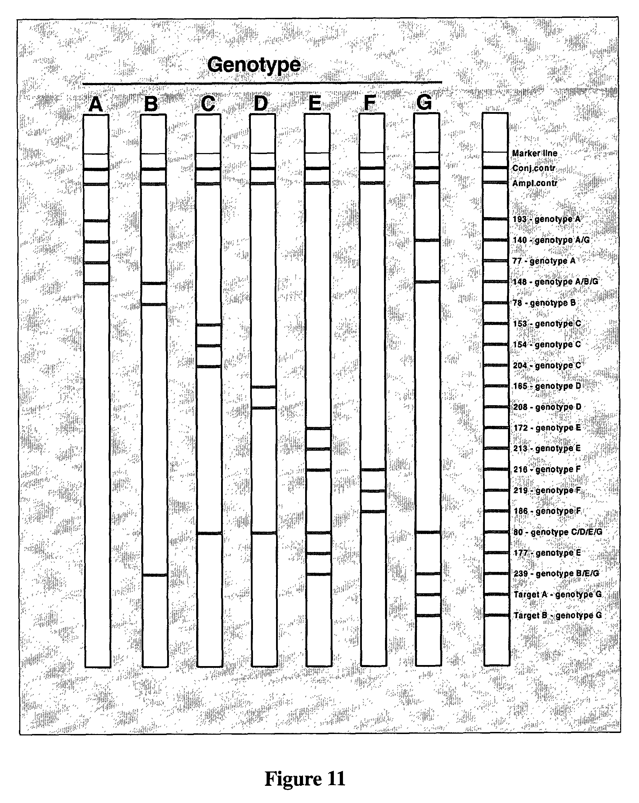

**Figure 11.** Result obtained with some representative samples of genotypes A, B, C, D, E, F and G after hybridization of their PCR product to the genotyping LiPA as designed in example 4, Strip G: incubated with PCR fragments of FR1. The right strip shows the positions of all probes applied. The numbering of the probes is described in Van Geyt et al. (1998). The probe lines with "target A" and "target B" contain probes recognizing the target A and target B region as indicated in figure 1. Conj.contr: conjugate control; Ampl.contr: amplification control (probe reacting with all genotypes, located in conserved area).

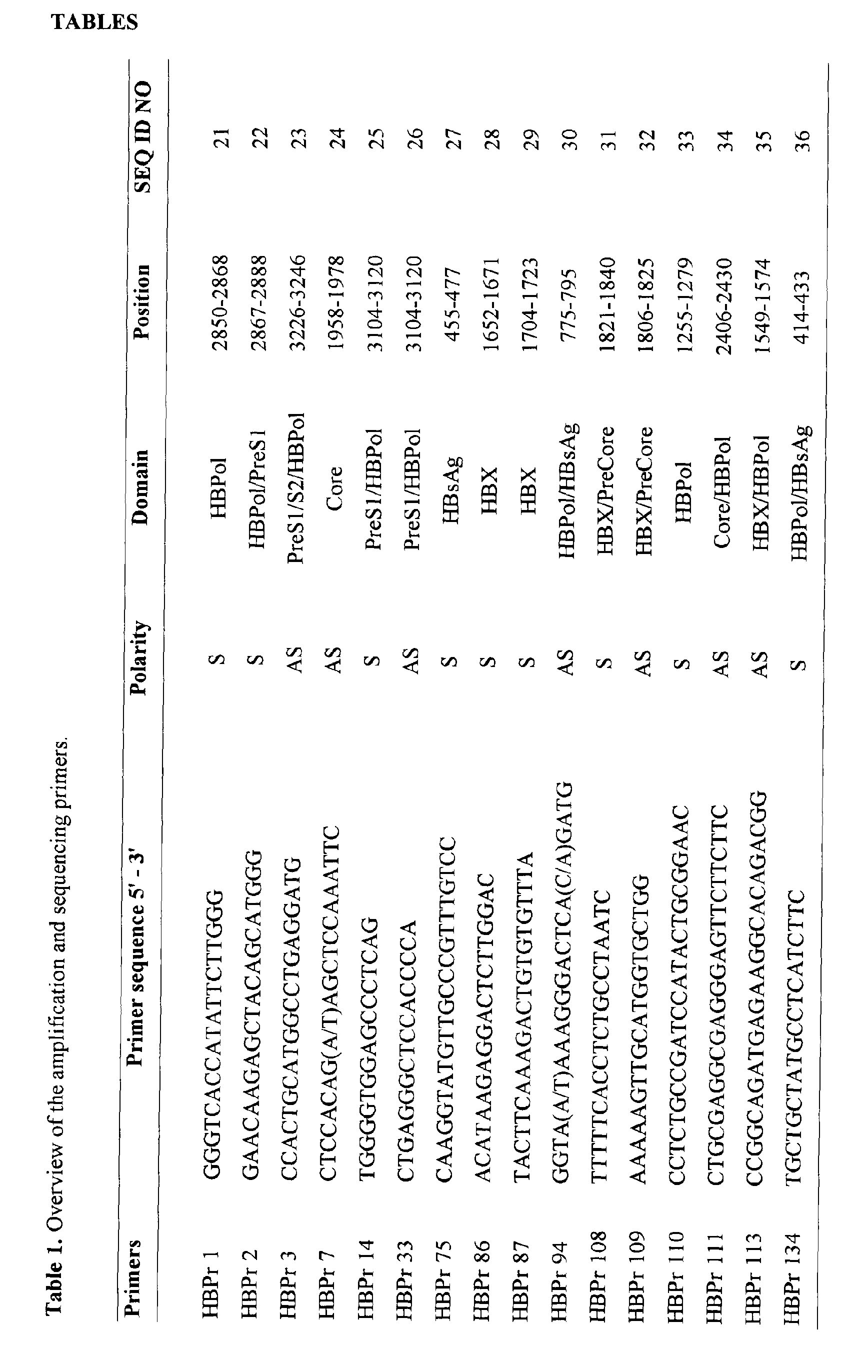

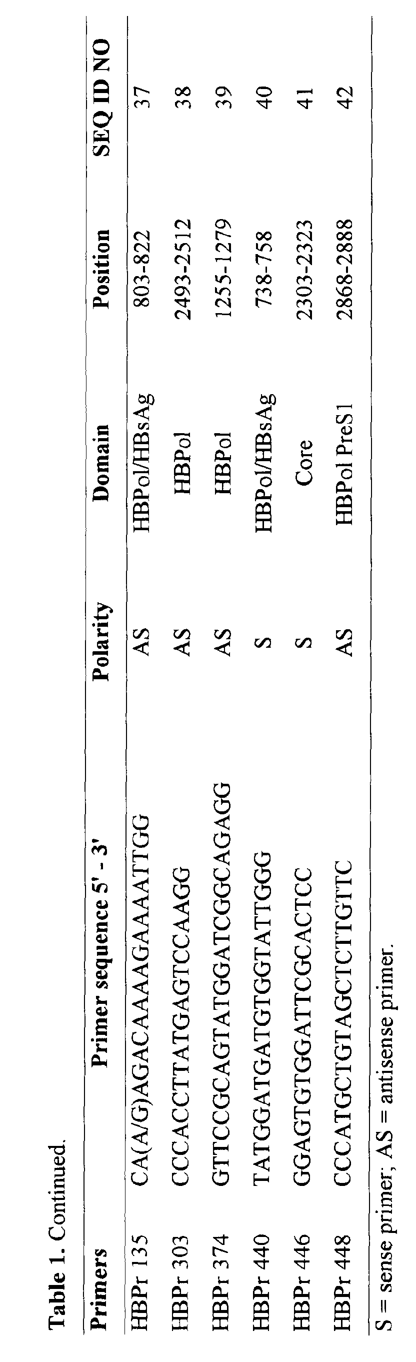

**Table 2.**

| Summary of the genotyping result obtained from 121 individuals infected with HBV. | | | | | | | | |
|---|---|---|---|---|---|---|---|---|
| Genotype | A | B | C | D | E | F | G | Total |
| France | 18 | 0 | 2 | 16 | 1 | 0 | 2 | 39 |
| Georgia, | 48 | 4 | 12 | 7 | 0 | 0 | 11 | 82 |
| U.S. | | | | | | | | |
| Total | 66(54%) | 4(3%) | 14(12%) | 23(19%) | 1(1%) | 0(0%) | 13(11%) | 121(100%) |

### DETAILED DESCRIPTION OF THE INVENTION

The present invention relates to an isolated and/or purified HBV polynucleic acid, having a nucleotide sequence which is unique to a theretofore-unidentified HBV genotype and which is phylogenetically different from HBV genotypes A, B, C, D, E and F characterized by:
(a) a sequence as defined in figure 1 (SEQ ID NO 1); or
(b) a sequence with at least 90, more preferably 91, most preferably 92% identity to the sequence as defined in figure 1; or
(c) a sequence which is degenerate as a result of the genetic code to the sequence as defined in (a) and (b);

An "individual genetic group" or "phylogenetically different genotype" of HBV can be defined if a certain viral strain differs by more than 8% from all other HBV genomes (Okamoto et al., 1988; Nordor et al., 1994). Thus, virus strains differing from other isolates by more than 8% over their entire genome (not focussing on possible deletions or insertions) are classified as new genotypes. Based on results obtained from several epidemiological studies (Blitz et al., 1998; Norder et al., 1994; Magnius and Norder, 1995; Telenta et al., 1997; Alvarado-Esquivel et al., 1998), six major genetic groups of HBV (A, B, C, D, E and F) were recognized. Apart from the detection of these commonly found genotypes, in a prevalence study on samples from chronic HBV carriers from Atlanta, USA and Lyon, France, the present inventors have isolated a new viral HBV strain with at least 11.7% (new genotype versus genotype E) and at most 15.3% (new genotype versus genotype F) divergence over the complete genome. Accordingly, this new viral HBV strain has been classified as a new HBV genotype G. Surprisingly, the prevalence of this viral variant in the US exceeded 11% of all infections, although it had not been identified in all of the previous molecular epidemiology studies. In addition to the prominent prevalence of this new genotype G virus in the USA samples, it was also found in samples originating from France.

The term "isolated" refers to material that is free from components that normally accompany it as found in its naturally occurring environment. However, it should be clear that the isolated nucleic acid of the present invention might comprise heterologous cell components or a label and the like.

The terms "nucleic acid" or "polynucleic acid" are used interchangeable throughout the present application and refer to a deoxyribonucleotide or ribonucleotide polymer in either single- or double stranded form, which may encompass known analogues of natural nucleotides that hybridize to nucleic acids in a manner similar to naturally occurring nucleotides. The term specifically refers to the DNA sequence as defined in figure 1 and given by SEQ ID NO 1 or the complementary strand thereof. Also within the scope of the present invention are all HBV nucleic acids that belong to the new HBV genotype G and that thus differ 10%, more preferably 9%, most preferably 8% or less from the nucleotide sequence as depicted in figure 1 or that are at least 90%, more preferably 91%, most preferably 92% identical to the sequence as depicted in figure 1.

A nucleic acid sequence that "differs 10%, 9%, 8% or less from another nucleic acid sequence" is a nucleic acid sequence of which respectively 10%, 9%, 8% or less of its total nucleotides are different from the nucleotides of the nucleic acid sequence it is compared with.

A nucleic acid sequence that "is at least 90%, 91%, or 92% identical to another nucleic acid sequence" is a nucleic acid sequence of which respectively at least 90%, 91%, or 92% of its total nucleotides are identical to the nucleotides of the nucleic acid sequence it is compared with.

As a result of the genetic code, also nucleic acid sequences that show less than 90%, more preferably 91%, most preferably 92% identity to the sequence as depicted in figure 1 can encode the same proteins as is encoded by the nucleotide sequences of HBV strains belonging to the new genotype G. Therefore also these nucleic acid sequences, that are degenerate as a result of the genetic code, fall within the scope of the present invention.

In contrast to the known HBV genotypes, the complete genome of the HBV sequence of the invention, belonging to the newly identified genotype G, was found to be 3248 bp long (Fig. 4). The preCore region has translational stops at codon 2 (TAA instead of CAA) and codon 28 (TAG instead of TGG) (Fig. 5). The Core region is 585 nucleotides long, encoding a Core protein of 195 amino acids, and has a nucleotide insert of 36 bp, located after the fifth nucleotide following the Core translation initiation point (A at position 1901). Similar to all other genotypes except genotype A, the HBV sequence belonging to genotype G showed a 6 nucleotide deletion in the carboxy-terminal part of the HBcAg ORF (Figs. 4 and 5). The preS1 region contains 354 bp (118 amino acids), the presS2 region 165 bp (55 amino acids) and the HBsAg region 678 bp (226 amino acids) (Fig. 6). Like genotype E, the HBV sequence belonging to genotype G showed a 3 nucleotide deletion (1 amino acid at position 11; Fig. 6) in the amino-terminal part of preS1.

In accordance, the present invention specifically relates to an HBV polynucleic acid as described above, further characterized that its nucleotide sequence differs from HBV genotype A, B, C, D, E or F in at least one of the following characteristics:
(a) it has a length of 3248 basepairs;
(b) it encodes a translational stop codon at amino acids 2 and 28 of the preCore region;
(c) it has a 36 nucleotide insert in the N-terminal part of the Core region;
(d) it has a 6 nucleotide deletion in the C-terminal part of the HBcAg ORF;
(e) it has a 3 nucleotide deletion in the N-terminal part of the preS1 ORF.

In this specific embodiment, the HBV polynucleic acid sequence of the invention thus has 1, 2, 3, 4 or 5 of the above mentioned characteristics.

In a more specific embodiment, the present invention relates to an HBV polynucleic acid as described above, comprising at least one of the following sequences (Figs. 7 and 8): SEQ ID NO 44, SEQ ID NO 45, SEQ ID NO 46, SEQ ID NO 47, SEQ ID NO 48, SEQ ID NO 49, SEQ ID NO 50, SEQ ID NO 51, SEQ ID NO 53, SEQ ID NO 54, SEQ ID NO 55, SEQ ID NO 56, SEQ ID NO 57, SEQ ID NO 58, SEQ ID NO 59, SEQ ID NO 60.

The present invention further relates to a fragment of a polynucleic acid as described above characterized that said fragment is unique to the theretofore-unidentified HBV genotype G and that said fragment contains at least one nucleotide or a combination of nucleotides different from known HBV genotypes A, B, C, D, E or F nucleotide sequences, or the complement of said fragment, provided that said fragment is not equal to the preC/C gene sequence as depicted in figure 2 (SEQ ID NO 7) or the preS/S gene sequence as depicted in figure 3 (SEQ ID NO 8).

Tran et al. (1991) have described the preS/S and preC/C sequences of an HBV recombinant strain which accidentally differ less than 8% from the preS/S and preC/C sequences of the strains of the present invention. In contrast to the newly identified HBV genotype G of the present invention, however, this atypical HBV strain was described in the context of an event of emergence of and take over by HBV, with rearrangements in the preS/S and preC/C genes (Tran et al., 1991). This atypical HBV strain was never recognized as a new genotype. In order to study the genotype G sequence with respect to such recombination events (as described by Tran et al. 1991), the present inventors have inspected the complete FR1 genome for such events on the nucleotide level, and phylogenetically for co-segregation with other known genotypes. Evidence for recombination was not found. The absence of recombination events in the newly isolated strain and the high prevalence of this new viral variant indicate that it concerns a new HBV genotype. The accidentally homologous HBV sequences described by Tran et al. (1991) which have emerged only as a result of a recombination event, are disclaimed from the present invention. In accordance, the HBV preC/C gene sequence as shown in figure 2 (SEQ ID NO 7; Accession number M74501; Tran et al. 1991) and the preS/S gene sequence as shown in figure 3 (SEQ ID NO 8; Accession number M74499; Tran et al. 1991) do not form part of the present invention.

The present invention also relates to a fragment of a polynucleic acid as described above characterized that said fragment is a universal fragment which is comprised in nucleic acid sequences of HBV genotypes A, B, C, D, E, F and G. Since the present invention provides nucleic acid alignments of 7 genotypes, the invention allows for the first time to delineate universal HBV nucleic acids sequences, characteristic for HBV genotypes A, B, C, D, E, F and G.

The term "universal" as used in the present application means that it is present in, correspondents to, or is characteristic to HBV genotypes A, B, C, D, E, F, and G.

The fragment of the invention can be single stranded or double stranded. It can be from 5 to 3247 bp long. More preferably this fragment is from 5 to 2600 bp long, from 5 to 1300 bp long, from 5 to 700 bp long, from 5 to 500 bp long, from 5 to 300 bp long and most preferably from 5 to 200 bp long. The fragment can be used for cloning and expression or the fragment can be used as primer or a probe in an HBV typing method.

The present invention therefore also relates to an oligonucleotide primer comprising a fragment as described above, with said primer being a HBV genotype G specific primer, able to act as primer for specifically sequencing or specifically amplifying a nucleic acid or part of a nucleic acid characteristic to HBV genotype G. The present invention also relates to an oligonucleotide primer comprising a fragment as described above, with said primer being a universal primer, able to act as primer for sequencing or amplifying nucleic acids from HBV genotypes A, B, C, D, E, F and G. The term "primer" refers to a single stranded oligonucleotide sequence capable of acting as a point of initiation for synthesis of a primer extension product that is complementary to the nucleic acid strand to be copied. The length and the sequence of the primer must be such that they allow to prime the synthesis of the extension products. Preferably, the length of the primer is about 5-50 nucleotides. More preferably, the length of the primer is about 10-30 nucleotides. Most preferably, the length of the primers is about 20-25 nucleotides. Specific length and sequence will depend on the complexity of the required DNA or RNA target, as well as on the conditions at which the primer is used, such as temperature and ionic strength.

The present invention also relates to an oligonucleotide probe comprising a fragment as described above, with said probe being a HBV genotype G specific probe, able to act as a hybridization probe for the specific detection of a nucleic acid or part of a nucleic acid characteristic to HBV genotype G. The present invention also relates to an oligonucleotide probe comprising a fragment as described above, with said probe being a universal probe, able to act as a hybridization probe for nucleic acids from HBV genotypes A, B, C, D, E, F and G. The term "probe" refers to a single stranded sequence-specific oligonucleotide that has a sequence that is complementary to the target sequence in the HBV genome. Preferably, these probes are about 5 to 50 nucleotides long, more preferably from about 10 to 25 nucleotides. Particularly preferred lengths of probes include 10, 11, 12, 13, 14, 15, 16, 17, 18, 19, 20, 21, 22, 23, 24 or 25 nucleotides. The nucleotides used in the probes of the present invention may be ribonucleotides, deoxyribonucleotides and modified nucleotides such as inosine, or nucleotides containing modified groups that do not essentially alter their hybridization characteristics.

The probes and/or primers according to the invention can be prepared by cloning recombinant plasmids containing inserts including the corresponding nucleotide sequence, if need be by excision of the latter from the cloned plasmids by use of the adequate nucleases and recovering them, e.g. by fractionation according to molecular weight. The probes according to the present invention can also be synthesized chemically, for instance by the conventional phospho-triester method. They can also be part of a branched DNA capture or detection probe (Sanchez-Pescador et al., 1988; Urdea et al., 1991)

The primers and/or probes of the invention may be labeled. Labeling may be carried out by any method known to the person skilled in the art. The nature of the label may be isotopic (³²P, ³⁵S, etc.) or non-isotopic (biotin, digoxigenin, etc.) or may reside on a branched DNA probe.

The oligonucleotides used as primers and/or probes may also contain or consist of nucleotide analogues such as phosphorothiates (Matsukura et al., 1987), alkylphosphorothiates (Miller et al., 1979) or peptide nucleic acids (Nielsen et al., 1991; Nielsen et al., 1993) or may contain intercalating agents (Asseline et al., 1984). The introduction of these modifications may be advantageous in order to positively influence characteristics such as hybridization kinetics, reversibility of the hybrid-formation, biological stability of the oligonucleotide molecules, etc.

As most other variations or modifications introduced into the original DNA sequences of primers and/or probes, these variations will necessitate adaptations with respect to the conditions under which the oligonucleotide should be used to obtain the required specificity and sensitivity. The eventual results of the priming or hybridization with these modified oligonucleotides, however, should be essentially the same as those obtained with the unmodified oligonucleotides.

In a preferred embodiment of the invention, the probe and/or primer recognizes one of the target sequences as indicated in figure 1.

The term "target sequence" of a probe and/or primer, according to the present invention, is a sequence within the genome of the newly identified genotype G that comprises one or more nucleotides that are unique on their own or as a combination to the newly identified genotype G and to which the probe or primer is complementary or partially complementary (i.e. with up to 20%, more preferably 15%, more preferably 10% or most preferably 5% mismatches). It is to be understood that the complement of said target sequence is also a suitable target sequence in some cases. It should be understood that probes that are designed to specifically hybridize to a target sequence of a nucleic acid, may fall within said target sequence or may to a large extent overlap with said target sequence (i.e. form a duplex with nucleotides outside as well as within said target sequence).

In a very specific embodiment of the invention the target sequence is chosen within the 36 nucleotide insert in the N-terminal part of the Core region of HBV genotype G:

TAGAACAACTTTGCCATATGGCCTTTTTGGCTTAGA (SEQ ID NO 61)

More specifically, the primer and/or probe of the invention recognizes one or more of the following sequences:

TAGAA (SEQ ID NO 62);
AGAAC (SEQ ID NO 63);
GAACA (SEQ ID NO 64);
AACAA (SEQ ID NO 65);
ACAAC (SEQ ID NO 66);
CAACT (SEQ ID NO 67);
AACTT (SEQ ID NO 68);
ACTTT (SEQ ID NO 69);
CTTTG (SEQ ID NO 70);
TTTGC (SEQ ID NO 71);
TTGCC (SEQ ID NO 72);
TGCCA (SEQ ID NO 73);
GCCAT (SEQ ID NO 74);
CCATA (SEQ ID NO 75);
CATAT (SEQ ID NO 76);
ATATG (SEQ ID NO 77);
TATGG (SEQ ID NO 78);
ATGGC (SEQ ID NO 79);
TGGCC (SEQ ID NO 80);
GGCCT (SEQ ID NO 81);
GCCTT (SEQ ID NO 82);
CCTTT (SEQ ID NO 83);
CTTTT (SEQ ID NO 84);
TTTTT (SEQ ID NO 85);
TTTTG (SEQ ID NO 86);
TTTGG (SEQ ID NO 87);
TTGGC (SEQ ID NO 88);
TGGCT (SEQ ID NO 89);
GGCTT (SEQ ID NO 90);
GCTTA (SEQ ID NO 91);
CTTAG (SEQ ID NO 92);

TTAGA (SEQ ID NO 93);
TAGAAC (SEQ ID NO 94);
AGAACA (SEQ ID NO 95);
GAACAA (SEQ ID NO 96);
AACAAC (SEQ ID NO 97);
ACAACT (SEQ ID NO 98);
CAACTT (SEQ ID NO 99);
AACTTT (SEQ ID NO 100);
ACTTTG (SEQ ID NO 101);
CTTTGC (SEQ ID NO 102);
TTTGCC (SEQ ID NO 103);
TTGCCA (SEQ ID NO 104);
TGCCAT (SEQ ID NO 105);
GCCATA (SEQ ID NO 106);
CCATAT (SEQ ID NO 107);
CATATG (SEQ ID NO 108);
ATATGG (SEQ ID NO 109);
TATGGC (SEQ ID NO 110);
ATGGCC (SEQ ID NO 111);
TGGCCT (SEQ ID NO 112);
GGCCTT (SEQ ID NO 113);
GCCTTT (SEQ ID NO 114);
CCTTTT (SEQ ID NO 115);
CTTTTT (SEQ ID NO 116);
TTTTTG (SEQ ID NO 117);
TTTTGG (SEQ ID NO 118);
TTTGGC (SEQ ID NO 119);
TTGGCT (SEQ ID NO 120);
TGGCTT (SEQ ID NO 121);
GGCTTA (SEQ ID NO 122);
GCTTAG (SEQ ID NO 123);
CTTAGA (SEQ ID NO 124);
TAGAACA (SEQ ID NO 125);
AGAACAA (SEQ ID NO 126);

GAACAAC (SEQ ID NO 127);
AACAACT (SEQ ID NO 128);
ACAACTT (SEQ ID NO 129);
CAACTTT (SEQ ID NO 130);
AACTTTG (SEQ ID NO 131);
ACTTTGC (SEQ ID NO 132);
CTTTGCC (SEQ ID NO 133);
TTTGCCA (SEQ ID NO 134);
TTGCCAT (SEQ ID NO 135);
TGCCATA (SEQ ID NO 136);
GCCATAT (SEQ ID NO 137);
CCATATG (SEQ ID NO 138);
CATATGG (SEQ ID NO 139);
ATATGGC (SEQ ID NO 140);
TATGGCC (SEQ ID NO 141);
ATGGCCT (SEQ ID NO 142);
TGGCCTT (SEQ ID NO 143);
GGCCTTT (SEQ ID NO 144);
GCCTTTT (SEQ ID NO 145);
CCTTTTT (SEQ ID NO 146);
CTTTTTG (SEQ ID NO 147);
TTTTTGG (SEQ ID NO 148);
TTTTGGC (SEQ ID NO 149);
TTTGGCT (SEQ ID NO 150);
TTGGCTT (SEQ ID NO 151);
TGGCTTA (SEQ ID NO 152);
GGCTTAG (SEQ ID NO 153);
GCTTAGA (SEQ ID NO 154);

The invention also relates to the use of a HBV genotype specific primer and/or a probe of the invention in a method to detect, in a biological sample, the presence of the new HBV genotype G.

The invention also relates to the use of a universal primer and/or a probe of the invention in a universal method to detect, in a biological sample, the presence of HBV.

The present invention further relates to a method for the detection in a biological sample of the presence of a polynucleic acid characteristic to HBV genotype G. More specifically, the present invention relates to a method as described above, further characterized that it comprises the following step:
(i) possibly extracting the nucleic acid present in the biological sample;
(ii) amplifying the nucleic acid characteristic to HBV genotype G;
(iii) detecting the amplified nucleic acid characteristic to HBV genotype G.

Primers used for amplification may be generic primers or genotype G specific primers. Generic primers allow the amplification of DNA from strains that belong to genotype G as well as DNA from other strains belonging to other HBV genotypes. Genotype G specific primers, in contrast, will allow only the amplification of the nucleic acids from strains that belong to genotype G. These genotype G specific primers also fall within the scope of the present invention.

The present invention further relates to a method for the universal detection in a biological sample of the presence of HBV.

More specifically, the present invention relates to a universal method as described above, further characterized that it comprises the following step:
(j) possibly extracting the nucleic acid present in the biological sample;
(iv) amplifying the nucleic acid;
(v) detecting the amplified nucleic acid.

Primers used for amplification in said universal method are universal primers that allow the amplification of DNA from all genotypes A, B, C, D, E, F and G.

The term "biological sample" as used in the present invention refers to any biological material (tissue or fluid) taken either directly form the infected human being, or after culturing (enrichment) and containing HBV nucleic acid sequences. Biological material may be e.g. expectoration's of any kind, broncheolavages, blood, skin tissue, biopsies, sperm, lymphocyte blood culture material, colonies, liquid cultures, fecal samples, urine, hepatocytes, etc. More particularly "biological sample" refers to blood serum or plasma samples.

The nucleic acids are released, concentrated and/or isolated from the biological sample by any method known in the art. Currently, various commercial kits are available such as the QIAamp Blood Kit from Qiagen (Hilden, Germany) for the isolation of nucleic acids from blood samples and the 'High pure PCR Template Preparation Kit' (Roche Diagnostics, Brussels, Belgium). Other well-known procedures for isolation of DNA or RNA from a biological sample are also available (Sambrook et al., 1989).

The nucleic acids of the invention can be amplified by polymerase chain reaction (PCR; Saiki et al., 1988), ligase chain reaction (LCR; Landgren et al., 1988; Wu and Wallace, 1989; Barany, 1991), nucleic acid sequence-based amplification (NASBA; Guatelli et al., 1990; Compton, 1991), transcription-based amplification system (TAS; Kwoh et al., 1989), strand displacement amplification (SDA; Duck, 1990) or amplification by means of Qβ replicase (Lomeli et al., 1989) or by any other suitable method known in the art, that allows the amplification of nucleic acid molecules. Also TMA (Guatelli et al., 1990) or bDNA (Sanchez-Pescador et al., 1988; Urdea et al., 1991) techniques can be used in the method of the present invention.

The products of this amplification step can subsequently be used for detection of the presence, and/ or for typing of the nucleic acids present in the sample. Analysis of the presence and typing of these nucleic acids can be done by any method known in the art, such as duplex analysis of the PCR products (Clay et al., 1994), single-stranded conformational polymorphism analysis of the PCR product (PCR-SSCP; Yoshida *et al.,* 1992), sequence-based typing (SBT; Santamaria et al., 1992 and 1993), the use of sequence specific primers in PCR reaction (PCR-SSP; Olerup and Zetterquist, 1991), the use of PCR in combination with sequence-specific oligonucleotide probing (PCR-SSOP; Saiki et al., 1986), TMA or bDNA techniques. Assay methods that rely on the formation of a hybrid between the nucleic acids in the biological sample and the oligonucleotide probe include Southern blot, Northern blot or dot blot format (Saiki et al., 1989), the unlabelled amplified sample being bound to a membrane, the membrane being incorporated with at least one labeled probe under suitable hybridization and wash conditions, and the presence of bound probe being monitored. An alternative is a "reverse" format, in which the amplified sequence contains a label. In this format, the selected probes are immobilized to certain locations on a solid support and the amplified polynucleic acids are labeled in order to enable the detection of the hybrids formed. The term "solid support" can refer to any substrate to which an oligonucleotide probe can be coupled, provided that it retains its hybridization characteristics and provided that the background level of hybridization remains low. Usually the solid substrate will be a microtiter plate (e.g. in the DEIA technique), a membrane (e.g. nylon or nitrocellulose) or a microsphere (bead) or a chip. Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH₂ groups, SH groups, carboxylic groups, or coupling with biotin, haptens or proteins.

A preferred embodiment of the present invention thus relates to a method as described above, further characterized that it comprises the following step:
(i) possibly extracting the nucleic acid present in the biological sample;
(ii) amplifying the nucleic acid;
(iii) hybridizing the nucleic acid, possibly under denatured conditions, at appropriate conditions with one or more probes, with said probes being possibly attached to a solid substrate;
(iv) possibly washing at appropriate conditions;
(v) detecting the hybrids formed.

In order to obtain fast and easy results, if a multitude of probes is involved, a reverse hybridization format may be convenient. In this preferred embodiment the selected probes are immobilized to certain locations on a solid support and the amplified polynucleic acids are labeled in order to enable the detection of the hybrids formed. The present invention further relates to a diagnostic kit for use in a method as described above, comprising the following components:
(i) when appropriate, a means for releasing, isolating or concentrating the nucleic acids present in the sample;
(ii) a primer set or a primer mix, possibly according to the invention;
(iii) a means for detection or typing of the nucleic acid, present in the sample.

The present invention specifically relates to a diagnostic kit as described above, said kit comprising at least a primer and/ or at least a probe of the invention. A specific and very user-friendly diagnostic kit is the line probe assay (Stuyver et al., 1996), comprising the following components:
(i) when appropriate, a means for releasing, isolating or concentrating the nucleic acids present in said sample;
(ii) a primer pair or a primer mix, possibly according to the invention;
(iii) at least one probe that specifically hybridizes with a nucleic acid characteristic to HBV genotype G, or a universal probe, fixed to a solid support;
(iv) a hybridization buffer, or components necessary for producing said buffer;
(v) a wash solution, or components necessary for producing said solution;
(vi) when appropriate, a means for detecting the hybrids resulting from the preceding hybridization.

In this embodiment, the selected set of probes is immobilized to a membrane strip in a line fashion. Said probes may be immobilized individually or as mixtures to the delineated locations. The amplified HBV polynucleic acids can be labeled with biotin, and the hybrid can then, via a biotin-streptavidine coupling, be detected with a nonradioactive color developing system.

The term "hybridization buffer" means a buffer allowing a hybridization reaction between the probes and the polynucleic acids present in the sample, or the amplified products, under the appropriate stringency conditions.

The term "wash solution" means a solution enabling washing of the hybrids formed under the appropriate stringency conditions.

The invention also relates to a peptide or a polypeptide having an amino acid sequence encoded by a polynucleic acid of the invention. The terms "peptide" and "polypeptide" are used interchangeable throughout the present application and refer to a polymer of amino acids (aa) and do not refer to a specific length of the product. Thus, oligopeptides, polypeptides, peptides and proteins are included within this definition. More particularly, the polypeptide of the present invention is a HBV genotype G specific polypeptide such as the preS1, preS2, surface antigen HBsAg, preCore precursor protein, HBeAg, HBcAg, the polymerase or the X protein of the HBV strains belonging to the newly identified genotype G, or any part thereof, provided that said polypeptide differs in at least one amino acid from known polypeptides from HBV strains belonging to genotypes A, B, C, D, E or F. In a very specific embodiment, the present invention relates to the HBcAg or HBeAg of the HBV genotype G or any part thereof The insert in the core region, encoding twelve additional amino acids RTTLPYGLFGLD (SEQ ID NO 155) may be responsible for a novel mechanism of expression of HBcAg and HBeAg, and core and e antigens with a unique structure and immunological properties may exist. Therefore, the present invention specifically relates to a peptide derived from the HBcAg or HBeAg of HBV genotype G comprising one of the following sequences:

RTTLPY (SEQ ID NO 156);
TTLPYG (SEQ ID NO 157);
TLPYGL (SEQ ID NO 158);
LPYGLF (SEQ ID NO 159);
PYGLFG (SEQ ID NO 160);
YGLFGL (SEQ ID NO 161);
GLFGLD (SEQ ID NO 162);

RTTLPYG (SEQ ID NO 163);
TTLPYGL (SEQ ID NO 164);
TLPYGLF (SEQ ID NO 165);
LPYGLFG (SEQ ID NO 166);
PYGLFGL (SEQ ID NO 167);
YGLFGLD (SEQ ID NO 168);

The present invention further relates to a universal peptide, characterized that said peptide is comprised in peptide sequences of HBV genotypes A, B, C, D, E, F and G. Since the present invention provides amino acid sequence alignments of 7 genotypes, the invention allows for the first time to delineate universal HBV amino acid sequences, characteristic for HBV genotypes A, B, C, D, E, F and G.

Also included within the present invention are post-translational modifications such as glycosylation, acetylation, phosphorylation, modifications with fatty acids and the like. Also included within the definition are, for example, polypeptides containing one or more analogues of an aa (including unnatural amino acids), polypeptides with substituted linkages, mutated versions or natural sequence variations of the polypeptides, polypeptides containing disulfide bounds between cysteine residues, biotinylated polypeptides as well as other modifications known in the art.

The polypeptides of the invention can be produced by any method known in the art such as classical chemical synthesis as described by Houbenweyl (1974) and Atherton and Shepard (1989) or by means of recombinant DNA techniques as described by Sambrook et al. (1989).

In accordance, the present invention also relates to a polypeptide as described above which is recombinantly expressed. The term "recombinantly expressed" refers to the fact that the peptide is produced by recombinant expression methods be it in prokaryotes, or lower or higher eukaryotes as discussed below. The present invention thus also relates to a method for the production of a recombinant polypeptide of the invention comprising the following steps:
(i) transformation of an appropriate cellular host with an expression vector;
(ii) culturing said transformed cellular host under conditions enabling the expression of the polynucleic acid inserted in the expression vector; and
(iii) harvesting said polypeptide.

Therefore, also comprised within the scope of the present invention is an expression vector, comprising a polynucleic acid or part thereof of the invention, operably linked to prokaryotic, eukaryotic or viral transcription and translation control elements. The term "expression vector" refers to a vector sequence of the type plasmid, cosmid, phage or viral DNA wherein a nucleic acid of the invention is inserted, and which contains the necessary elements to promote the transcription and translation of the latter polypeptide(s) by a host cell.

The present invention also relates to a host cell transformed with an expression vector as defined above. The term "host cell" denotes micro-organisms or higher eukaryotic cell lines cultured as unicellular entities and refers to cells which can be or have been used as recipients for a recombinant vector. The prokaryotes used as host cell can be chosen from bacteria such as *Escherichia coli, Lactobacillus, Lactococcus, Salmonella, Streptococcus, Bacillus* or *Streptomyces.* Preferred lower eukaryotic host cells are yeasts, particularly species within *Saccharomyces, Schizosaccharomyces, Kluveromyces, Pichia (*e.g. *Pichia pastoris), Hansenula (*e.g. *Hansenula polymorpha), Yarowia, Schwanniomyces, Zygosaccharomyces* and the like. *Saccharomyces* *cerevisiae, S. carlsbergensis* and *K. lactis* are the most commonly used yeast hosts. Fungal host cells include *N. crassa.* Host cells from higher eukaryotes include those from higher animals such as mammals, reptiles, insects, and the like. Presently, preferred higher eukaryotic host cells are derived from Chinese hamster (e.g. CHO), monkey (e.g. COS and Vero cells), baby hamster kidney (BHK), pig kidney (PK15), rabbit kidney 13 cells (RK13), the human osteosarcoma cell line 143 B, the human cell line HeLa and human hepatoma cell lines like Hep G2, and insect cell lines (e.g. *Spodoptera frugiperda*). The host cells may be provided in suspension of flask cultures, tissue cultures, organ cultures and the like.

Any of the known purification methods for recombinant peptides can be used for the production of the recombinant peptides of the present invention.

The present invention also relates to the use of a HBV genotype G specific polypeptide of the invention in a method for detecting antibodies specific to the theretofore-unidentified HBV genotype G, preferably specific to the HBcAg and/or HBeAg of the theretofore-unidentified HBV genotype G present in a biological sample.

The present invention also relates to a method for detecting antibodies specific to the theretofore-unidentified HBV genotype G, preferably specific to the HBcAg and/or HBeAg of the theretofore-unidentified HBV genotype G present in a biological sample, comprising the following steps:
(i) contacting the biological sample with a HBV genotype G specific polypeptide according to the invention;
(ii) detecting the immunological complex formed between said antibodies and said polypeptide.

The present invention also relates to a universal method for detecting antibodies specific to genotypes A, B, C, D, E, F and G present in a biological sample, comprising the following steps:
(iii) contacting the biological sample with a universal polypeptide according to the invention;
(iv) detecting the immunological complex formed between said antibodies and said polypeptide.

Design of the immunoassay is subject to a great deal of variation, and many formats are known in the art. Protocols may, for example, use solid supports, or immunoprecipitation. Most assays involve the use of labeled polypeptide. The label may be, for example, enzymatic, fluorescent, chemiluminescent, radioactive, or dye molecules. Assays that amplify the signals from the immune complex include the use of biotin and avidin or streptavidin, and enzyme-labeled and mediated immunoassays, such as ELISA assays.

The immunoassay may be, without limitation, in a heterogeneous or in a homogeneous format, and of a standard or competitive type. In a heterogeneous format, the polypeptide is typically bound to a solid matrix or support to facilitate separation of the sample from the polypeptide after incubation. Examples of solid supports that can be used are nitrocellulose (e.g., in membrane or microtiter well form), polyvinyl chloride (e.g., in sheets or microtiter wells), polystyrene latex (e.g., in beads or microtiter plates, polyvinylidene fluoride (known as Immunolon™), diazotized paper, nylon membranes, activated beads, and Protein A beads. For example, Dynatech Immunolon™ 1 or Immunolon™ 2 microtiter plates or 0.25-inch polystyrene beads (Precision Plastic Ball) can be used in the heterogeneous format.

The solid support containing the antigenic peptides is typically washed after separating it from the biological sample, and prior to detection of the bound antibodies. Both standard and competitive formats are known in the art.

In a homogeneous format, the test sample is incubated with the combination of antigens in solution. For example, it may be under conditions that will precipitate any antigen-antibody that is formed. Both standard and competitive formats for these assays are known in the art.

The present invention also relates to a diagnostic kit for use in a method as described above, said kit comprising at least one polypeptide according to the invention, with said polypeptide possibly bound to a solid support. In a preferred embodiment said kit may comprise a range of said polypeptides, wherein said polypeptides are attached to specific locations on a solid substrate. More preferably, said solid support is a membrane strip and said polypeptides are coupled to the membrane in the form of parallel lines.

The present invention further relates to the use of a polypeptide as described above for the preparation of a vaccine for the prevention or treatment of HBV infections.

The present invention also relates to a vaccine composition comprising as an active substance a polypeptide of the invention that can be used as an inoculum to vaccinate humans (or non human mammals) against infection with HBV or to therapeutically vaccinate human (or non human mammal) carriers of HBV.

The term "a vaccine composition" relates to an immunogenic composition capable of eliciting protection against HBV, whether partial or complete. The term "as an active substance" relates to the component of the vaccine composition that elicits protection against HBV. An active substance (i.e. the polypeptides of the present invention) can be used as such, in a biotinylated form (as explained in WO 93/18054) and/or complexed to *Neutralite Avidin* according to the manufacturer's instruction sheet (Molecular Probes Inc., Eugene, OR). It should also be noted that "a vaccine composition" comprises, in addition to an active substance, a suitable excipient, diluent, carrier and/or adjuvant that, by them, do not induce the production of antibodies harmful to the individual receiving the composition nor do they elicit protection. Suitable carriers are typically large slowly metabolized macromolecules such as proteins, polysaccharides, polylactic acids, polyglycolic acids, polymeric amino acids, amino acid copolymers and inactive virus particles. Such carriers are well known to those skilled in the art. Preferred adjuvants to enhance effectiveness of the composition include, but are not limited to: aluminium hydroxide, aluminium in combination with 3-0-deacylated monophosphoryl lipid A as described in WO 93/19780, aluminium phosphate as described in WO 93/24148, N-acetyl-muramyl-L-threonyl-D-isoglutamine as described in U.S. Patent N° 4,606,918, N-acetyl-normuramyl-L-alanyl-D-isoglutamine, N-acetylmuramyl-L-alanyl-D-isoglutamyl-L-alanine2(1'2'dipalmitoyl-sn-glycero-3-hydroxyphosphoryloxy) ethylamine and RIBI (ImmunoChem Research Inc., Hamilton, MT) which contains monophosphoryl lipid A, detoxified endotoxin, trehalose-6,6-dimycolate, and cell wall skeleton (MPL + TDM + CWS) in a 2% squalene/Tween 80 emulsion. Any of the three components MPL, TDM or CWS may also be used alone or combined 2 by 2. Additionally, adjuvants such as Stimulon (Cambridge Bioscience, Worcester, MA) or SAF-1 (Syntex), RC259 (Coryxa), SBAS2, SBAS3, SBAS4, SBAS5, SBAS6, SBAS7, SBAS8 (Smith Kline Beecham, Rixensart, Belgium) may be used. Furthermore, Complete Freund's Adjuvant (CFA) and Incomplete Freund's Adjuvant (IFA) may be used for non-human applications and research purposes. A "vaccine composition" will further contain excipients and diluents, which are inherently non-toxic and non-therapeutic, such as water, saline, glycerol, ethanol, wetting or emulsifying agents, pH buffering substances, preservatives, and the like. Typically, a vaccine composition is prepared as an injectable, either as a liquid solution or suspension. Solid forms, suitable for solution on, or suspension in, liquid vehicles prior to injection may also be prepared. The preparation may also be emulsified or encapsulated in liposomes for enhancing adjuvant effect. The polypeptides may also be incorporated into Immune Stimulating Complexes together with saponins, for example Quil A (ISCOMS). Vaccine compositions comprise an immunologically effective amount of the polypeptides of the present invention, as well as any other of the above-mentioned components. "Immunologically effective amount" means that the administration of that amount to an individual, either in a single dosis or as part of a series, is effective for prevention or treatment. This amount varies depending upon the health and physical condition of the individual to be treated, the taxonomic group of the individual to be treated (e.g. nonhuman primate, primate, etc.), the capacity of the individual's immune system to mount an effective immune response, the degree of protection desired, the formulation of the vaccine, the treating's doctor assessment, the strain of the infecting HBV, and other relevant factors. It is expected that the amount will fall in a relatively broad range that can be determined through routine trials. Usually, the amount will vary from 0.01 to 1000 µg/dose, more particularly from 0.1 to 100 µg/dose. The vaccine compositions are conventionally administered parenterally, typically by injection, for example, subcutaneously or intramuscularly. Additional formulations suitable for other methods of administration include oral formulations and suppositories. Dosage treatment may be a single dose schedule or a multiple dose schedule. The vaccine may be administered in conjunction with other immunoregulatory agents. It should be noted that a vaccine might also be useful for treatment of an individual, in which case it is called a "therapeutic vaccine".

The present invention also relates to a HBV genotype G specific antibody specifically raised upon immunization with at least one HBV genotype G specific polypeptide of the invention, with said antibody being specifically reactive with said polypeptide and not reactive with a peptide characteristic for HBV genotype A, B, C, D, E and/or F. In a preferred embodiment, the antibody specifically recognizes the HBV polymerase, X protein, preCore, HBcAg, HBeAg, preS1, preS2 or HBsAg of HBV genotype G.

The present invention also relates to a universal antibody specifically raised upon immunization with a universal polypeptide of the invention, with said antibody being reactive with said universal polypeptide. In a preferred embodiment, the antibody specifically recognizes the HBV polymerase, preCore, HBcAg, HBeAg, preS1, preS2 or HBsAg protein of genotypes A, B, C, D, E, F and G.

The antibody may be polyclonal or monoclonal. Also fragments derived from these monoclonal antibodies such as Fab, F(ab)'₂, scFv ("single chain variable fragment") and other antibody like constructs that retain the variable region of the antibody, providing they have retained the original binding properties, fall within the scope of the present invention and can be used in a method of the present invention. Such fragments are commonly generated by, for instance, enzymatic digestion of the antibodies with papain, pepsin, or other proteases. It is well known to the person skilled in the art that monoclonal antibodies, or fragments thereof, can be modified for various uses. Also mini-antibodies and multivalent antibodies such as diabodies, triabodies, tetravalent antibodies and peptabodies can be used in a method of the invention. The preparation and use of these fragments and multivalent antibodies has been described extensively in International Patent Application WO 98/29442. The monoclonal antibodies used in a method of the invention may be humanized versions of the mouse monoclonal antibodies made by means of recombinant DNA technology, departing from the mouse and/or human genomic DNA sequences coding for H and L chains or from cDNA clones coding for H and L chains. Alternatively the monoclonal antibodies used in a method of the invention may be human monoclonal antibodies. The term "humanized antibody" means that at least a portion of the framework regions of an immunoglobulin is derived from human immunoglobulin sequences. The antibodies used in a method of the present invention may be labeled by an appropriate label of the enzymatic, fluorescent, or radioactive type.

The present invention further relates to the use of a HBV genotype G specific antibody of the invention in a method for detecting a HBV genotype G specific polypeptide of the invention, preferably for detecting the HBcAg and/or HBeAg of the theretofore-unidentified HBV genotype G, present in a biological sample. The present invention also relates to a method for detecting a HBV genotype G specific polypeptide of the invention, preferably for detecting the HBcAg and/or HBeAg of the theretofore-unidentified HBV genotype G present in a biological sample, comprising the following steps:
(i) bringing said biological sample into contact with a HBV genotype G specific antibody of the invention recognizing said HBV genotype G specific polypeptide, under conditions being suitable for producing an antigen-antibody complex; and
(ii) detecting the immunological binding of said antibody to said polypeptide.

The present invention further relates to the use of a universal antibody of the invention in a method for detecting a universal polypeptide of the invention, present in a biological sample.

The present invention also relates to a method for detecting a universal polypeptide of the invention, present in a biological sample, comprising the following steps:
(iii) bringing said biological sample into contact with a universal antibody of the invention recognizing said universal polypeptide, under conditions being suitable for producing an antigen-antibody complex; and
(iv) detecting the immunological binding of said antibody to said polypeptide.

The process for the detection of the immunological binding can then be carried out by bringing together said polypeptide-antibody complex formed by the polypeptide of the invention and the antibody recognizing said polypeptide with:
(i) a secondary antibody (or detector antibody) recognizing a specific epitope of the polypeptide-antibody complex but not recognizing the primary antibody alone;
(ii) a marker either for specific tagging or coupling with said secondary antibody, with said marker being any possible marker known to the person skilled in the art;
(iii) appropriate buffer solutions for carrying out the immunological reaction between the secondary antibody and the polypeptide-primary antibody complex and/or the bound secondary antibody and the marker on the other hand.

Advantageously, the antibodies used in the invention are in an immobilized state on a suitable support. The secondary antibody itself may carry a marker or a group for direct or indirect coupling with a marker. Alternatively, the present process may be put into practice by using any other immunoassay format known to the person skilled in the art.

The term "epitope" refers to that portion of the polypeptide-antibody complex that is specifically bound by an antibody-combining site. Epitopes may be determined by any of the techniques known in the art or may be predicted by a variety of computer prediction models known in the art.

The expression "recognizing", "reacting with", "immunological binding" or "producing a polypeptide-antibody complex" as used in the present invention is to be interpreted that binding between the polypeptide and the antibody occurs under all conditions that respect the immunological properties of the antibody and the polypeptide of the invention.

The present invention also relates to a diagnostic kit for use in the detection of a theretofore-unidentified HBV genotype G present in a biological sample, said kit comprising at least one HBV genotype G specific antibody according to the invention. In a specific embodiment the present invention relates to a diagnostic kit for use in the detection of a theretofore-unidentified HBV genotype G present in a biological sample, said kit comprising:
(i) at least a primary antibody recognizing a HBV genotype G specific polypeptide of the invention (or capturing antibody);
(ii) possibly, a secondary antibody (or detector antibody) forming an immunological complex with an epitope of the polypeptide-primary antibody complex but not with the primary antibody alone;
(iii) possibly, a marker either for specific tagging or coupling with said secondary antibody;
(iv) possibly, appropriate buffer solutions for carrying out the immunological reaction between the primary antibody and the polypeptide of the invention, between the secondary antibody and the polypeptide-primary antibody complex and/or between the bound secondary antibody and the marker;
(v) possibly, for standardization purposes, a purified or synthetic peptide that is specifically recognized by the antibodies of the kit.

The present invention also relates to a diagnostic kit for use in the universal detection of HBV present in a biological sample, said kit comprising at least one universal antibody according to the invention.

In a specific embodiment the present invention relates to a diagnostic kit for use in the universal detection of HBV present in a biological sample, said kit comprising:
(i) at least a primary antibody recognizing a universal polypeptide of the invention (or capturing antibody);
(ii) possibly, a secondary antibody (or detector antibody) forming an immunological complex with an epitope of the polypeptide-primary antibody complex but not with the primary antibody alone;
(iii) possibly, a marker either for specific tagging or coupling with said secondary antibody;
(iv) possibly, appropriate buffer solutions for carrying out the immunological reaction between the primary antibody and the polypeptide of the invention, between the secondary antibody and the polypeptide-primary antibody complex and/or between the bound secondary antibody and the marker;
(v) possibly, for standardization purposes, a purified or synthetic peptide that is specifically recognized by the antibodies of the kit.

The present invention further relates to the use of an antibody of the invention as a medicament to prevent humans of HBV infection or to treat humans with HBV infection.

The present invention also relates to a pharmaceutical composition or medicament comprising as an active substance an antibody of the invention that can be used for the treatment of HBV infection.

The term "medicament" or "pharmaceutical composition" (both terms can be used interchangeable) refers to a composition comprising an antibody according to the present invention, possibly in the presence of suitable excipients known to the skilled man such as saline, Ringer's solution, dextrose solution, Hank's solution, fixed oils, ethyl oleate, 5% dextrose in saline, substances that enhance isotonicity and chemical stability, buffers and preservatives. The "medicament" may be administered by any suitable method within the knowledge of the skilled man. The preferred route of administration is parenterally. In parental administration, the medicament of this invention will be formulated in a unit dosage injectable form such as a solution, suspension or emulsion, in association with the pharmaceutically acceptable excipients as defined above. However, the dosage and mode of administration will depend on the individual. Generally, the medicament is administered so that the antibody is given at a dose between 1 µg/kg and 10 mg/kg, more preferably between 10 µg/kg and 5 mg/kg, most preferably between 0.1 and 2 mg/kg. Preferably, it is given as a bolus dose. Continuous infusion may also be used. If so, the medicament may be infused at a dose between 5 and 20 µg/kg/minute, more preferably between 7 and 15 µg/kg/minute. Dosage forms suitable for oral administration include but are not limited to tablets, pills, capsules, caplets, granules, powders, elixirs, syrups, solutions and aqueous or oil suspensions. A suitable daily dose of the active compound for administration to human beings is generally from about 1 mg to about 5000 mg, more usually from about 5 mg to about 1000 mg, given in a single dose or in divided doses at one or more times during the day.

It should also be clear that the pharmaceutical composition of the present invention might comprise a functionally equivalent variant of the antibody of the invention. The latter terms refer to a molecule which contains the antibody of the invention, to which certain modifications have been applied, and which retains all or part of its biological properties. Such modifications include but are not limited to the addition of polysaccharide chains, the addition of certain chemical groups, the addition of lipid moieties, the fusion with other peptide or protein sequences and the formation of intramolecular cross-links.

The present invention also provides methods for identifying compounds or agents (herein referred to as "test compound") that can be used to treat disorders characterized by (or associated with) HBV infection. These methods are also referred to herein as "drug screening assays" or "bioassays."

In one embodiment, the drug screening assay may include the step of screening a test compound for the ability to interact with (e.g., bind to) an HBV genotype G protein, or any functionally equivalent part thereof, to modulate the interaction of an HBV protein and a target molecule. Typically, the assay is a cell-free assay including the following steps:
(i) combining the HBV genotype G protein of the invention or an immunogenic fragments thereof, and a test compound under conditions which allow for interaction of (e.g., binding of) the test compound to the HBV genotype G protein or a portion thereof to form a complex;
(ii) detecting the formation of a complex.

The ability of the test compound to interact with (e.g. bind to) the HBV genotype G protein or portion thereof is indicated by the presence of the test compound in the complex. Formation of complexes between the HBV genotype G protein and the test compound can be quantitated, for example, using a standard immunoassay. The HBV genotype G protein or its immunogenic fragment employed in such a test, may be free in solution, affixed to a solid support, borne on a cell surface, or located intracellularly.

In another embodiment, the invention provides a screening assay to identify test compounds which modulate (e.g., stimulate or inhibit) the interaction (and most likely HBV genotype G protein activity as well) between an HBV genotype G protein and:
- a molecule (target molecule) to which the HBV genotype G protein normally interacts; or
- antibodies which specifically recognize the HBV genotype G protein.

Target molecules include proteins in the same signaling path as the HBV genotype G protein, e.g., proteins which may function upstream (including both stimulators and inhibitors of activity) or downstream of the HBV protein signaling pathway [Zn-fingers, protease activity, regulators of cysteine redox status]. Typically, the assay is a cell-free assays, which includes the following steps:
(i) combining an HBV genotype G protein or a immunogenic fragment thereof, an HBV protein target molecule (e.g., an HBV protein ligand) or a specific antibody and a test compound under conditions which allow for interaction of (e.g., binding of) the HBV genotype G protein or a portion thereof with the target molecule or the antibody;
(ii) detecting the formation of a complex which includes the HBV genotype G protein and the target molecule or the antibody, or detecting the interaction/reaction of the HBV genotype G protein and the target molecule or antibody.

To perform the above-described drug-screening assay, it is feasible to immobilize either the HBV genotype G protein or its target molecule to facilitate separation and to accommodate automation of the assay. Detection of complex formation can include direct quantitation of the complex by, for example, measuring inductive effects of the HBV genotype G protein. A statistically significant change, such as a decrease, in the interaction of the HBV genotype G protein and the target molecule (e.g., in the formation of a complex between the HBV genotype G protein and the target molecule) in the presence of a test compound (relative to what is detected in the absence of the test compound) is indicative of a modulation (e.g., stimulation or inhibition) of the interaction between the HBV genotype G protein and the target molecule. Modulation of the formation of complexes between the HBV genotype G protein and the target molecule can be quantitated using, for example, an immunoassay.

It should be clear that a modulator of the interaction between a HBV genotype G protein and a target molecule, when identified by any of the herein-described methods, is contemplated in the invention.

Another technique for drug screening which provides for high throughput screening of compounds having suitable binding affinity to the HBV genotype G protein is described in detail in "Determination of Amino Acid Sequence Antigenicity" by Geysen HN, WO Application 84/03564, published on 13/09/84, and incorporated herein by reference. In summary, large numbers of different small peptide test compounds are synthesized on a solid substrate, such as plastic pins or some other surface. The protein test compounds are reacted with fragments of HBV genotype G protein and washed. Bound HBV genotype G protein is then detected by methods well known in the art.

This invention also contemplates the use of a competitive drug screening assays in which neutralizing antibodies capable of binding a HBV genotype G protein specifically compete with a test compound for binding the HBV genotype G protein. In this manner, the antibodies can be used to detect the presence of any protein that shares one or more antigenic determinants with the HBV genotype G protein.

In yet another embodiment, the invention provides a method for identifying a compound capable of modulating HBV nucleic acid expression and/or HBV protein activity. Methods for assaying the ability of a test compound to modulate the expression of a HBV nucleic acid or activity of a HBV genotype G protein are typically cell-based assays. However, HBV genotype G infected animals are also contemplated herein. HBV genotype G infected or transfected cells which transduce signals via a pathway involving a HBV genotype G protein can be induced to overexpress an HBV protein in the presence and absence of a test compound. These HBV genotype G infected cells also form part of the present invention.

Throughout this specification and the claims which follow, unless the context requires otherwise, the word "comprise", and variations such as "comprises" and "comprising", will be understood to imply the inclusion of a stated integer or step or group of stated integers or steps but not to the exclusion of any other integer or step or group of integers or steps.

### EXAMPLES

### Example 1: Prevalence of the different HBV genotypes

### 1.1 Sample collection

A total of 121 HBV-positive plasma samples where collected in France (n=39) and the United States (n=82). The samples were divided into aliquots and stored at -20°C until use. Samples were taken from chronic HBV carriers and were randomly selected as they became available.

### 1.2 HBV DNA extraction and amplification

HBV DNA was extracted from 100 µl serum sample using the High Pure PCR Template Preparation kit (Roche Diagnostics, Brussels, Belgium) essentially as previously described (Stuyver et al., 1999). A PCR fragment was generated covering the preS1, preS2 and HBsAg region by use of primers HBPr1 and HBPr135 (outer PCR) followed by a nested reaction using HBPr2 and HBPr94 (Fig. 4; Table 1). Outer PCR amplified the viral DNA over 40 cycles, with denaturation at 94°C for 30 s, annealing at 50°C for 30 s, and elongation at 72 °C for 30 s. Samples negative in first-round PCR were further amplified with nested PCR primers for 35 cycles with the same thermal profile.

### 1.3 HBV genotyping

The 121 serum samples were further genotyped by using a research version of the HBV genotyping Line Probe Assay (LiPA) (Van Geyt et al., 1998). The results are summarized in Table 2. A very typical but previously unrecognized reactivity pattern was obtained for 2 samples from Europe and 11 samples from the United States: hybridization reactions were observed on probe 140 (specifically designed for genotype A), probe 148 (designed for genotypes A and B), probe 80 (designed for genotypes C, D and E) and probe 239 (designed for genotypes B and E) (Van Geyt et al. 1998). Because this mixed hybridization pattern did not allow unique type recognition, further characterization was done via sequence analysis.

### Example 2: Characterization of the samples that showed a mixed hybridization pattern

### 2.1 HBV DNA extraction and amplification

HBV DNA was extracted from 100 µl serum sample using the High Pure PCR Template Preparation kit (Roche Diagnostics, Brussels, Belgium) essentially as previously described (Stuyver et al., 1999). The complete genome of HBV was amplified using the Expand High Fidelity PCR system (Roche Diagnostics, Brussels, Belgium). The amplification was performed on 5 µl of the extracted DNA with the primers HBPr108 and HBPr109 (Table 1). A 45 µl reaction mix was made, containing 5 µl 10 x Expand High Fidelity PCR system buffer, 2.6 U Expand High Fidelity PCR system enzyme mix, 200 µM dNTPs, 300 nM of each primer and sterile H₂O. Amplification was performed with denaturation at 94 °C for 40 s, annealing (after shifting to 60 °C in 50 s) for 1 min and elongation (after shifting to 72 °C in 15 s) for 4 min, with an increment of 5 s/cycle (Gunther et al., 1998). Two shorter PCR fragments were also generated: (i) the first amplicon covered the preS1, preS2, and HBsAg region and was amplified using primers HBPr1 and HBPr135 (outer PCR) followed by a nested reaction using HBPr2 and HBPr94 (Table 1); and (ii) the second amplicon covered the preCore/Core region and was amplified by means of a hemi-nested set of PCR primers (HBPr86 and HBPr303, followed by HBPr87 and HBPr303). Outer PCR amplified the viral DNA over 40 cycles, with denaturation at 94 °C for 30 s, annealing at 50 °C for 30 s and elongation at 72 °C for 30 s. Samples negative in first-round PCR were further amplified with nested PCR primers for 35 cycles with the same thermal profile.

### 2.2 Sequencing

Sequencing was performed on an automated DNA sequencer ABI 377 (PE Applied Biosystems, Foster City, CA, USA), using fluorescence-labeled dideoxynucleotide chain terminators (ABI Prism BigDye Terminator Cycle Sequencing Ready Reaction Kit with Ampli*Taq* DNA polymerase FS; PE Applied Biosystems). The primers used for sequencing the complete genome are summarized in Table 1. The primers used to sequence the shorter PCR fragments are the same as the amplification primers. One European virus isolate (FR1) was selected for whole genome sequencing (Fig. 1; SEQ ID NO 1), whereas the preCore/Core and preS/S genes were sequenced from another seven samples (Figs. 7 and 8; SEQ ID NO 43-56).

### 2.3 Homology percentages with other genotypes

In order to compare the genetic relatedness of the FR1 strain with 36 other complete HBV genomes, homology percentages were calculated. The FR1 sequence showed an average homology of: 87.1% (min. 86.2%, max. 87.7%) with genotype A (five sequences); 86.6% (min. 86.5%, max. 86.6%) with genotype B (four sequences); 86.5% (min. 86.0%, max. 87.0%) with genotype C (14 sequences); 86.9% (min. 86.3%, max. 87.3%) with genotype D (eight sequences); 88.3% (min. 88.2%, max. 88.4%) with genotype E (two sequences); and 84.7% (min. 84.5%, max. 84.8%) with genotype F (three sequences).

### 2.4 Phylogenetic distance with other genotypes

In order to compare the genetic relatedness of the FR1 strain with 36 other complete HBV genomes, also the phylogenetic distances were calculated. Phylogenetic distances between the six recognized HBV genotypes (36 sequences) were compared to each other and to the FR1 strain (Fig. 9). A clear difference emerged between the phylogenetic distances (i) within one genotype (distance range of 0.01 - 0.06) and (ii) between different genotypes (distance range 0.08 - 0.17). Using a t-test for the mean and a distance of 0.08 as border value between genotypes, FR1 was found to be significantly different from genotypes A - F (range 0.11 - 0.17; *P* < 0.019).

Phylogenetic trees were created and analyzed by distance matrix comparison using DNADIST, Neighbor and Drawgram software programs of PHYLIP version 3.5c (Felsenstein, 1993). Statistical analysis (t-test for the mean) was performed using MedCalc Version 4.05-Windows 95 (Medcalc Software, Mariakerke, Belgium). A P value of 0.05 was considered to be statistically significant. Complete genome sequences representing the different genotypes were retrieved from the GenBank; their accession numbers are indicated in figure 10. Phylogenetic trees of the complete genome sequences (Fig. 10a), as well as of the individual ORF [Fig. 10b for the surface region (preS1, preS2, HBsAg); data not shown for the other ORFs] were constructed, illustrating that FR1 is indeed located on a separate branch. Fig. 10b further illustrates that the HBV samples from the USA and France, are closely related to each other. Based on these calculations and illustrated by means of phylogenetic trees, FR1 and the other virus strains shown belong to a new HBV genotype, called genotype G.

### Example 3: Characterization of the genome structure and open reading frames in HBV strains belonging to genotype G

The complete genome structure of FR1 was similar to that described for the known HBV genotypes, but was found to be 3248 bp long (Fig. 4).

The preCore region has translational stops at codon 2 (TAA instead of CAA) and codon 28 (TAG instead of TGG) in all eight isolates sequenced. Based on the presence of this dual stop codon, the presence of HBeAg is generally not expected. Paradoxically, sample FR2 showed the presence of HBeAg in the plasma. The Core region is 585 nucleotides long and encodes a Core protein of 195 amino acids (Fig. 5). In contrast to the other genotypes, the Core region had a nucleotide insert of 36 bp, located after the fifth nucleotide following the Core translation initiation point (A at position 1901). Therefore, the insert in the core region encoding twelve additional amino acids RTTLPYGLFGLD may be responsible for a novel mechanism of expression of HBcAg and HBeAg, and core and e antigens with unique structures and immunological properties may exist. Genotype G, as well as all other genotypes except A, showed a 6 nucleotide deletion in the carboxy-terminal part of the HBcAg ORF (Figs. 4 and 5).

The preS1 region contains 354 bp (118 amino acids), the preS2 region 165 bp (55 amino acids), and the HBsAg region 678 bp (226 amino acids) (Fig. 6). Like genotype E, genotype G strains showed a 3 nucleotide deletion (1 amino acid at position 11; Fig. 6) in the amino-terminal part of preS1. Based on the presence of a lysine (K) at HBsAg position 122, a lysine (K) at position 160 and a proline (P) at position 127, the serological subtype of this genotype G strain was predicted to be adw2.

The polymerase region of this genotype contains 2526 bp (842 amino acids). The deletion in the carboxy-terminal part of HBcAg, as well as the deletion at the amino terminus of preS1 affects the numbering of the polymerase protein. The methionine residue, which is prone to changes during lamivudine therapy, is located at position 549 in the highly conserved YMDD motif (Bartholomeusz et al., 1998). Figure 6 also shows the exact numbering for this methionine residue in the other genotypes.

### Example 4: Method for the detection of HBV genotype G

### 4.1 HBV DNA extraction and amplification

HBV DNA was extracted from serum samples with the High Pure PCR Template Preparation Kit (Roche Diagnostics, Brussels, Belgium). A nested set of PCR primers was designed, allowing the amplification of the HBsAg region and generating a 341 bp fragment (outer sense primer HBPr 134; outer antisense primer HBPr 135; nested sense primer HBPr 75; nested antisense primer HBPr 94; table 1). All primers were tagged with a biotin group at the 5' end. Outer PCR amplified the DNA over 40 cycles (30 s at 95°C; 30 s at 45°C; 30 s at 72°C). Samples negative in first round PCR were further amplified with nested PCR primers for 35 cycles with the same thermal profile.

### 4.2 Design of a LiPA for HBV genotyping

A total of 20 genotype-specific probes are designed. Probes designed for the identification of genotypes A, B, C, D, E and F are described in Van Geyt et al. (1998). By way of example, probes specifically selected for the identification of HBV genotype G nucleic acid sequences recognize the regions indicated as target A or target B in figure 1. After enzymatic addition of a poly d(T) tail, the selected oligonucleotide probes are applied as horizontal lines on nitrocellulose membranes. One line of biotinylated DNA is applied alongside as a positive control.

### 4.3 Testing of the LiPA with a cloned references panel and with representative clinical samples

The assay is essentially carried out as described for the HCV line probe assay (Stuyver et al., 1996). The reactivity of the different probes on the HBV genotyping LiPA is assessed with a cloned reference panel and with clinical samples. Figure 11 shows the result for some representative samples after hybridization of their PCR product to the probes on the LiPA. PCR fragments from HBV strain FR1 hybridize with the generic probes 140, 148, 80 and 239 and with the genotype G specific probes selected to recognize target A or target B as indicated in figure 1.

### REFERENCES

Alvarado-Esquivel C, Wyseur A, Herrera-Ortiz F, Ruiz-Maya L, Ruiz-Astorga R, Zarate-Aguilar A, Carrillo-Maravilla E, Herrera-Luna R, Moralers-Macedo M, Maertens G and Stuyver L (1998) Hepatitis B and C virus infections in Mexico: genotypes and geographical distribution in blood donors and patients with liver disease. In *Therapies for Viral Hepatitis,* pp. 35-41. Eds. RF Schinazi, J-P Sommadossi and HC Thomas. International Medical Press, London, UK.

Asseline U, Delarue M, Lancelot G, Toulme F and Thuong N (1984) Nucleic acid-binding molecules with high affinity and base sequence specificity: intercalating agents covalently linked to oligodeoxynucleotides. Proc Natl Acad Sci USA 81: 3297-3301.

Atherton and Shepard (1989) Solid phase peptide synthesis. IRL Press, Oxford, UK.

Barany F (1991) The ligase chain reaction in a PCR world. PCR Methods Appl 1: 5-16.

Bowyer SM, van Staden L, Kew MC and Sim JGM (1997) A unique segment of the hepatitis B virus group A genotype identified in isolates from South Africa. J Gen Virol 78: 1719-1729.

Carman WF, Van Deursen FJ, Mimms LT, Hardie D, Coppola R, Decker R and Sanders R (1997) The prevalence of surface antigen variants of hepatitis B virus in Papua New Guinea, South Africa, and Sardinia. Hepatology 25: 1658-1666.

Clay TM, Culpan D, Howell WM, Sage DA, Bradley BA and Bidwell JL (1994) UHG crossmatching. A comparison with PCR-SSO typing in the selection of HLA-DPB-compatible bone marrow donors. Transplantation 58: 200-207.

Compton J (1991) Nucleic acid sequence-based amplification. Nature 350: 91-92.

Duck P (1990) Probe amplifier system based on chimeric cycling oligonucleotides. Biotechniques 9: 142-147.

Felsenstein J (1993) PHYLIP (Phylogeny Inference Package) version 3.5c. Distributed by the author. Department of Genetics, University of Washington, Seattle, USA.

Guatelli J, Whitfield K, Kwoh D, Barringer K, Richman D and Gengeras T (1990) Isothermal, in vitro amplification of nucleic acids by a multienzyme reaction modeled after retroviral replication. Proc Natl Acad Sci USA 87: 1874-1878.

Gunther S, Sommer G, Von Breunig F, Iwanska A, Kalinina T, Sterneck M and Will H (1998) Amplification of full-length hepatitis B virus genomes from samples from patients with low levels of viremia: frequency and functional consequences of PCR-introduced mutations. J Clin Microbiol 36: 531-538.

Houbenweyl (1974) Method of organic chemistry. Vol. 15-I et II. Eds. E Wunch, Thieme, Stuttgart, Germany.

Kwoh D, Davis G, Whitfield K, Chappelle H, Dimichele L and Gingeras T (1989) Transcription-based amplification system and detection of amplified human immunodeficiency virus type 1 with a bead-based sandwich hybridization format. Proc Natl Acad Sci USA 86: 1173-1177.

Landgren U, Kaiser R, Sanders J and Hood L (1988) A ligase-mediated gene detection technique. Science 241: 1077-1080.

Lomeli H, Tyagi S, Pritchard C, Lisardi P and Kramer F (1989) Quantitative assays based on the use of replicatable hybridization probes. Clin Chem 35: 1826-1831.

Magnius LO and Norder H (1995) Subtypes, genotypes and molecular epidemiology of the hepatitis B virus as reflected by sequence variability of the S-gene. Intervirology 38: 24-34.

Matsukura M, Shinozuka K, Zon G, Mitsuya H, Reitz M, Cohen J and Broder S (1987) Phosphorothioate analogs of oligodeoxynucleotides: inhibitors of replication and cytopathic effects of human immunodeficiency virus. Proc Natl Acad Sci USA 84: 7706-7710.

Miller P, Yano J, Yano E, Carroll C, Jayaram K and Ts'o P (1979) Nonionic nucleic acid analogues. Synthesis and characterization of dideoxyribonucleoside methylphosphonates. Biochemistry 18: 5134-5143.

Nielsen P, Egholm M, Berg R and Buchardt O (1991) Sequence-selective recognition of DNA by strand displacement with a thymine-substituted polyamide. Science 254: 1497-1500.

Nielsen P, Egholm M, Berg R, Buchardt O (1993) Sequence specific inhibition of DNA restriction enzyme cleavage by PNA. Nucleic Acids Res 21: 197-200.

Norder H, Couroucé A-M and Magnius LO (1992) Molecular basis of hepatitis B virus serotype variations within the four major subtypes. J Gen Virol 73: 3141-3145.

Norder H, Couroucé A-M and Magnius LO (1994) Complete genomes, phylogenetic relatedness, and structural proteins of six strains of the Hepatitis B virus, four of which represent two new genotypes. Virology 198: 489-503.

Okamoto H, Tsuda F, Sakugawa H, Sastrosoewinjo RI, Imai M, Miyakawa Y and Mayumi M (1988) Typing hepatitis B virus by homology in nucleotide sequence: Comparison of surface antigen subtypes. J Gen Virol 69: 2575-2583.

Olerup O and Zetterquist H (1991) HLA-DRB1*01 subtyping by allele-specific PCR amplification: a sensitive, specific and rapid technique. Tissue Antigens 37: 197-204.

Saiki RK, Bugawan TL, Horn G,TMullis KB and Erlich HA (1986) Analysis of enzymatically amplified beta-globin and HLA-DQ alpha DNA with allele-specific oligonucleotide probes. Nature 324: 163-166.

Saiki RK, Gelfland DH, Stoffel S, Scharf SJ, Higuchi R, Horn GT, Mullis KB and Erlich HA (1988) Primer-directed enzymatic amplification of DNA with a thermostable DNA polymerase. Science 239: 487-491.

Saiki RK, Walsh PS, Levenson CH and Erlich HA (1989) Genetic analysis of amplified DNA with immobilizes sequence-specific oligonucleotide probes. Proc Natl Acad Sci USA 86: 6230-6234.

Sambrook J, Fritsch E, Maniatis T (1989) Molecular Cloning: A Laboratory Manual, 2^{nd} Edition, Cold Spring Harbour Laboratory Press, Cold Spring Harbor, NY, USA.

Sanchez-Pescador R, Stempien MS, Urdea MS (1988) Rapid chemiluminescent nucleic acid assays for detection of TEM-1 beta-lactamase-mediated penicillin resistance in *Neisseria gonorrhoeae* and other bacteria. J Clin Microbiol 26: 1934-1938.

Santamaria P, Boyce-Jacino MT, Lindstrom AL, Barbosa JJ, Faras AJ and Rich SS (1992) HLA class II "typing": direct sequencing of DRB, DQB and DQA genes. Hum Immunol 33: 69-81.

Santamaria P, Lindstrom AL, Boyce JM, Jacino MT, Myster SH, Barbosa JJ, Faras AJ and Rich SS (1993) HLA class I sequence-based typing. Hum Immunol 37: 39-50.

Stuyver L, Wyseur A, van Arnhem W, Hernandez F and Maertens G (1996) A second generation line probe assay for hepatitis C virus. J Clin Microbiol 34: 2259-2266.

Stuyver L, De Gendt S, Cadranel JF, Van Geyt C, Van Reybrouck G, Dorent R, Gandjbachkc I, Rosenheim M, Opolon P, Huraux JM and Lunel F (1999) Three cases of severe subfulminant hepatitis in heart transplanted patients after nosocomial transmission of a mutant hepatitis B virus. Hepatology 29: 1876-1883.

Swenson PD, Riess JT and Krueger LE (1991) Determination of HBsAg subtypes in different high risk populations using monoclonal antibodies. J Virol Methods 33: 27-28.

Telenta PFS, Poggio GP, Lopez JL, Gonzalez J, Lemberg A and Campos RH (1997) Increased prevalence of genotype F hepatitis B virus isolates in Buenos Aires, Argentina. J Clin Microbiol 35: 1873-1875.

Tran A, Kremsdorf D, Capel F, Housset C, Dauguet C, Petit M-A and Brechot C (1991) Emergence of and takeover by hepatitis B virus (HBV) with rearrangements in the pre-S/S and pre-C/C during chronic HBV infection. J Virol 65: 3566-3574.

Urdea MS, Horn T, Fultz TJ, Anderson M, Running JA, Hamren S, Ahle D, Chang CA (1991) Branched DNA amplification multimers for the sensitive, direct detection of human hepatitis viruses. Nucleic Acids Symp Ser 24:197-200.

Van Geyt C, De Gendt S, Rombaut A, Wyseur A, Maertens G, Rossau R and Stuyver L (1998) A line probe assay for hepatitis B virus genotypes. In: *Therapies of viral hepatitis.* pp. 139-145. Eds. RF Schinazi, JP Sommadossi and H Thomas. International Medical Press, London, UK.

Wu D and Wallace B (1989) The ligation amplification reaction (LAR) - amplification of specific DNA sequences using sequential rounds of template-dependent ligation. Genomics 4: 560-569.

Yoshida M, Kimura A, Numano F and Sasazuki T (1992) Polymerase-chain-reaction-based analysis of polymorphism in the HLA-B gene. Hum Immunol 34: 257-266.

## Claims

1. An isolated and/or purified HBV polynucleic acid, having a nucleotide sequence which is unique to a theretofore-unidentified HBV genotype and which is phylogenetically different from HBV genotypes A, B, C, D, E and F characterized by:
(a) a sequence as defined in figure 1 (SEQ ID NO 1); or
(b) a sequence with at least 90%, more preferably 91%, most preferably 92% identity to the sequence as defined in figure 1; or
(c) a sequence that is degenerate as a result of the genetic code to the sequence as defined in (a) and (b).

2. An HBV polynucleic acid according to claim 1, further characterized that its nucleotide sequence differs from HBV genotype A, B, C, D, E or F in at least one of the following characteristics:
(a) it has a length of 3248 basepairs;
(b) it encodes a translational stop codon at amino acids 2 and 28 of the preCore region;
(c) it has a 36 nucleotide insert in the N-terminal part of the Core region;
(d) it has a 6 nucleotide deletion in the C-terminal part of the HBcAg ORF;
(e) it has a 3 nucleotide deletion in the N-terminal part of the preS1 ORF.

3. An HBV polynucleic acid according to any of claims 1 or 2 comprising at least one of the following sequences (Figs. 7 and 8): SEQ ID NO 44, SEQ ID NO 45, SEQ ID NO 46, SEQ ID NO 47, SEQ ID NO 48, SEQ ID NO 49, SEQ ID NO 50, SEQ ID NO 51, SEQ ID NO 53, SEQ ID NO 54, SEQ ID NO 55, SEQ ID NO 56, SEQ ID NO 57, SEQ ID NO 58, SEQ ID NO 59, SEQ ID NO 60.

4. A fragment of a polynucleic acid according to any of claims 1 to 3 characterized:
- that said fragment is unique to the theretofore-unidentified HBV genotype G, containing at least one nucleotide or a combination of nucleotides different from known HBV genotypes A, B, C, D, E or F nucleotide sequences; or
- that said fragment is a universal fragment, comprised within the nucleic acid sequence of genotypes A, B, C, D, E, F and G;
or the complement of said fragment, provided that said fragment is not equal to the preC/C gene sequence as depicted in figure 2 (SEQ ID NO 7) or the preS/S sequence as depicted in figure 3 (SEQ ID NO 8).

5. A method for the detection in a biological sample of the presence of a polynucleic acid according to any of claims 1 to 4.

6. A polypeptide having an amino acid sequence encoded by a polynucleic acid according to any of claims 1 to 4.

7. A method for detecting antibodies specific to the theretofore-unidentified HBV genotype, more specifically for detecting antibodies specific to the HBcAg and/or the HBeAg protein of HBV genotype G or for detecting antibodies recognizing HBV genotypes A, B, C, D, E, F and G, present in a biological sample, comprising the following steps:
(a) contacting the biological sample with a polypeptide according to claim 6;
(b) detecting the immunological complex formed between said antibodies and said polypeptide.

8. An antibody specifically raised upon immunization with at least one polypeptide according to claim 6, more specifically with the HBcAg or HBeAg protein of HBV genotype G or part thereof, with said antibody being specifically reactive with said polypeptide and not reactive with a peptide characteristic for HBV genotype A, B, C, D, E and/or F or with said antibody being reactive with a universal peptide present in HBV genotype A, B, C, D, E, F and G.

9. A method for detecting a polypeptide according to claim 6 comprising the following steps:
(a) contacting said polypeptide with an antibody according to claim 8, binding to said polypeptide;
(b) determining the immunological complex formed between said polypeptide and said antibody.

10. Use of a polypeptide or antibody according to claims 6 or 8 for the preparation of a vaccine and or a medicament for the prevention and/or treatment of HBV infections.
